# EUROPEAN PATENT SPECIFICATION

(11) **EP 1 364 034 B1**
(45) Date of publication and mention of the grant of the patent: **09.06.2010**
(21) Application number: 02702375.3
(22) Date of filing: 27.02.2002
(51) Int. Cl.: C12N 15/82, C12N 15/05

(54) **RECOMBINANT VIRAL SWITCHES FOR THE CONTROL OF GENE EXPRESSION IN PLANTS**
RECOMBINANTE VIRALE SCHALTER ZUR KONTROLLE VON GENEXPRESSION IN PFLANZEN
COMMUTATEURS VIRAUX RECOMBINANTS PERMETTANT DE REGULER L'EXPRESSION GENIQUE CHEZ LES VEGETAUX

(30) Priority: 27.02.2001 DE 10109354
(43) Date of publication of application: 26.11.2003
(73) Proprietor: Icon Genetics GmbH, 80333 München (DE)
(72) Inventor: KLIMYUK, Victor, 06114 Halle (DE); BENNING, Gregor, 06108 Halle/Saale (DE); GLEBA, Yuri, 06114 Halle/Saale (DE)
(74) Representative: Hartz, Nikolai
(86) International application number: PCT/EP2002/002091
(87) International publication number: WO 2002/068664

(56) References cited:
- WO-A-02/29068
- WO-A-98/54342
- US-A- 5 576 198
- US-A- 5 723 765
- WILDE R J ET AL: "CONTROL OF GENE EXPRESSION IN TOBACCO CELLS USING A BACTERIAL OPERATOR-REPRESSOR SYSTEM" EMBO JOURNAL, OXFORD UNIVERSITY PRESS, SURREY, GB, vol. 11, no. 4, 1992, pages 1251-1259, XP000617625 ISSN: 0261-4189
- GATZ ET AL: "Regulation of a modified CaMV 35S promoter by the Tn10-encoded Tet repressor in transgenic tobacco" MOLECULAR AND GENERAL GENETICS, SPRINGER VERLAG, BERLIN, DE, no. 227, 1991, pages 229-37, XP002075578 ISSN: 0026-8925
- ALBERT H ET AL: "Site-specific integration of DNA into wild-type and mutant lox sites placed in the plant genome" PLANT JOURNAL, BLACKWELL SCIENTIFIC PUBLICATIONS, OXFORD, GB, vol. 7, no. 4, 1995, pages 649-659, XP002097329 ISSN: 0960-7412
- HOFF T ET AL: "A RECOMBINASE-MEDIATED TRANSCRIPTIONAL INDUCTION SYSTEM IN TRANSGENIC PLANTS" PLANT MOLECULAR BIOLOGY, NIJHOFF PUBLISHERS, DORDRECHT, NL, vol. 45, no. 1, 2001, pages 41-49, XP001022118 ISSN: 0167-4412

## Description

### FIELD OF THE INVENTION

The present invention relates to a process of controlling a biochemical process or biochemical cascade of interest in a plant. Moreover, the present invention relates to a process for producing a product in a transgenic plant by using the process of controlling a biochemical process or biochemical cascade of interest according to the invention. Further, the present invention relates to a kit-of-parts for performing the processes of the invention. The process of the invention allows for the selective control of transgene expression in a transgenic plant whereby a biochemical process or biochemical cascade of interest previously non-operable in the plant may be selectively switched on at any predetermined time.

### BACKGROUND OF THE INVENTION

### Controllable transgene expression systems in plants

One of the major problems in plant biotechnology is the achievement of reliable control over transgene expression. Tight control over gene expression in plants is essential if a downstream product of transgene expression is growth inhibitory or toxic, like for example, biodegradable plastics (Nawrath, Poirier & Somerville. 1994, Proc. Natl. Acad. Sci., 91, 12760-12764; John & Keller, 1996, Proc. Natl. Acad Sci., 93, 12768-12773; US6103956; US5650555) or protein toxins (US6140075).

Existing technologies for controlling gene expression in plants are usually based on tissue-specific and inducible promoters and practically all of them suffer from a basal expression activity even when uninduced, i.e. they are "leaky". Tissue-specific promoters (US05955361;WO09828431) present a powerful tool but their use is restricted to very specific areas of applications, e.g. for producing sterile plants (WO9839462) or expressing genes of interest in seeds (WO00068388; US05608152). Inducible promoters can be divided into two categories according to their Induction conditions - those induced by abiotic factors (temperature, light, chemical substances) and those that can be induced by biotic factors, for example, pathogen or pest attack. Examples of the first category are heat-inducible (US 05187287) and cold-inducible (US05847102) promoters, a copper-inducible system (Mett et al., 1993, Proc. Natl. Acad Sci., 90, 4567-4571), steroid-inducible systems (Aoyama & Chua, 1997, Plant J., 11, 605-612; McNellis et al., 1998, Plant J., 14 247-257; US06063985), an ethanol-inducible system (Caddick et al., 1997, Nature Biotech., 16. 177-180; WO09321334), and a tetracycline-inducible system (Weinmann et al., 1994, Plant J., 5, 559-569). One of the latest developments in the area of chemically inducible systems for plants is a chimaeric promoter that can be switched on by glucocorticoid dexamethasone and switched off by tetracycline (Bohner et al., 1999, Plant J., 19, 87-95). For a review on chemically inducible systems see: Zuo & Chua, ( 2000, Current Opin. Biotechnol., 11. 146-151). Other examples of inducible promoters are promoters which control the expression of patogenesis-related (PR) genes in plants. These promoters can be induced by treatment of the plant with salicylic acid, an important component of plant signaling pathways in response to pathogen attack, or other chemical compounds (benzo-1,2,3-thiadiazole or isonicotinic acid) which are capable of triggering PR gene expression (US05942662).

There are reports of controllable transgene expression systems using viral RNA/RNA polymerase provided by viral infection (for example, see US6093554; US5919705). In these systems, a recombinant plant DNA sequence includes the nucleotide sequences from the viral genome recognized by viral RNA/RNA polymerase. The effectiveness of these systems is limited because of the low ability of viral polymerases to provide functions in trans, and their inability to control processes other than RNA amplification.

US5723765 describes transgenic plants produced by a triple vector transfection system. The system is composed of a first gene, which in order to be expressed and change the plant phenotype, should recombine to excise an intervening sequence that blocks its expression; of a second gene encoding a recombinase (e.g. CRE) capable of excising the intervening sequence; and of a third gene that encodes a repressor specific for the repressible promoter encoding the recombinase (TetR system).

The systems described above are of significant interest as opportunities of obtaining desired patterns of transgene expression, but they do not allow tight control over the expression patterns, as the inducing agents (copper) or their analogs (brassinosteroids in case of steroid-controllable system) can be present in plant tissues at levels sufficient to cause residual expression. Additionally, the use of antibiotics and steroids as chemical inducers is not desirable for the large-scale applications. When using promoters of PR genes or viral RNA/RNA polymerases as control means for transgenes the requirements of tight control over transgene expression are also not fulfilled, as casual pathogen infection or stress can cause expression. The tissue or organ-specific promoters are restricted to very narrow areas of applications, since they confine expression to a specific organ or stage of plant development, but do not allow the transgene to be switched on at will.

### Plant viral vectors and their use in the field of applied plant virology

Presently, there are three distinct major fields in the area of applied plant virology: a) use of viruses as vectors for transgene overexpression; b) use of viruses as vectors for plant functional genomics, and c) use of viral components in the field of phytopathology for generating virus-resistant transgenic plants.

Plant viruses can serve as efficient tools for high level expression of transgenes in host plant species. The use of transgenic plant virus in field does not seem to compromise any biosafety issues. For example, Animal and Plant Health Inspection Service, USDA, did not find any significant impact after field trials with genetically modified TMV (tobacco mosaic virus) and tobacco etch viruses containing heterologous genes of pharmaceutical interest. As a result, two permissions were issued in 1996 and 1998. Work has been conducted in the area of developing viral vectors for transferring foreign genetic material into plant hosts for the purposes of expression (US4885248; US5173410). There are several patents which describe the first viral vectors suitable for systemic expression of transgenic material in plants (US5316931; US5589367; US5866785). In general, these vectors can express foreign genes from an additional subgenomic promoter (US5466788; US5670353; US5866785), as translational fusions with viral proteins (US5491076; US5977438) or from polycistronic viral RNA using IRES elements for independent protein translation, also used herein, according to ANNEX A corresponding to German Patent Application No 10049587.7. Carrington *et al.,* (US5491076) describe the use of an endogenous viral protease to cleave heterologous proteins from viral polyproteins. Another area of application for viral vectors is plant functional genomics. Della-Cioppa et al., (WO993651) describe the use of TMV-based viral vectors for expression of plant cDNA libraries with the purpose of silencing endogenous genes.

Angell & Baulcombe (1997, EMBO J, 16, 3675-3684; WO9836083) describe a PVX-based system called "Amplicon^{™}" designed for down-regulating the targeted genes in plants. The same system in combination with Hc-Pro that suppresses transgene silencing in plants (Pruss et al, 1997, Plant Cell, 9, 859-868; US5939541) is used for overexpression of transgenes. US patent 5939541 describes an approach based on using the 5'proximal region (booster sequence, including the Hc-Pro gene) of the potyvirus to enhance expression of any gene in plants. This sequence can be stably integrated into the plant genome or delivered by a virus. It is worth mentioning that Hc-Pro has a pronounced pleiotropic effect and enhances the expression of both transgenes and endogenous plant genes. Thus, these systems provide at best a quantitative improvement of total protein expression over existing processes. They do so by influencing many components of the protein production machinery by an unknown mechanism and in a hardly controlled manner.

There is an abundant literature including patent applications which describe the design of virus resistant plants by the expression of viral genes or mutated forms of viral RNA (e.g. US5792926; US6040496). It is also worth mentioning that an environmental risk is associated with the use of such plants due to the possibility of forming novel viruses by recombination between the challenging virus and transgenic viral RNA or DNA (Adair & Keamey, 2000, Arch. Virol, 145, 1867-1883).

Therefore, it is an object of the present invention to provide an environmentally safe process of controlling a biochemical process or a biochemical cascade of interest in a plant whereby the process or cascade may be selectively switched on at any predetermined time.

It is another object of this invention to provide a process for producing a product in a transgenic plant wherein the production of the product may be selectively switched on after the plant has grown to a desired stage, whereby the process is environmentally safe and does not lead to the release of potentially hazardous functional transgenes in the environment.

Another object of this invention is to provide a kit of parts for performing such processes.

### GENERAL DESCRIPTION OF THE INVENTION

These objects are achieved by a process according to claims 1, 23 and 40. More specifically, these objects are achieved by a process of controlling a biochemical process or biochemical cascade of interest in a plant, said process being characterized by comprising the following steps:
(a) introducing into the nuclear genome of the plant one or more first heterologous DNA sequences,
(b) infecting the plant with at least one viral transfection vector containing in its genome one or more second heterologous DNA sequences, thus triggering a process of interaction in the plant between
   (i) expression products of the first heterologous DNA sequences, and
   (ii) one or more second heterologous DNA or RNA sequences of the transfection vector and/or expression products of the second heterologous DNA or RNA sequences, and
   (iii) optionally one or more externally added low molecular weight components, thus switching on the biochemical process or cascade of interest that was not operable prior to said interaction. Preferably, said first heterologous DNA sequence(s) in the above processes are of non-plant viral origin, i.e. do not originate from a plant virus.

The present invention further provides a process of producing a product in a transgenic plant comprising the process of controlling a biochemical process or a biochemical cascade of interest in a plant according to the invention. In particular, the process further comprises the following steps:
(a) growing the transgenic plant to a desired stage, followed by
(b) infecting the plant with one or more vectors, and optionally contacting the plant with one or more low molecular weight components, thus switching on the biochemical process or cascade necessary for the production of the product, said process or cascade not being operable prior to said interaction, and
(c) producing the product in the plant,
   whereby said vector is preferably a viral transfection vector.

Further a kit of parts is described for the above processes comprising a transgenic plant or seeds thereof and a virus-based vector. Also, a kit of parts is described comprising a transgenic plant and one or more vectors, whereby said vector(s) may give rise to one or more viral transfection vectors in a plant. Said transgenic plant contains a first heterologous DNA sequence according to step (a) of the process of the invention.

Further, a vector for performing step (b) of the process of the invention and a plant obtained or obtainable by the process of the invention are described.

According to the invention it is possible to selectively switch on a biochemical process or biochemical cascade in a transgenic plant by infecting the transgenic plant with one or more viral transfection vectors. The biochemical process or cascade is not operable in the transgenic plant prior to the infection with the viral vector for lack of essential elements or functions necessary to perform the biochemical process or cascade. Essential elements may be e.g. a promoter, an RNA polymerase, a transcription factor or the like. Essential functions may be transcription, translation or enzymatic activity which is not operable e.g. for lack of functional coupling of a promoter with a downstream sequence to be expressed. The biochemical process or cascade becomes operable by a process of interaction triggered by the infection. The process of interaction in the plant requires one or more first heterologous DNA sequences of the nuclear genome and/or expression products of the first heterologous DNA sequences, and one or more second heterologous DNA sequences of the transfection vector and/or expression products of the second heterologous DNA sequences, and optionally one or more externally added low molecular weight components. Preferably the process of interaction switching on the biochemical process or cascade of interest requires one first heterologous DNA sequence of the nuclear genome and/or expression product of the first heterologous DNA sequence, and one second heterologous DNA sequence of the transfection vector and/or expression product of the second heterologous DNA sequence.

The DNA sequences used according to the invention may be obtained via the use of RNA sequences. Specifically, the DNA sequences of steps (a) or (b) may be an expression product of RNA sequences, e.g. of an RNA virus.

In the absence of any one of the first and second heterologous DNA sequences or expression products of the first and second heterologous DNA sequences required for the process of interaction, none of the present first and second heterologous DNA sequences, expression products of the first and second heterologous DNA sequences or the externally added low molecular weight components are able, alone or in combination, to switch on the biochemical process or cascade of interest. Moreover, the biochemical process or cascade of interest is not a process which has been silenced by a mechanism such as post-transcriptional gene silencing which may be still operating at a low level. The biochemical process or biochemical cascade of interest is not operable in the transgenic plant prior to the infection with a corresponding viral transfection vector and prior to the optional addition of a low molecular weight component. Moreover, a viral transfection vector according to the invention is unable to switch on the biochemical process or biochemical cascade of interest in a plant not having the corresponding first heterologous DNA sequence required according to the invention.
Finally, the biochemical process or cascade of interest cannot be switched on in a plant by contacting the plant with a low molecular weight component in the absence of the first and second heterologous DNA sequences or expression products required for switching on the process or cascade of interest according to the invention.

The process of the invention provides control over a biochemical process or cascade of interest with a hitherto unattainable technical precision and environmental safety. Thereby novel applications in plant biotechnology are available for solving problems which cannot be solved by conventional technologies involving basal transgene expression activity in the plant, particularly when producing toxic substances or biodegradable polymers.

Moreover, the precise control according to the invention allows to grow a transgenic plant to a desired stage where the plant is best suited for performing the biochemical process or cascade of interest without burdening the plant with a basal expression activity slowing down the growth of the plant. Once the plant is ready for efficiently performing the biochemical process or cascade of interest, the process or cascade of interest may be switched on and performed with high efficiency. Accordingly, the process of the invention allows to safely decouple the growth phase and the production phase of a transgenic plant.

Moreover, it is possible to design multi-component systems for multiple biochemical processes or cascades of interest, whereby one or more desired processes or cascades can be selectively switched on.

In a first embodiment, the system comprises a transgenic plant containing a heterologous DNA sequence providing an expression product which is necessary to control expression of a desired product encoded by a viral transfection vector. The system further comprises different viral vectors each encoding a different product to be expressed in the transgenic plant. Thereby, it is possible to safely use the same transgenic plant for the production of different products depending on the viral vector used. The advantage of this system is clear in the light of the fact that it may take years to provide a stably transformed transgenic plant whereas the preparation of a viral vector may be accomplished in a few weeks.

In a second embodiment, the transgenic plant contains multiple heterologous DNA sequences which may encode for different desired gene products. Each of the multiple heterologous DNA sequences may be controlled by a different viral vector. Thereby, it is possible to selectively control the heterologous DNA sequences of the transgenic plant by the choice of the corresponding viral vector.

Moreover, in a third embodiment, it is possible to design a system wherein the process of interaction switching on the biochemical process or cascade of interest requires the infection with more than one viral vector and the optional application of one or more externally added low molecular weight components whereby present technology is even safer to operate.

A biochemical process or cascade to be controlled according to the invention may be any process or cascade which may take place in a living plant system. Preferred biochemical processes or cascades lead to the production of a product in the plant. Examples of products of interest which may be obtained by the process of this invention include polypeptides or proteins as primary products, (posttranslationally) modified or otherwise processed proteins which may be enzymes, proteins having a desired glycosylation pattern, non-proteinaceous low-molecular weight products and oligomerisation products thereof like carbohydrates or biodegradable plastics etc. Most preferred are pharmaceutical polypeptides.

Said biochemical process or cascade, notably expression of a protein, may involve formation of sub-genomic RNA, notably from a viral transfection vector.

The process of controlling a biochemical process or cascade involves at least the following two components:
(1) a transgenic plant containing a first heterologous DNA sequence preferably not originating from a plant virus and
(2) a viral transfection vector containing a second heterologous DNA sequence.

For the purposes of this invention, a heterologous DNA sequence is a sequence which neither occurs naturally in the plant species employed nor in the wild-type virus on which the viral vector is based on, respectively. Nevertheless, such a sequence may comprise sequence portions native to the plant and/or the virus of interest besides heterologous portions. Said heterologous DNA sequence may comprise more than one functional element. Examples of such functional elements include promoter, enhancer, transcription termination region, coding region, non-translated spacer region, translation initiation region, IRES (internal ribosome entry site) region, stop codon etc. or parts thereof.

Said first heterologous DNA sequence is preferably heterologous to said host plant. Said first heterologous DNA sequence may be of viral origin. Preferably, however, said first heterologous DNA sequence is of non-plant viral origin, i.e it is not of plant viral origin. Said second heterologous DNA (or RNA) sequence is preferably heterologous to the virus on which the viral vector is based on. Said second heterologous DNA sequence may be of plant origin.

Infection of the transgenic plant (1) with a viral vector (2) triggers a process of interaction between said at least first and second heterologous DNA sequence or expression product(s) thereof, thus switching on the biochemical process or cascade of interest. The fact that the at least two components (1) and (2) are required means that interaction of said components is a necessary condition for switching on said biochemical process or cascade. Prior to said interaction, said biochemical process is not operable whereby "leaky" expression of a transgene cannot occur. In prior art systems, expression of a transgene can merely be induced by a quantitative increase or an enhancement of an already existing, albeit lower, expression level. The present invention not only provides a quantitative increase but also a qualitative change in that a previously not operable process or cascade becomes operable. This advantage of the present invention is of particular importance when a biochemical process or cascades of interest involves formation of a toxic or growth-retarding product. According to the invention it is possible to entirely separate plant growth and production of said product whereby interference with or retardation of plant growth by the presence of the desired product in the growing plant is avoided. Therefore, the stages of biomass accumulation and production of a product of interest may be decoupled.

The transgenic plant and the transgenic vector of the invention are not functional for controlling a biochemical process or biochemical cascade with viruses or plants not containing the corresponding heterologous DNA sequences, respectively. Consequently, this invention represents a significant progress in terms of biological safety in plant biotechnology.

Said processes of interaction which are triggered by infecting the transgenic plant with a viral vector and which lead to switching on of a biochemical process include DNA recombination, DNA replication, transcription, restriction, ligation, hybridisation, RNA replication, reverse transcription, RNA processing, splicing, translation, protein folding, assembly, targeting, posttranslational processing, enzymatic activity. Said expression products of said first or said second heterolgous DNA sequence include RNA, notably mRNA, and polypeptides or proteins.

Said process of interaction between said first and said second heterologous sequences (and optionally further sequences) does preferably not include complementation (genetic reassembly) of viral functions or of an infectious viral vector.

This invention preferably relates to multicellular plants. Examples for plant species of interest are monocotyledonous plants like wheat, maize, rice, barley, oats, millet and the like or dicotyledonous plants like rape seed, canola, sugar beet, soybean, peas, alfalfa, cotton, sunflower, potato, tomato, tobacco and the like. The fact that there are specific viruses for each of such plants, contributes to the broad versatility and applicability of this invention. The viral transfection vector used in this invention may be derived from any such plant specific virus. The viral vector may be based on an RNA or on a DNA double-stranded or single-stranded virus. Specific examples of viral transfection vectors are given below and in ANNEX A and ANNEX B.

In step (a), the plant may be a natural plant or a genetically modified plant. The genetic modification may be either in the nuclear genome of the plant or in an organelle genome such as plastid or mitochondria genome. In step (a) a heterologous sequence is introduced in the nuclear genome, and preferably a stable genome modification is provided. Step (a) may be carried out more than once in order to introduce more than one heterologous DNA sequence. In this way several heterologous functions may be introduced in the target plant e.g. for engineering a whole biochemical pathway.

In step (b), the transgenic plant obtained according to step (a) is infected with a viral transfection vector. The infecting may be achieved by supplying the plant with an assembled virus particle, or with infectious viral nucleic acids, or by activating a transfection process by release of viral nucleic acids previously incorporated into the plant genome. The assembled virus particle may contain RNA and the infectious viral nucleic acids may be RNA, notably if they are based on an RNA virus (cf. examples 2 and 3).

More than one vector may be used to control the biochemical process or biochemical cascade of interest. Preferably, only one vector containing the desired heterologous sequence(s) is used for reasons of reproducibility of the process. Infection may be done by contacting the viral vector with said transgenic plant. Preferably, mechanical stimulation like rubbing or scatching of leaves or other plant tissue may be used to initiate infection. Infection may also be achieved by activating the viral vector previously integrated in the genome of the host plant. Viral vectors capable of systemic infection of the plant are preferred.

The infection of the plant in step (b) may further comprise *Agrobacterium*-mediated transfer of nucleic acid sequences into cells of said plant. *Agrobacterium*-mediated transfer may e.g. be used to integrate sequences into the genome of the host plant. A viral vector may be activated from such sequences integrated the genome of the plant. An RNA virus-based vector may e.g. be activated by transcribing a cDNA copy of said virus, notably by transcribing a cDNA copy integrated into the genome. However, integration of sequences introduced into plant cells by *Agrobacterium*-mediated transfer do not have to lead to integration into the genome. *Agrobacterium*-mediated transfer may provide transient expression of a gene flanked by T-DNA. Notably, sequences on a Ti-plasmid may exert a function in the process of the invention without or before integration into the genome. If more than one vector is introduced in step (b), the same or different methods may be used for these vectors. Notably, more than one vector may be introduced by *Agrobacterium*-mediated transfer using different *Agrobacterium* strains simultaneously (e.g. using an *Agrobacterium* mixture) or consecutively.

In one embodiment of the invention, a further vector in addition to said viral infection vector may be introduced in step (b) of the process of the invention. Said further vector may be or may not be a viral transfection vector. Said further vector may provide a further nucleic acid sequence as a necessary condition for switching on said biochemical process of the invention (cf. example 6).

In another embodiment of the invention, infecting the plant in step (b) is achieved by introducing one or more vectors into cells of said plant, whereby said vector(s) are adapted to undergo processing to generate a viral transfection vector in cells of said plant. Three, four or more vectors may be introduced in cells of said plant in this embodiment. Preferably, two vectors are introduced. Said vectors may or may not be viral transfection vectors. Preferably, at least one of said vectors is a viral transfection vector (cf. example 6). However, according to this embodiment, a viral transfection vector may also be generated from introduced vectors none of which is a viral transfection vector. Said biochemical process or pathway may be switched on by the assembly and appearance of said viral transfection vector in cells of the plant by said processing, triggered by measures (a) and (b) of the process of the invention.

Steps (a) and (b) of the process of this invention may be carried out on the same plant.
However, it is preferred that a stable plant line is obtained according to conventional processes based on a plant in which at least one first heterologous DNA sequence of interest was introduced according to step (a). Transgenic plants may then be grown from seeds of a stably transformed plant, and infection according to step (b) may be performed when initiation of said biochemical process is desired. Step (b) is preferably carried out in a greenhouse.

A viral transfection vector is a nucleic acid (RNA or DNA) or nucleoprotein which upon invading a wild type or genetically engineered host is capable of replication or amplification in cells of said host and of amplification and/or expression of heterologous sequence(s) of interest. Preferably, said viral transfection vector is further capable of cell to cell movement. More preferably, a viral vector retains additional viral capabilities such as long distance movement, assembly of viral particles or infectivity. In the process of this invention, a viral vector might not have all the properties mentioned above, but such functions can be provided in trans in the context of host cell. Preferred viral transfection vectors encode and express a movement protein. Further, they may encode a virus-specific DNA or RNA polymerase (replicase); a RNA-dependent RNA polymerase (RdRp) is preferred.

In a first specific embodiment of this invention, the biochemical process of interest is expression of a heterologous DNA sequence of interest. This process may be called primary biochemical process. This primary process results in an RNA or polypeptide molecule. In the simplest case, one of the RNA or polypeptide molecule is the product of interest. In a biochemical cascade, the product of such a primary process may cause a secondary biochemical process e.g. by way of its catalytic activity or by way of regulating the other biochemical process. In a biochemical cascade, more than one biochemical process takes place, whereby each such process depends on a previous biochemical process. Said controlling or switching is preferably directed to said primary biochemical process in this embodiment.

In the first specific embodiment, the heterologous DNA sequence of interest which is to be expressed, may either be a first heterologous DNA sequence of the plant nuclear genome or a second heterologous DNA (or RNA) sequence of said viral transfection vector.

In a second specific embodiment of this invention, the biochemical process of interest is the production of non-proteinaceous compound of interest by the plant.

In a further specific embodiment of the invention, a process of controlling a biochemical process (II) or biochemical cascade (III) of interest in a plant is provided, said process being
characterized by comprising the following steps:
(a) introducing into the nuclear genome of the plant one or more first heterologous nucleic acid sequences,
(b) infecting the plant with at least one vector containing in its genome one or more second heterologous nucleic acid sequences,
   thus triggering a process of interaction (I) in the plant between
   (i) one or more first heterologous nucleic acid sequences of the nuclear genome and/or expression products of the first heterologous nucleic acid sequences, and
   (ii) one or more second heterologous nucleic acid sequences of the transfection vector and/or expression products of the second heterologous nucleic acid sequences, and
   (iii) optionally one or more externally added low molecular weight components, whereby a viral transfection vector is generated in cells of said plant, thus switching on the biochemical process (II) or biochemical cascade (III) of interest that was not operable prior to said interaction; as defined in claim 40.
Further, specific embodiments of this process may be as described above, where applicable.

### BRIEF DESCRIPTION OF THE FIGURES

- Fig.1A: is a schematic representation of a process according to the invention.
- Fig.1 B: is a schematic representation of possible classes of processes of interaction in an infected plant cell.
- Fig. 2: depicts crTMV-based vectors pIC1111 and pIC1123 containing IRES_{cp,148}^{CR} - Ac transposase and and IRES_{mp.75}^{CR} - Ac transposase, respectively. Also shown is the T-DNA region of binary vector pSLJ744 containing p35S::Ds::GUS-3'ocs.
- Fig. 3: depicts crTMV-based vectors pIC2541 and pIC2531 containing IRES_{cp,148}^{CR} - Cre recombinase and and IRES_{mp,75}^{CR} - Cre recombinase, respectively. Also shown is the T-DNA region of the binary vector pIC2561 containing the GUS gene flanked by two IoxP sites in direct orientation.
- Fig. 4: depicts crTMV-based vectors pIC2541 and pIC2531 (see also Fig. 3) in combination with the T-DNA region of the binary vector pIC1641 containing the GUS gene flanked by two inverted IoxP sites.
- Fig. 5: depicts the T-DNA region of the binary vector pIC2691 carrying the GUS gene under control of T7 promoter and crTMV-based vector pIC2631 containing the T7 polymerase gene.
- Fig. 6: shows X-gluc stained leaves of transgenic *Arabidopsis* plants transformed with pSLJ744. Transcription of the GUS gene is prevented by the insertion of Ds element.
A- leaves inoculated with the transcript from pIC1123.
B - leaves inoculated with the transcript from pIC1111.
C - leaves inoculated with water.
- Fig. 7: depicts the TMV-based viral provectors pICH4371 and pICH4461 end of provector (RdRp: RNA dependent RNA polymerase; MP: movement protein; sGFP: synthetic green fluorescent protein; 3'NTR: 3' non-translated region of TMV; sgp: subgenomic promoter).
- Fig. 8: depicts the T-DNA of binary vector pICH1754 providing a Cre recombinase expression cassette.
- Fig. 9: depicts a scheme of formation of viral vectors from provectors in the presence of Cre recombinase.
Appendices 1 to 11 depict vectors and constructs used in example 6.

### DETAILED DESCRIPTION OF THE INVENTION

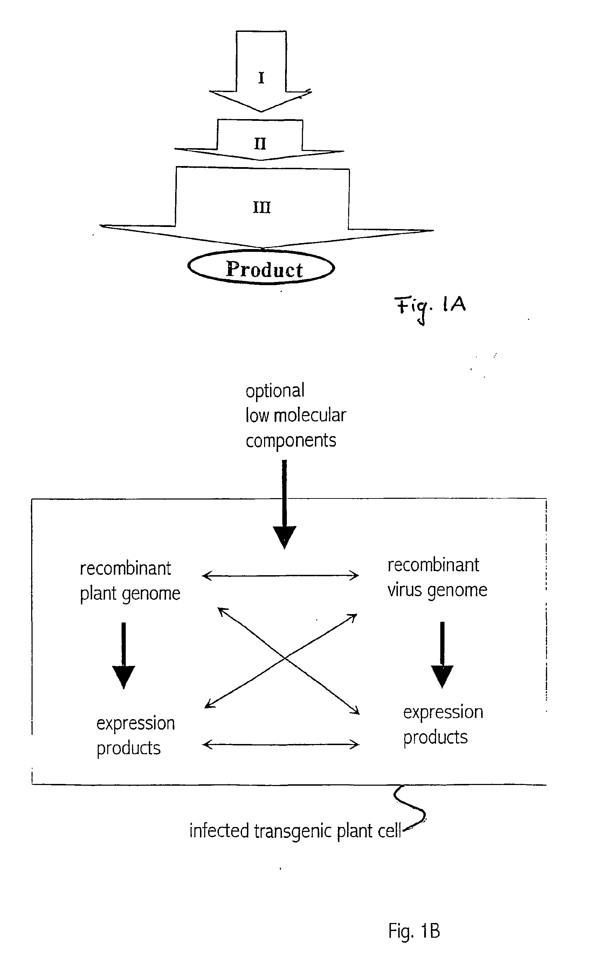

As shown by Figure 1A, the present invention provides a process of controlling a biochemical process or biochemical cascade of interest in a plant whereby the process comprises a process of interaction (I), switching on a biochemical process of interest (II), which in turn may be causal for a biochemical cascade of interest (III). The process of interaction may involve any one of the following reactions or combinations of the reactions of DNA, RNA and Proteins. DNA reactions contemplated in this invention are restriction, recombination, replication, transposition, amplification, and transcription. RNA reactions contemplated in this invention are RNA processing, replication, reverse transcription, hybridisation, and translation. Protein reactions contemplated in this invention are protein processing, folding, assembly, post-translational modifications, activation, targeting, binding activity modification, signal transduction. Process (III) may be present or absent. The production of a product is the preferred result of the process of the invention.

As shown by Figure 1B, possible processes of interaction may belong to one or more of the classes of interaction shown by the figure. In the figure, a transgenic plant cell infected with a viral transfection vector is shown schematically. The recombinant plant genome contains one or more heterologous DNA sequences which may lead to one or more expression products. The genome of the viral transfection vector contains one or more heterologous DNA sequences which may lead to one or more expression products. The transgenic plant cell may be contacted with one or more low molecular weight components capable of entering the cell. In the process of interaction switching on the biochemical process or biochemical cascade of interest in the plant cell, the following interactions may occur which are indicated by arrows in Figure 1B. One or more heterologous DNA sequences of the recombinant plant genome may interact with one or more heterologous DNA sequences of the viral transfection vector. One or more heterologous DNA sequences of the recombinant plant genome may interact with one or more expression products of the heterologous DNA sequences of the viral transfection vector. One or more expression products of the heterologous DNA sequences of the recombinant plant genome may interact with one or more expression products of the heterologous DNA sequences of the viral transfection vector. The expression product may be an RNA or a polypeptide. Any of these interactions may also involve or require the presence of one or more low molecular weight components added externally to the infected transgenic plant cell. The low molecular weight components may be necessary or desired for switching on or for promoting the biochemical process or biochemical cascade of interest. The low molecular weight components are unable to switch on the processes or cascades of interest in the absence of the viral transfection vector or the heterologous DNA sequence in the plant nuclear genome.

According to the first specific embodiment of this invention, a novel process to achieve transfection-based reliable control over either the expression of a transgene stably integrated into a plant, or over expression of a heterologous gene of a viral vector inside a transgenic plant host is provided. This process makes use of an interaction of at least two heterologous DNA sequences or expression products thereof, which is triggered only when the virus vector infection process is initiated. One of these sequences may be stably incorporated in the plant nuclear genome and the other one is provided by said viral vector. Such a switchable two-component expression system can be used to control a biochemical process or cascade that may be controlled at various levels, e.g. by triggering interaction reactions such as, but not limited to: DNA recombination, replication, transcription, restriction, RNA replication, reverse transcription, processing, translation, protein folding, assembly, targeting, posttranslational processing, enzymatic activity, etc.

This process requires at least a heterologous DNA in a transgenic plant and a recombinant virus-based vector comprising a heterologous DNA or RNA sequence. The general scheme of this process is shown in Fig. 1. A transgenic plant containing in its nuclear genome one or more stably integrated heterologous DNA sequence(s) of interest can be engineered using standard transcriptional or translational vectors and standard transformation protocols. Construction of transcriptional vectors for stable plant transformation has been described by numerous authors (for review, see Hansen & Wright, 1999, Trends in Plant Science, 4, 226-231; Gelvin, S.B., 1998, Curr. Opin. Biotech., 9, 227-232). The basic principle of all these constructs is identical: a fully functional transcription unit consisting of, in 5' to 3' direction, a plant-specific promoter, the structural part of a gene of interest and a transcriptional terminator has to be introduced into the plant cell and stably integrated into the genome in order to achieve expression of the gene of interest. Construction of translational plant vectors is described in German Patent Application Nos. 100 49 587.7 and 10061150.8 (ANNEX A and ANNEX B). The principal difference to transcriptional vectors is that translational vectors do not require a transcriptional promoter for expression of the gene of interest but rely on the plant transcription machinery following their integration into plant genome.

Different methods may be used for the delivery of an expression vector into plant cells such as direct introduction of said vector into the cells by the means of microprojectile bombardment, electroporation or PEG-mediated transformation of protoplasts. *Agrobacterium-*mediated plant transformation also represents an efficient way of vector delivery. Thus, DNA may be transformed into plant cells by various suitable technologies such as by a Ti-plasmid vector carried by *Agrobacterium* (US 5,591,616; US 4,940,838; US 5,464,763), particle or microprojectile bombardment (US 05100792; EP 00444882B1; EP 00434616B1). *Agrobacterium* can serve not only for stable nuclear transformation, but also for an efficient delivery of T-DNA for transient expression of gene(s) of interest. This so called agroinfiltration protocol was first developed to analyze foreign genes expression and gene silencing in plants (Kaplia et al., 1997, Plant Science, 122, 101-108; Schöb et al., 1997, Mol. Gen. Genet., 256, 581-588).

In principle, other plant transformation methods can also be used e.g. microinjection (WO 09209696; WO 09400583A1; EP 175966B1), electroporation (EP00564595B1; EP00290395B1; WO 08706614A1) etc. The choice of the transformation method depends on the plant species to be transformed. For example, microprojectile bombardment may be prefered for monocots transformation, while for dicots, *Agrobacterium*-mediated transformation gives generally better results.

Construction of plant viruses for the expression of non-viral genes in plants has been described in several papers (Dawson et al., 1989, Virology, 172, 285-293; Brisson et al., 1986, Methods in Enzymology, 118, 659; MacFarlane & Popovich, 2000, Virology, 267, 29-35; Gopinath et al., 2000, Virology, 267, 159-173; Voinnet et al., 1999, Proc. Natl. Acad. Sci. USA, 96. 14147-14152) and can be easily performed by those skilled in the art.

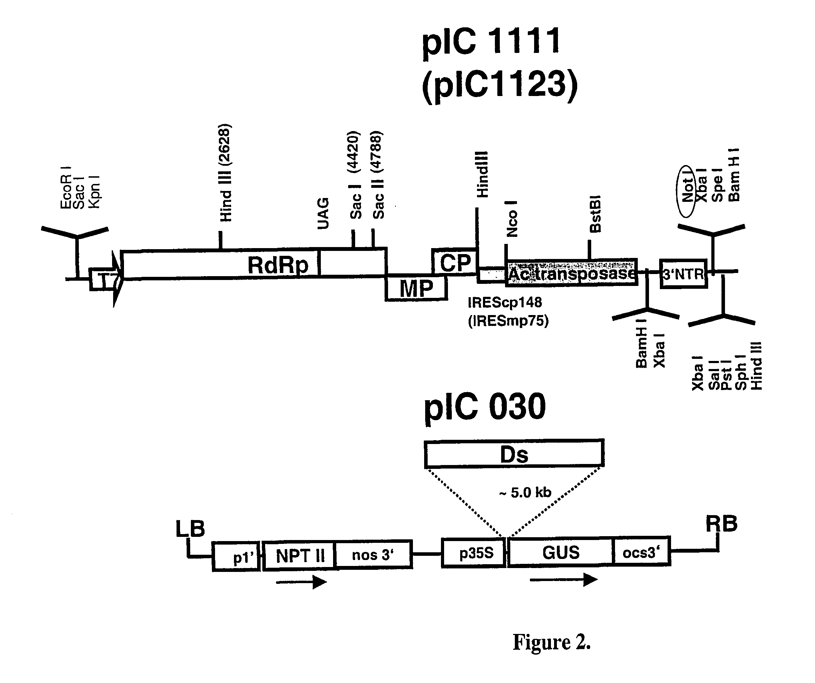

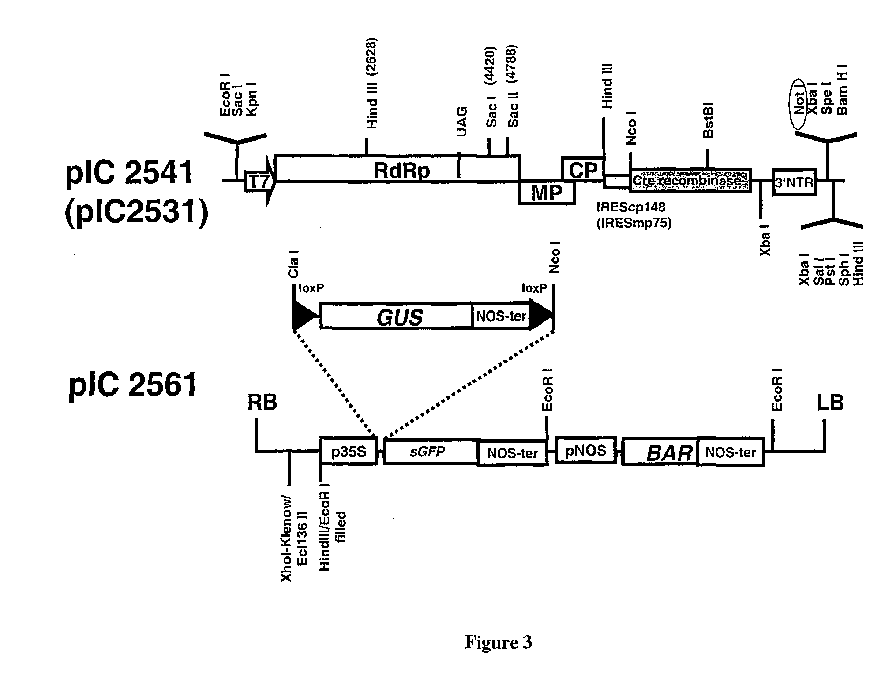

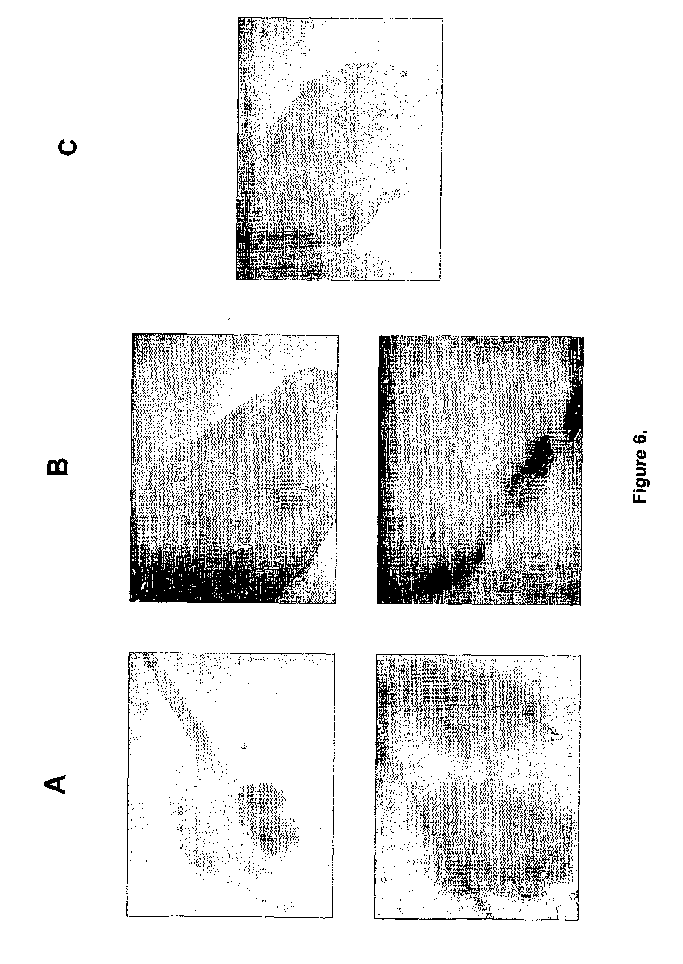

In one specific embodiment of our invention, the transgene in a plant genome is separated from its promoter by a DNA insert sufficiently long to prevent the transcription of said transgene (Figures 2, 3). Said DNA insert may be, for example, a non-autonomous transposable element or any DNA fragment flanked by unidirected sites recognizable by a site-specific DNA recombinase. The appropriate transposase or site-specific DNA recombinase may be delivered by a viral vector which functions as a vector switch (Figures 2, 3). After expression of said vector-encoded transposase or recombinase, the catalytic activity of such an enzyme leads to excision of the DNA insert or fragment that was separating the promoter from the transgene switching on expression of the transgene (Figure 6).

Site-specific recombinases/integrases from bacteriophages and yeasts are widely used for manipulating DNA in vitro and in plants. Preferred recombinases- recombination sites for the use in this invention are the following: Cre recombinase-LoxP recombination site, FLP recombinase-FRT recombination sites, R recombinase-RS recombination sites, etc. Transposons are widely used for the discoveryof gene function in plants. Preferred transposon systems for use in the present invention include *Ac*/*Ds, En*/*Spm*, transposons belonging to "mariner" family, etc.

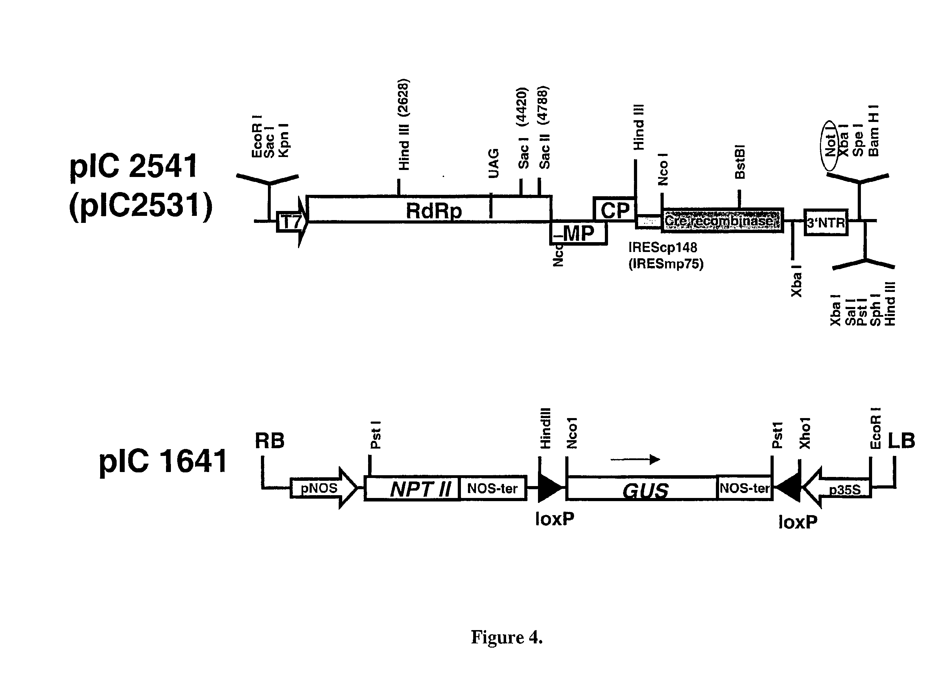

In another embodiment of this invention, the transgenic plant may carry a promoterless transgenic sequence flanked by two inverted IoxP sites (Figure 4). Such an orientation of recombination sites may lead to the inversion of the flanked DNA sequence when exposed to Cre recombinase. As a consequence of such an inversion, a promoterless gene withnos terminator will be placed from anti-sense in sense orientation towards the constitutive promoter. Example 4 exemplifies this approach using a promoterless GUS gene with a nos terminator.

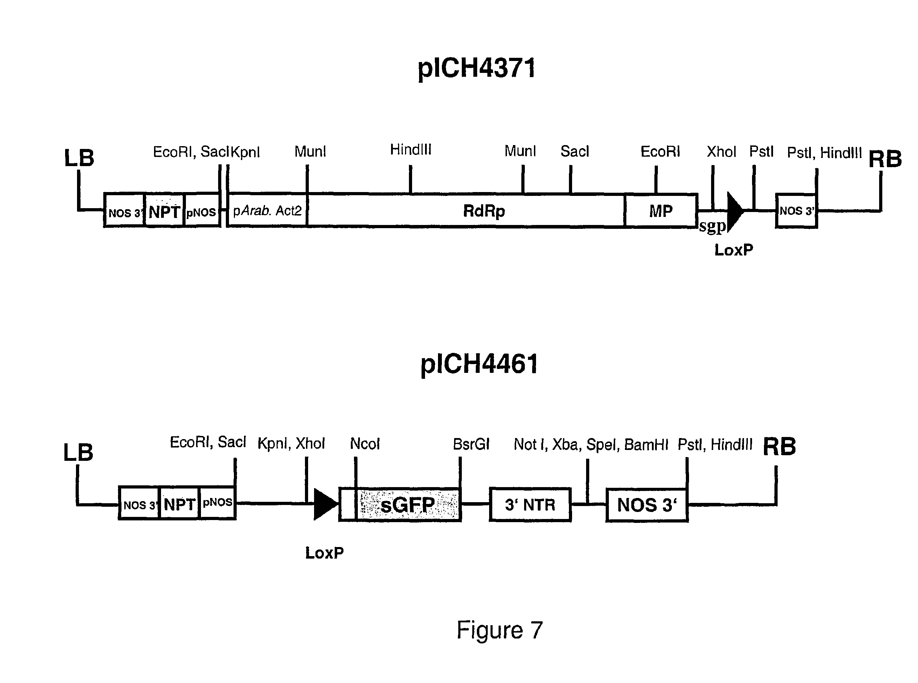

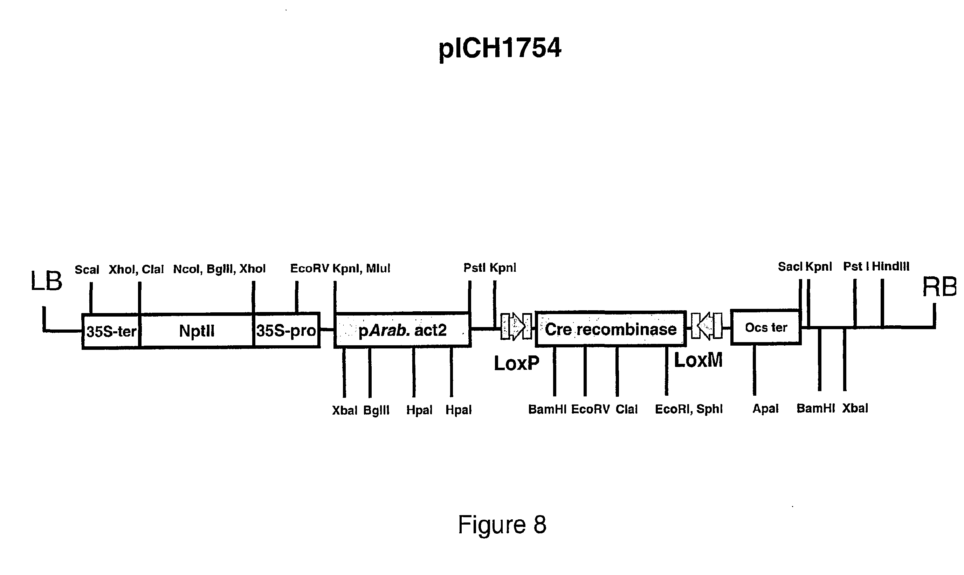

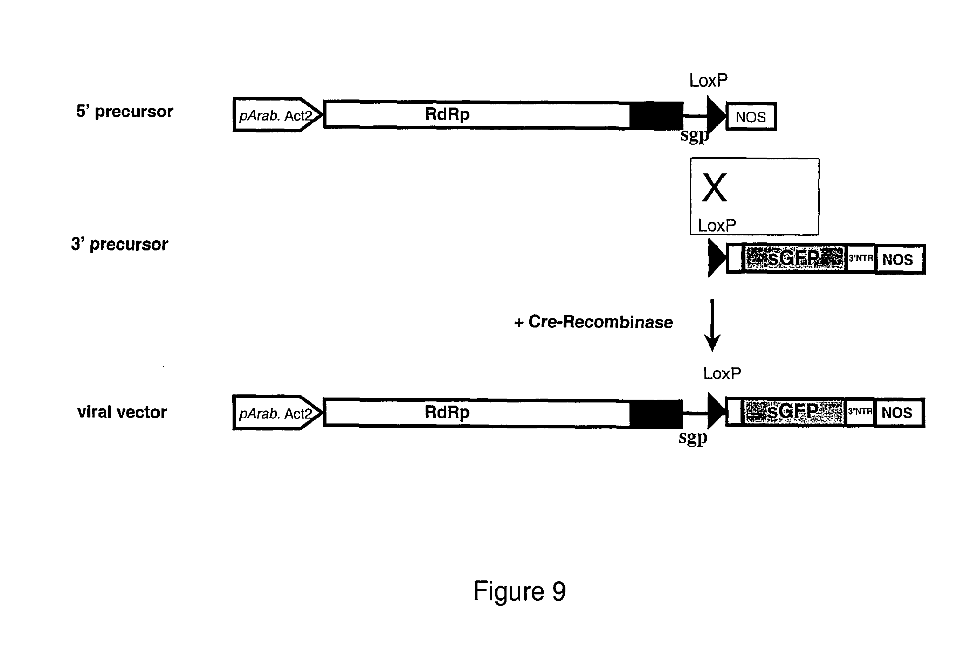

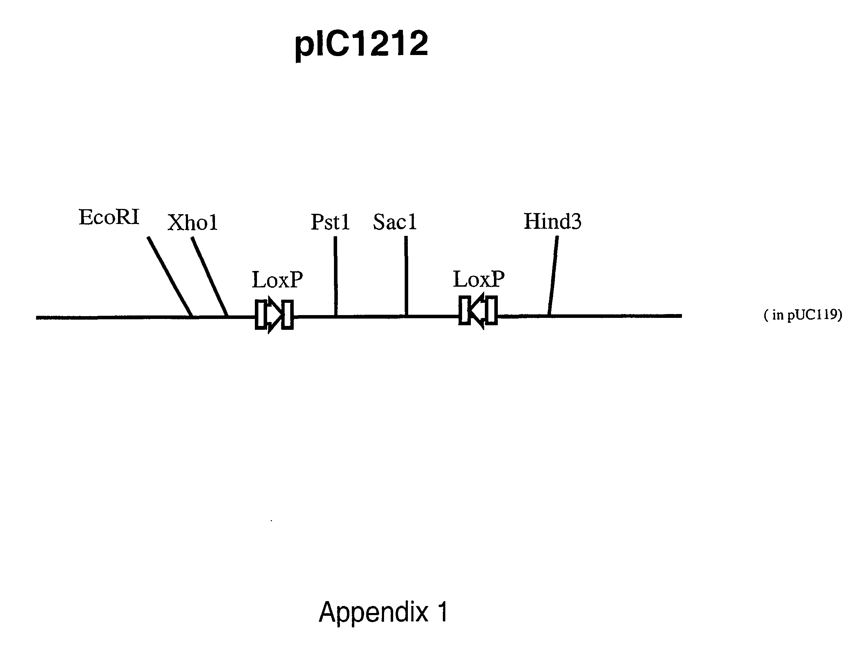

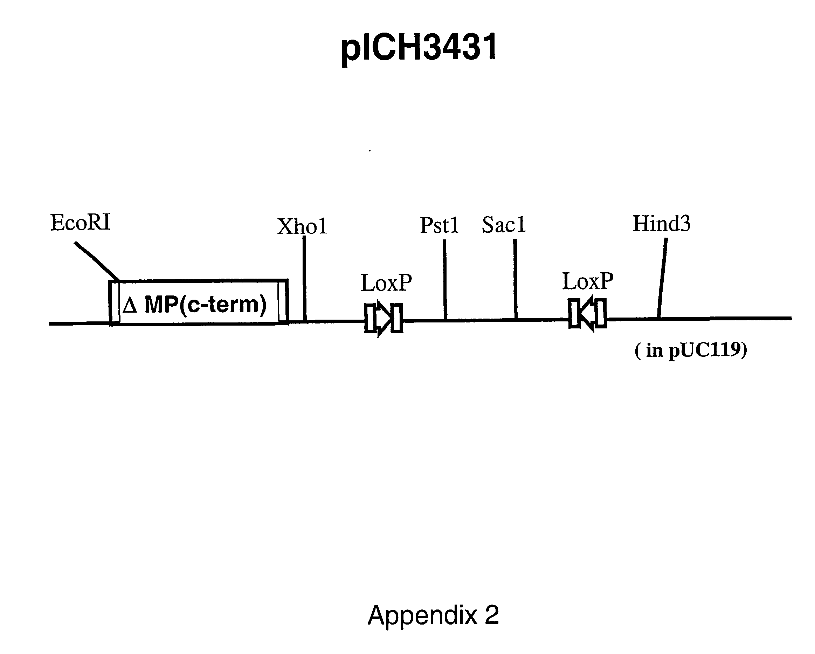

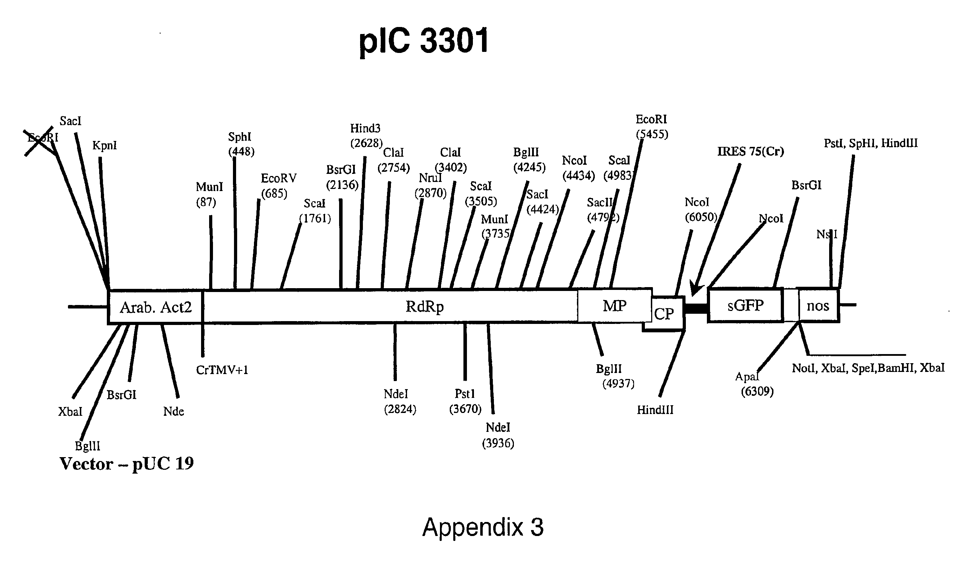

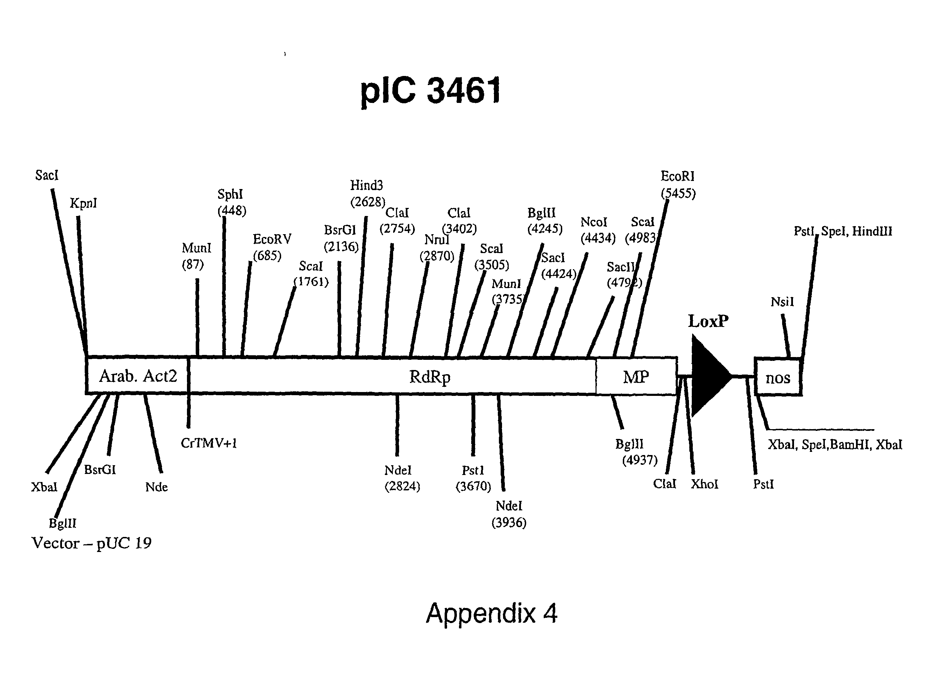

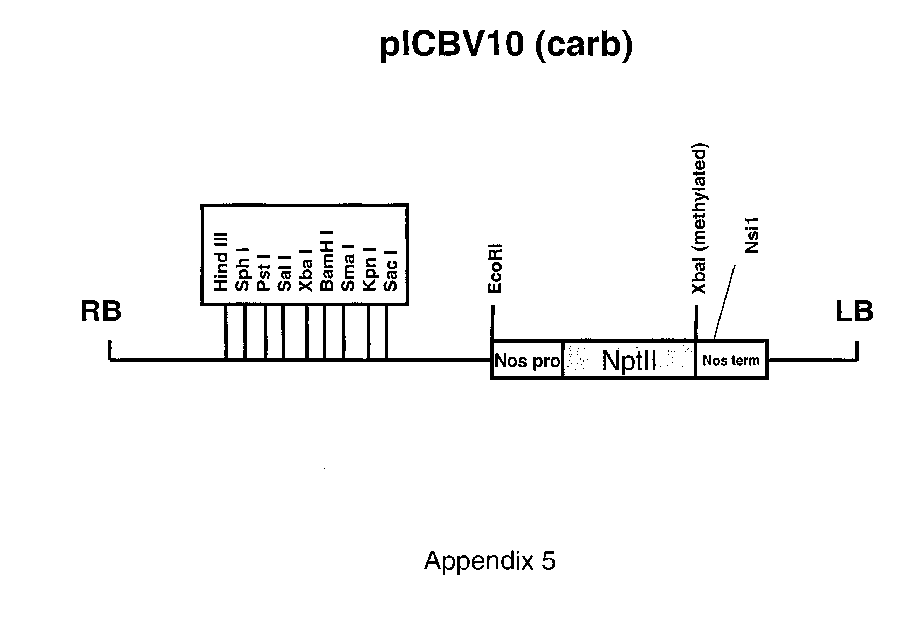

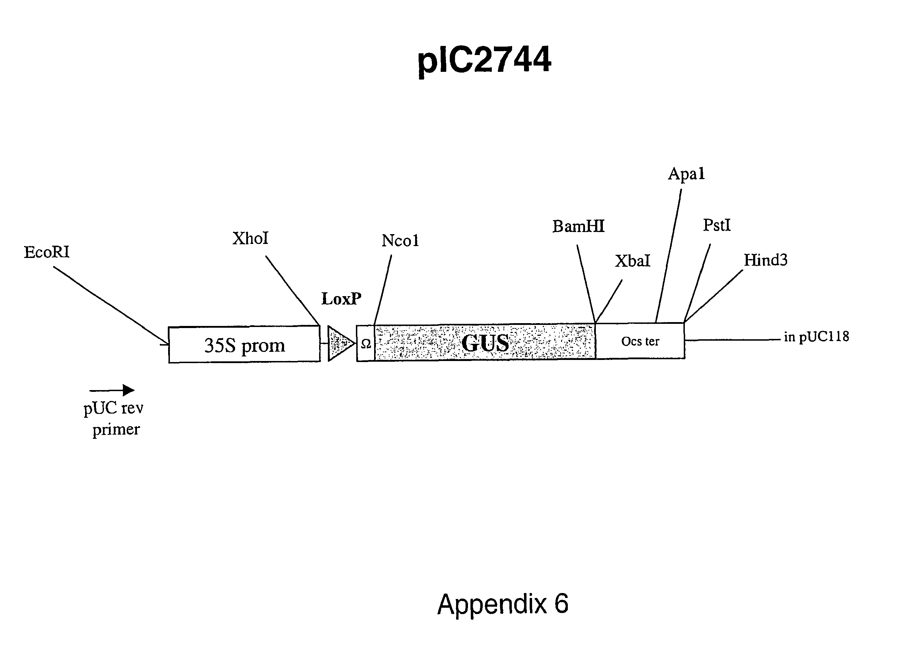

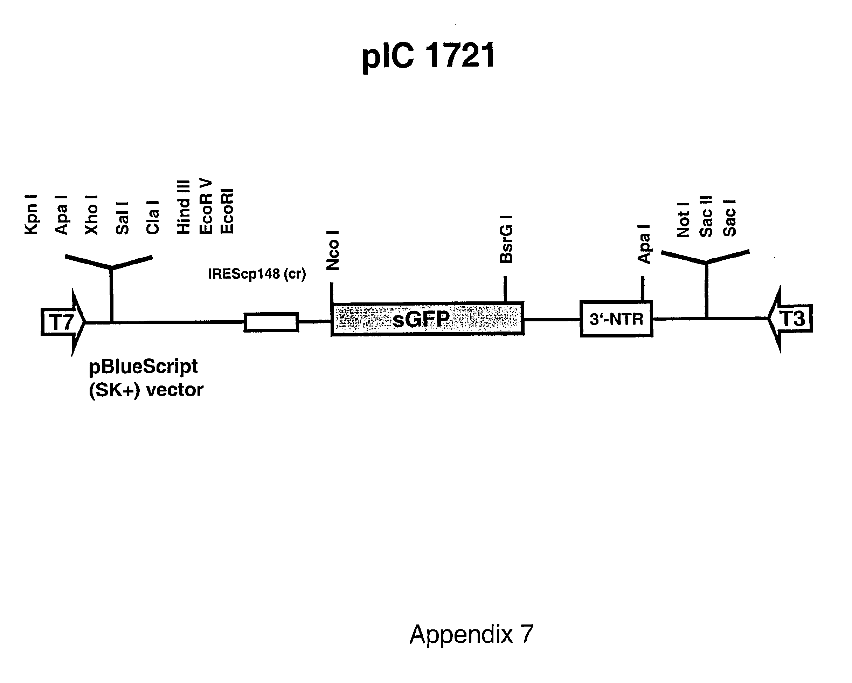

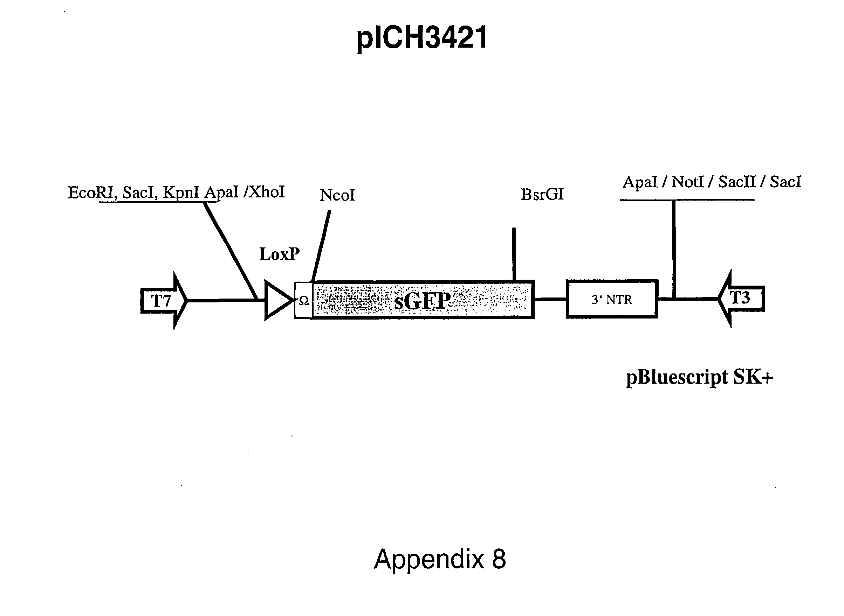

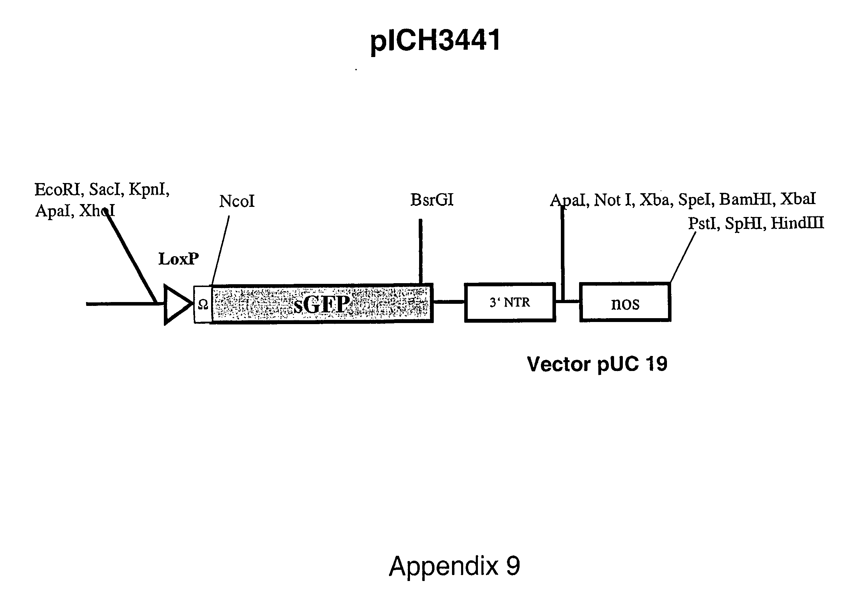

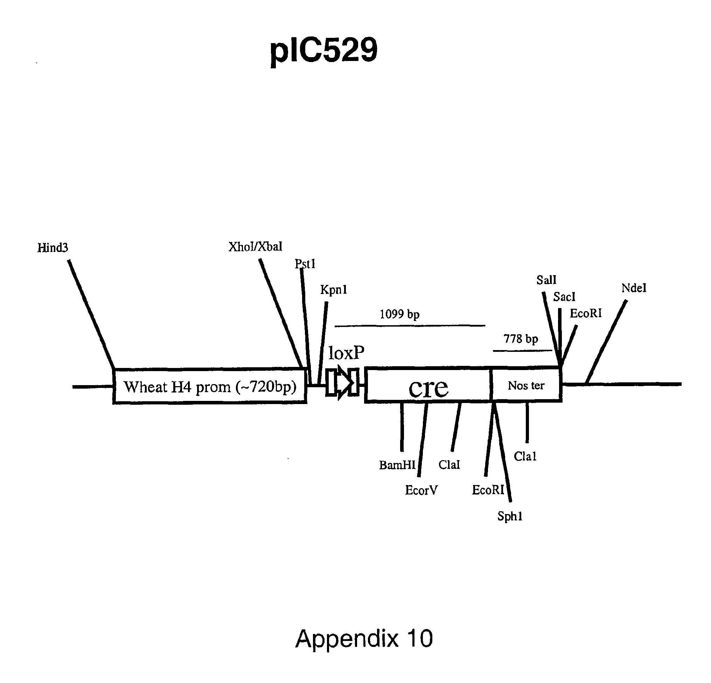

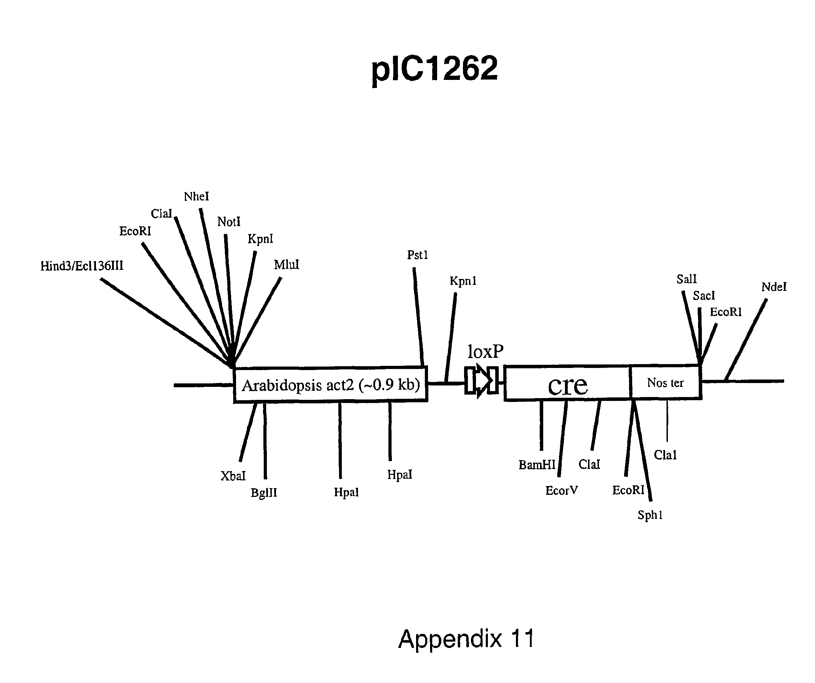

Another embodiment of this invention describes the possibility to assemble a functional viral vector construct *in vivo* in an engineered plant cell. This means required elements of a viral vector (precursors) are delivered separately with two (Figure 7) or more constructs into the plant cell. After e.g. *Agrobacterium tumefaciens* mediated delivery of such precursors into a plant cell expressing the site-specific DNA recombinase (Figure 8), site specific recombination can lead to the assembly of functional viral vector expressing transgene of interest (example 6, Figure 9).

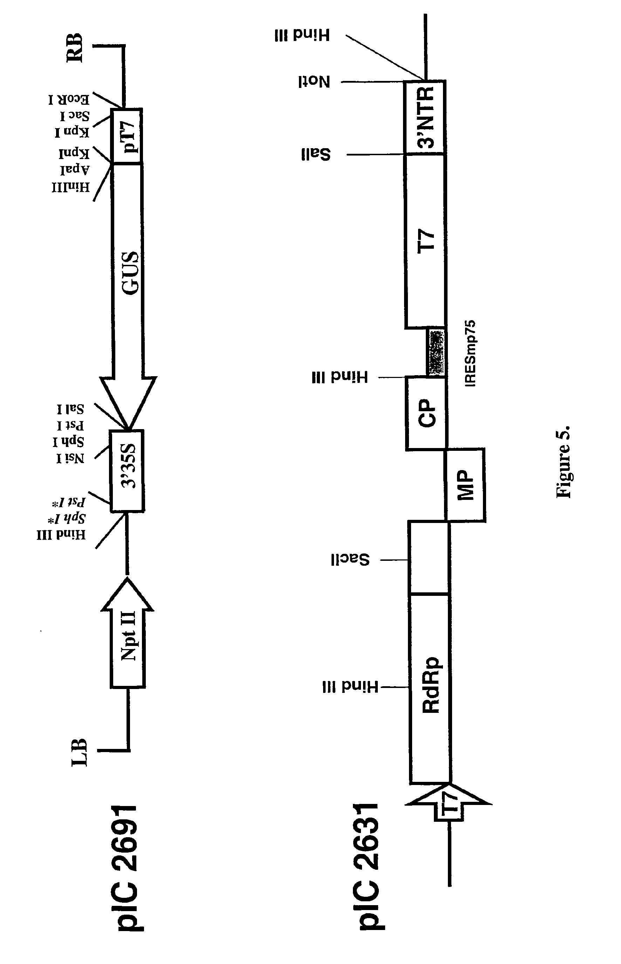

Heterologous transcription factors and RNA polymerases may also be used as transgene switches. This approach is demonstrated in Example 5 wherein a transgenic plant carries the GUS gene under control of a bacteriophage T7 promoter (see Figure 5). No GUS expression can be detected in transgenic *Arabidopsis* containing such construct as the plant RNA polymerases do not recognize prokaryotic promoters. Viral delivery of the bacteriophage T7 RNA polymerase triggers expression of the GUS gene (Figure 5).

The expression of a plant transgene that is under control of a bacteriophage promoter (e.g. T3, T7, SP6, K11) with the corresponding DNA/RNA polymerase delivered by a viral vector may be another efficient approach for the development of transgene switches contemplated in this invention. Another useful approach may be the use of heterologous or chimaeric or other artificial promoters which require heterologous or engineered transcription factors for their activation. In some cases, the existing inducible systems for transgene expression may be used. Examples are the copper-controllable (Mett et al., 1993, Proc. Natl. Acad. Sci. USA., 90, 4567-4571*)* and the ethanol-inducible gene expression systems (Caddick et al., 1998, Nature Biotech., 16. 177-180) which may be modified such that the transcription factors (ACE1 for copper-inducible or ALCR for the ethanol-inducible system) are provided in *trans* by viral delivery, thus further reducing the leakiness of the expression systems. Alternatively, heterologous transcription factors may be modified in such that no activating ligand-inducer will be required to drive the transcription factor into the active state.

Other embodiments contemplated in this invention include triggering reactions such as DNA restriction and/or DNA replication. An example of a biochemical cascade that can be triggered by restriction is a two-component system wherein a DNA sequence containing an origin of replication and being integrated into a nuclear genome is specifically excised and converted into an autosomally replicating plasmid by a rare-cutting restriction enzyme delivered by viral vector, thus triggering the cascade. Alternatively, a DNA viral vector with a modified system of replication initiation may be made operable only in the presence of a factor in a transgenic host that allows for efficient replication of the modified viral vector in question.

There are numerous reactions affecting RNA molecules that may be used as efficient triggering devices of a cascade according to the present invention. These include, *inter alia,* reactions such as RNA replication, reverse transcription, editing, silencing, or translation. For example, a DNA derived from an viral RNA vector may be reverse transcribed by a transgenic host into a DNA which in turn could participate in processes such as DNA integration into a nuclear genome or DNA-mediated mutagenesis.

Another recombinant viral switch contemplated under the invention is a process that relies on posttranslational modification of one or more transgene expression products. There are many possible implementations of such switches that could operate by controlling steps such as polypeptide folding, oligomer formation, removal of targeting signals, conversion of a pro-enzyme into an enzyme, blocking enzymatic activity, etc. For example, expression of a polypeptide from a viral expression vector may trigger a biochemical process of interest only if a genetically engineered host specifically cleaves a pro-enzyme thus converting it into an active enzyme, if a product is targeted to a particular cellular compartment because of the host's ability to cleave or modify targeting motif, or if a product is specifically mobilized due to the removal of a specific binding sequence.

The process of this invention relies on the interaction of at least two components, but multi-component systems based on interactions between more than one heterologous DNA in host nuclear genome or more than one viral transfection vectors are also contemplated. The same is true with regard to multi-component systems that involve, in addition to the above named two components (heterologous DNA or its product in a host plant and a heterologous DNA or its product in a viral vector), additional elements such as low molecular weight effectors or nucleic acids or proteins that are not integrated into a plant chromosome. Such a low molecular weight component is defined as a non-proteinaceous molecule or ion having a molecular weight of less than 5kD. The ultimate purpose of a recombinant switch system contemplated herein is an operational control of a process in a plant production system, such as biochemical pathway or a cascade of biochemical reactions of interest. A pathway or a biochemical cascade is a chain of biochemical reactions in a host production system that upon completion, yields a specific product, effect or trait.

The approaches described herein, in addition to being versatile and leakage-proof gene switches, provide an efficient production control method. The two-component process described above is in essence a "key-lock" system, whereby a company can efficiently control access to production by selling the transfection switch component.

### EXAMPLES

With regard to additional disclosure of specific vectors and constructs used in the following examples, reference is made to ANNEX A and ANNEX B.

### EXAMPLE 1

### Construction of viral vectors for plant infection, carrying the genes involved in DNA recombination: Ac transposase and Cre recombinase

Series of crTMV-based viral expression vectors carrying the genes involved in DNA recombination, were constructed according to a standard molecular biology protocols (Maniatis et al., 1982, Molecular cloning: a Laboratory Manual. Cold Spring Harbor Laboratory, New York). Detailed information concerning commonly used vectors, genes and gene fragments used in this and the following examples can be found in public domain databases. Two-step cloning strategy was used for all constructs. First, an intermediate construct was made to fuse the gene of interest (GUS) with the appropriate IRES-sequence and the 3'-nontranslated region (NTR) of the crTMV (pseudoknots and t-RNA-like structure). For the IRESmp75^{CR} and IREScp148^{CR}-fusions (Skulachev et al. 1999, Virology 263, 139-154) the gene of interest (GUS) was subcloned into the plasmid pIC766 (IRESmp75^{CR}-GUS-3'-NTR in pBS(SK+) and into the plasmid pIC751 (IREScp148^{CR}-GUS-3'-NTR in pBS(SK+), respectively. Convenient restriction sites for sub-cloning, like Nco I at the 5'-end and BamH I- or Xba I at the 3'-end of the gene of interest were introduced by PCR if necessary. DNA sequencing analysis was used to confirm the sequences of all PCR-amplified parts of the construct.

In the final step of cloning, the IRES/GUS/3'-NTR- fragment was sub-cloned further into the viral expression vector pIC797 (T7 promoter - crTMV cDNA with the GUS gene following the viral CP gene (RdRp-MP-CP-Hindlll-IRESmp228^{CR}-GUS-3'NTR)-Notl-Xbal-Spel-BamHl in pUC19) as a Hindlll/Notl fragment. For this purpose, the plasmid pIC797 was digested with Sacll and Notl, the large fragment was gel purified and ligated with the 1.3 kb SacII/HindIII fragment of the same plasmid and the Hindlll/Notl-fragment of the intermediate construct (pIC2251 for Cre recombinase). In case of the Ac-transposase a four-fragments ligation was necessary due to the presence of a Hindlll-restriction site in the coding part of the Ac gene. The final constructs (pIC1111 and pIC1123 for Ac transposase; pIC2541 and pIC2531 for Cre recombinase) are shown in Figures 2 and 4 respectively.

### EXAMPLE 2

### In vitro transcription of viral vector constructs

The plasmids pIC1111, pIC1123, pIC2541 and pIC2531 (Figures 2 and 4, respectively) were linearized by digestion with Not I restriction endonuclease. The linearized plasmids were transcribed *in vitro* as described by Dawson et al. (1986, Proc. Natl. Acad. Sci. USA., 83, 1832-1836). Quality and quantity of full-length RNA transcripts were determined by agarose gel electrophoresis (Maniatis et al., 1982, Molecular cloning: a Laboratory Manual, Cold Spring Harbor Laboratory, New York).

### EXAMPLE 3

### Activation of a transgene stably integrated in a plant genome by virus-delivered Ac transposase

The T-DNA of plasmid pSLJ744 (obtained from J. Jones, Sainsbury Laboratory, JIC, Norwich, UK) (Fig. 2) was introduced in *Arabidopsis thaliana* (Col-0) plants as descried by Bent et al., (1994, Science, 285, 1856-1860). Seeds were harvested three weeks after vacuum-infiltration, sterilised and screened for transformants on GM + 1% glucose medium (Valvekens et al., 1988, Proc. Natl. Acad. Sci. USA, 85, 5536-5540.) containing 50 mg/L kanamycin.

Rosette leaves of five weeks old *Arabidopsis* transformants were inoculated with full-length transcript-RNA as obtained in example 2 by mechanical wounding. For this purpose, the RNA was mixed with 3x GKP-buffer (50 mM glycine, 30 mM K₂HPO₄, 3% celite, 3% benthonite) and scratched gently on the upper side of the leaves. The T-DNA of plasmid SLJ744 contained a non-autonomous Ds element inserted between the CaMV 35S promoter and the GUS gene (Fig. 2). Excision of the Ds element caused by action of virus-delivered Ac transposase leads to the expression of the GUS-gene, which can be easily monitored by histochemical staining of inoculated leaves (Jefferson, 1987, Plant Mol. Biol. Reporter, 5, 387-405). Inoculated leaves were collected 9-14 days after the transfection with full-length transcript RNA. Samples were infiltrated using X-gluc solution (Jefferson, 1987, Plant Mol. Biol. Reporter, 5, 387-405). After incubation overnight at 37 °C, the leaves were destained in 70 % ethanol and examined by light microscopy. Large sectors of GUS-stained tissues were observed in primarily inoculated leaves. No GUS staining was detected in the control transgenic plants inoculated by distilled H₂O. The results are shown in Figure 6. The sectors of GUS staining are consistent with the sectors of viral infection in primarily inoculated leaves. This is evidence for the high efficiency of this approach: Ds excision and, as a consequence, GUS expression took place in all infected cells. In comparison, constant presence of Ac transposase in plants carrying a copy of the Ac transgene stably integrated in the genome leads to Ds excision sectors only in a minor fraction of the plant tissue (results not shown).

### EXAMPLE 4

### Activation of a transgene stably integrated in the plant genome by virus-delivered Cre recombinase

Two different constructs pIC2561 and pIC1641 (Figures 3 and 4, respectively) with IoxP-recombination sites were designed as targets for Cre-mediated recombination. In construct pIC2561, the GUS gene with the 3'NOS transcription termination signal is flanked by two direct IoxP-sites. This fragment was inserted between the CaMV 35S promoter and a synthetic GFP gene (sGFP). The recombination between the two IoxP sites, once exposed to virus-delivered Cre recombinase, leads to excision of the GUS gene. This event can be easily monitored by GFP expression and absence of GUS-acitivity in the inoculated leaves.

For the construction of plasmid pIC2561, the SLJ4K1 (Jones et al. 1992, Transgenic Research 1, 285-292) the GUS gene was amplified with primers carrying IoxP sites and **Cla1** (5'CCG **ATC GAT** ATA ACT TCG TAT AGC ATA CAT TAT ACG AAG TTA TAT GTT ACG TCC TGT AGA AAC CC3') and **Nco1** (5'GGC **CAT GGA** TAA CTT CGT ATA ATG TAT GCT ATA CGA AGT TAT TGC ATG CCT GCA GGT CGA TCT AGT AAC3') restriction sites were introduced at the 5' and 3' ends of the gene, respectively. Said PCR-product was digested with Cla1 and Nco1 restriction enzymes and subcloned into the Nco1- Cla1 sites of the plasmid pIC591 (pHBT:Clal/Ncol-sGFP-3'NOS). The HBT promoter (Sheen, J. 1995, EMBO J., 12, 3497-3505) (not functional in *Arabidopsis*) of this intermediate construct was replaced by the CaMV 35S promoter by ligating together its gel-purified large Hindlll/Klenow - Cla1 fragment with the 1.4 kb EcoR1/Klenow - Cla1 fragment of (35S promoter) of SLJ4K1. Functional clones were determined in microprojectile co-bombardment experiments with DNA of pIC1422 (cre recombinase under control of HBT promoter). For further subcloning into the binary vector pICBV1 (proprietary development of Icon Genetics AG, Munich, Germany, however, any other binary vector is suitable as well), the pICBV1-DNA was digested with EcoRI and Ecl136II restriction enzymes, gel-purified and ligated with the large Xho1/Klenow - EcoR1 fragment (p35S:-IoxP-GUS-3'OCS-IoxP-sGFP-3'NOS) of said functional intermediate clone. The T-DNA region of the final construct pIC2561 is shown in Figure 3.

The second construct carrying the GUS gene flanked by two inverted IoxP sites is shown in Figure 4. To make this construct, two PCR primers (5'CTG **AAG CTT** ATA ACT TCG TAT AGC ATA CAT TAT ACG AAG TTA TA**C CAT GG CTG CAG** ATA ACT TCG TAT3' and 5'GC**C TCG AGA** TAA CTT CGT ATA ATG TAT GCT ATA CGA AG TT AT**C TGC AGC CAT GG**T ATA ACT TCG TA3') with 18 bb of complementary 3' ends were designed, annealed and filled in with the Klenow fragment of DNA polymerase I.

The final DNA fragment contained two inverted IoxP sites separated by Pst1, Nco1 and flanked by Xho1 (from the Pst1 side) and Hind111 restriction sites. After a Xho1-Hind111 digestion, the fragment was ligated with large Xho1-Hind111 fragment of pSLJ4D4 (Jones et al., 1992, Transgenic Research, 1, 285-292). The resulting plasmid was digested with Pst1 - Nco1, gel-purified and ligated with the 2.6 kb Nco1 - Pst1 fragment of pSLJ4D4.

As the final step of cloning, the whole cassette (CaMV p35S-IoxP-3'nos-GUS-IoxP) was subcloned into the binary vector pBIN19 (Bevan, M. 1984, Nucl. Acid Research, 12, 8711-8721) as Hindlll/EcoRI-fragment. The T-DNA of this construct (pIC1641) is shown in Figure 4. Transgenic *Arabidopsis* lines were obtained by *Agrobacterium tumefaciens* mediated transformation according to the modified vacuum infiltration protocol of Bent et al. (1994, Science, 265, 1856-1859). The presence of the transgene in segregating T1-population was confirmed by PCR-analysis.

Transcription of viral a cDNA clone (pIC2531) and inoculation of transgenic *Arabidopsis* lines with viral RNA was performed as described in examples 2 and 3, respectively.

### GFP and GUS detection

A LEICA stereo fluorescent microscope system was used to monitor GFP expression (excitation at 450-490 nm, emission at 500-550 nm). The sGFP used in our experiments can be excited by blue and UV-light. GUS detection was performed as described in example 3.

### EXAMPLE 5

### Activation of a transgene stably integrated in a plant genome by virus-delivered T7 RNA polymerase

### Construction of the vectors

The binary vector for plant transformation with the GUS gene reporter under control of the T7 promoter was made as follows. The gel-purified 2 kb BssH11/T4 polymerase - Sal 1 fragment of pIC057 carrying the T7 promoter-GUS gene construct was ligated with Sma1 - Sal1 digested expression vector pIC056, adding the 35S transcription termination signal to the 3' end of the GUS gene. The resulting construct pIC2641 was digested with Sac1 and Xho1, gel-purified from the vector backbone and ligated with Sac1/Sal1 digested pBIN19. The final construct pIC2651 (Figure 5) was used for the *Arabidopsis* transformation as described above. The viral vector expressing T7 polymerase was made as follows. The plasmid pIC2603 was digested with Sph1 and Sal1 and the gel purified 2.8 kb fragment carrying the T7 polymerase gene was ligated with the large Nco1-Sal1 fragment of pIC1018. Resulting plasmid pIC2621 has the T7 gene flanked by the IRESmp75^{CR} at its 5' end and by the 3' nontranslated region (NTR) of crTMV at its 3' end. The final cloning step included the ligation of the small Hind111 - Not1 fragment of pIC2621 with the large Sac11-Not1 and small Sac11-Hind111 fragments of pIC1087. The final construct pIC 2631 (Figure 5) containing the T7 polymerase gene in a crTMV viral vector was used for transcription and plant transfection as described in examples 2 and 3, respectively.

### EXAMPLE 6

### Activation of a transgene from viral amplicon precursors

### Construction of the vectors

In order to introduce LoxP-sites recognized by Cre recombinase into a basic construct, IPCR was performed with primers containing LoxP-sites in opposite orientation flanked by convenient restriction sites (primer 1: 5'-TATCTGCAGG AGCTCATAAC TTCGTATAAT GTATGCTATA CGAAGTTATA AGCTTCTGGC CGTCGTTTTA C-3'; primer 2: 5'-CTCCTGCAGA TAACTTCGTA TAATGTATGC TATACGAAGT TATCTCGAGG AATTCGGCGT AATCATGGTC A-3'). These primers were annealed to the multi-cloning site of the pUC119 vector in order to amplify the whole plasmid in an IPCR-reaction. Overlapping sequences of the primers contained a Pst I-restriction site. After restriction of the IPCR-product with Pst I and religation, the intermediate construct pICH1212 (Appendix 1) was obtained.

The Xho1-EcoR1 fragment of MP-gene containing a translation stop codon 25 AA before the natural translation termination signal was ligated with Xho1-EcoR1 large fragment of pICH1212. In the resulting construct pICH3431 (Appendix 2) the 3'-MP-part is located next to a LoxP-site. To fuse this MP-LoxP element to the 5'-part of a MP-gene in a vector, which contains also the *Arabidopsis* Actin 2-promoter and the RdRp-polymerase, the MP-LoxP element from pICH3431 was subcloned as EcoRI-EcI136II fragment into the plasmid pICH3301 (Appendix 3) cut with EcoRI and NotI, resulting in the plasmid pICH3461 (Appendix 4). The Notl restriction site was treated with Klenow fragment of DNA polymerase 1 before subcloning. The Kpnl-Xhol and Xho-Hindlll fragments at 5'-end of the resulting vector were further used for cloning into the Kpn1 and Hindlll-treated binary vector pICBV10 (T-DNA region of pICBV10 is shown in Appendix 5) in a three fragment ligation reaction. The final construct pICH4371 is depicted in Figure 7.

For making a 3'-end of the viral vector precursor, an Xhol-Ncol fragment containing a LoxP site next to an Ω-leader-sequence from construct pICH2744 (Appendix 6) was subcloned into plasmid pICH1721 (Appendix 7) to fuse the LoxP-site/Ω-leader-sequence-element to the 5'-end of the sGFP-gene which was flanked by a 3'NTR-sequence at the 3'-end (construct pICH3421, Appendix 8). In order to add a nopaline synthase transcription termination signal to this ORF, plasmid pICH3421 was cut by KpnI and NotI and the resulting small fragment was cloned into the plasmid pICH3232 resulting in construct pICH3441 (Appendix 9). For the *Agrobacterium* tumefaciens-mediated delivery this 3'-end of the viral precursor vector was further subcloned into the binary vector pICBV10 (Appendix 5) as KpnI/HindIII fragment. The final construct pICH4461 is shown in Figure 7.

The Cre recombinase construct pICH529 (wheat histone H4 promoter-LoxP-Cre recombinase-NOS terminator, see Appendix 10) was modified to clone the Cre recombinase into the binary vector used for obtaining nuclear transformants of Nicotiana. First the wheat H4 promoter was replaced by the Actin2 promoter from *Arabidopsis* by subcloning an EcI136II/Pst I- fragment from construct pIC04 (*Arabidopsis* Actin2-promoter without intron, not shown) into pICH529 digested with HindII (blunt) and PstI. This resulted in construct pICH1262 (Appendix 11). In order to replace the NOS-terminator by the OCS-terminator flanked at its 5'-end by a LoxM recombination-site, the OCS-terminator was PCR-amplified from plasmid pICH495 (NOS promoter-BAR-gene-OCS terminator, not shown) and further subcloned as SphI/SacI-fragment into the plasmid pICH1262 , producing the construct pICH1321 (not shown). The sequence of the forward primer (5'-CGGCATGCAT AACTTCGTAT AATCTATACT ATACGAAGTT AGGATCGATC CTAGAGTCCT GC-3') used for this amplification, included the Sphl-restriction site and the LoxM-recombination site. The Sacl-restriction site at the 3'-end of the PCR-product was introduced by the sequence of the reverse primer (5'-CGGAGCTCGT CAAGGTTTGA CCTGCACTTC-3'). Finally, the resulting construct (Actin2 promoter-LoxP-Cre recombinase-LoxM-OCS terminator) was further subcloned into the binary vector pIC00015 as Notl/Sacl-fragment, resulting inconstruct pICH1754 (Figure 8). To clone this fragment into the binary vector it was necessary to fill in the Notl-site of the fragment and the EcoRl-site in the polylinker of the binary vector.Transformation of tobacco leaf discs

Transgenic Nicotiana lines (species *tabacum* and *benthamiana*), containing T-DNA of pICH1754, were obtained by *Agrobacterium* -mediated transformation of leaf discs as described by Horsch et al., (1985, Science, 227, 129-131). Leaf discs were incubated for 30 min with Agrobacterium strain GV3101 transformed with the construct pICH1754. After three days of incubation on medium (MS-medium 0.1 mg/l NAA, 1 mg/l BAP) without selective agent, selection of transformants was performed on the same MS-medium supplemented with 100 mg/L Kanamycin. In ordero reduce the growth of Agrobacterium, the medium was also supplemented with 300 mg/L carbenicilin and 300 mg/L cefataxime. Regenerants were incubated on selective MS-medium without hormones supplemented with the same concentration of the selective agents to induce the rooting. The presence of the transgene in segregating T2-populations was confirmed by PCR-analysis.

### Delivery of viral vector precursors by agro-infiltration

The agroinfiltration of transgenic tobacco plants was performed according to a modified protocol described by Yang et al., 2000, Plant Journal, 22(6), 543-551. *Agrobacterium tumefaciens* strain GV3101 transformed with individual constructs (pICH4371 and pICH4461) was grown in LB-medium supplemented with Rifampicin 50 mg/l, carbencilin 50 mg/l and 100 µM acetosyringone at 28 °C. Agrobacterium cells of an overnight culture (5 ml) were collected by centrifugation (10 min, 4500 g) and resuspended in 10 mM MES (pH 5.5) buffer supplemented with 10 mM MgSO₄ and 100 µM acetosyringone. Bacterial suspension was adjusted to a final OD₆₀₀ of 0.8. In case of delivery of several constructs agrobacterial clones carrying different constructs were mixed before infiltration.

Agroinfiltration was conducted on near fully expanded leaves that were still attached to the intact plant. Bacterial suspension was infiltrated with a 5 ml syringe. By infiltrating 100 µl of bacterial suspension into each spot (typically 3-4 cm² of infiltrated area) eight to 16 spots separated by veins could be placed in a single tobacco leaf. After infiltration plants were further grown under greenhouse conditions at 22 °C and 16 h light.

Sixteen days after infiltration, leaves of transgenic tobacco plants (pICH1754, *Nicotiana tabacum)* infiltrated with construct pICH4371 and pICH4461 showed growing sectors of strong GFP-expression which could be observed under UV-light on intact plants. No GFP-expression was visible on leaves of non-transformed tobacco infiltrated with the same Agrobacterium suspension mix.

### ANNEX A

### Vector system for plants

### FIELD

This annex relates to a vector capable of amplification and expression and/or suppression of a gene in a plant, as well as uses thereof, and a method and pro-vector for generating said vector.

### BACKGROUND

Vectors for genetic engineering of plants are highly desirable for the production of proteins, for endowing a host plant with a new trait, for suppressing a gene of the host plant, or for determining the function of a gene, notably a gene determined by genomic.

Vectors, notably viral vectors, for the genetic engineering of plants are already known. These must be capable of infection, amplification and movement (both cell-to-cell and long-distance) in a plant in addition to having at least one sequence for gene expression or suppression.

Prior art vectors rely on subgenomic promoters as transcriptional elements. A subgenomic promoter has the effect that, in a transfected plant cell, transcription of a vector nucleic acid sequence starts in part at said subgenomic promoter to generate a shorter RNA so that translation of a gene downstream from said promoters by the plant translation machinery is enabled. Translation may then proceed cap-dependent. Such multiple transcriptions are kinetically disadvantageous because of waste of replicase capacity.

Such vectors have a number of further shortcomings. The introduction of a virus subgenomic promoter into a vector sequence makes said sequence longer and thus less efficient.

Moreover, the presence of several identical or similar subgenomic promoters which are well adapted to transcription in the host gives rise to frequent recombination events and instability with loss of sequence portions. On the other hand, if significantly different subgenomic promoters are used, recombination may be suppressed but such promoters may be too different to be effectively recognized by the transcription system, which means loss of efficiency. Moreover, vectors are usually highly integrated entities with several interdependent functional elements or genes tightly packed into a sequence. This is the reason why the operability of a vector for certain heterologous genes or the like is somewhat idiosyncratic and frequently gives unpredictable results, notably in terms of infectivity and expression. Further, the available sequence space for promoters is usually constrained if sequence overlaps with upstream genes are present.

Therefore, it is an object to provide a novel vector for plant genetic engineering which is capable of efficient and stable operation in a host plant. It is a further object to provide a vector which is capable of high-level expression of a gene in a plant.

It has been surprisingly found that these objects can be achieved with a vector capable of amplification and expression of a gene in a plant comprising a nucleic acid having a sequence for at least one non-viral gene to be expressed and having or coding for at least one IRES element necessary for translation of a genes downstream thereof.

It has been previously suggested (WO 98/54342) to use a plant IRES element in a recombinant DNA molecule that has merely the function of gene expression (after integration into the host genome). However, the expression level is low. The exact reasons for this low expression level are not known. In any event, expression is limited to the very plant cells transformed, thus the overall efficiency in whole plants is extremely low.

It has been surprisingly found that it is possible to construct a plant vector which, when introduced into a plant cell, has not only the capability of gene expression but which has several additional functions which are all required for amplification arid spreading throughout the plant so that the overall efficiency is extremely high. These functions comprise infection, amplification, cell-to-cell movement and long-distance movement. It is surprising that the required high degree of integration of functional and structural elements on a vector does not impair gene expression from said vector.

The IRES element of said vector can be located upstream of said non-viral gene to be expressed for directly supporting its translation. Alternatively, said IRES element may indirectly support the translation of said gene to be expressed by directly supporting the translation of another gene essential for a function of said vector selected from the group of infection, amplification and cell-to-cell or long-distance movement of said vector.

It is a further object to provide a vector which is capable of the effective suppression of a gene in a plant. This object has been achieved by a vector capable of amplification in a plant comprising a nucleic acid having or coding for at least one IRES element necessary for translation of a gene required for amplification of said vector and located downstream of said IRES element, said vector further comprising at least a portion of a sequence of the host plant genome in an anti-sense orientation for suppressing a gene of the host plant.

Further preferred embodiments are defined in the subclaims.

Here, the first plant expression and amplification vectors based on plant active translational (IRES) elements are described. Existing IRES elements isolated from animal viruses do not support translation in plant cells. Therefore, knowledge accumulated in animal expression system is not applicable to plants. Animal IRES elements have never been tested for other functional properties, such as residual promoter activity, so this annex discloses the first bona fide cases of gene expression in plants relying exclusively on translation rather than on transcription with a subgenomic promoter necessary for expression of a gene downstream thereof.

The vectors described allow preferably for regulation and preferential expression of a gene of interest in a plant by suppressing cap-dependent translation. In another preferred embodiment, very short homologous or artificial IRES elements are used, thus adding to the stability of the resulting vectors.

A preferred advantage of this strategy is that IRES sequences can be inserted upstream or downstream of viral gene(s) (e.g. the coat protein gene of tobacco mosaic virus such that translation of downstream foreign gene(s) or the viral gene(s), respectively, may occur via cap-independent internal ribosome entry pathway. Thus, said cap-independent translation of foreign gene(s) will occur from bicistronic or/and polycistronic RNAs.

### General Problem Situation and Definitions

Upon infection of a plant with a virus the early events of viral infection (entry and genome uncoating) occur. Then the virus must engage in activities that enable its genome to be expressed and replicated. The viral genome may consist of one (monopartite) or more (multipartite) RNA or DNA segments, and each of these segments may under certain conditions be capable of replicating in the infected cell. A viral replicon has been defined as "a polynucleotide of viral sequences that is replicated in host cells during the virus multiplication cycle" (Huisman et al., 1992, "Genetic engineering with plant viruses,", T.M.A.Wilson and J.W.Davies eds.,1992, CRC Press, Inc.). In this invention we use the term "amplification-based expression system" to designate either a full-length viral genome or any fragment of viral RNA or DNA that (i) contains and is able to express foreign sequences, non-native for the wild-type parental virus (ii) replicates either by itself or as a result of complementation by a helper virus or by a product of the transgenic plant host. The terms "amplification-based expression system" and "recombinant viral vector" are closely similar. These systems represent a recombinant nucleic acid containing additional sequences, homologous (native) or foreign, heterologous (non-native) with respect to the viral genome. The term "non-native" means that this nucleic acid sequence does not occur naturally in the wild-type genome of the virus and originates from another virus or represents an artificial synthetic nucleotide sequence. Such an amplification-based system derived from viral elements is capable of replicating and, in many cases, dell-to-cell as well as long-distance movement either in a normal or/and in a genetically modified transgenic host plant In the latter case the transgenic plant should complement the viral components of a vector which may be deficient in a certain functions, i.e. the product(s) of a transgene essential for vector replication and/or expression of its genes or long-distance transport should be provided by the transgenic plant

Plant virus amplification-based vectors based on the monopartite (e.g. tobacco mosaic virus, TMV) or multipartite (e.g. members of *Bromoviridae* family) genome have been shown to express foreign genes in host plants (for review, see "Genetic engineering with plant viruses", T.M.A.Wilson and J.W.Davies eds.,1992, CRC Press, Inc.).

The majority (about 80%) of known plant viruses contains plus-sense single-stranded RNA (ssRNA) genomes that are infectious when being isolated from the virions in a form of free RNA. This means that at the first step of the virus replication cycle, genomic RNA must be translated in order to produce the virus-specific RNA-dependent RNA polymerase (replicase) that is absent from uninfected plant cells and, therefore, is essential for viral RNA replication (for review, see Y. Okada ,1999, Philosoph. Transact. of Royal Soc., B, 354, 569-582). It should be mentioned that plus-sense ssRNA viruses differ in translation strategies used for genome expression: the genomes of so called picoma-like viruses represent a single continuous open reading frame (ORF) translated by the ribosome into a large polyprotein which is then proteolytically processed into functionally active virus-coded proteins. The virus-specific proteinase(s) are involved in polyprotein processing. A second peculiar feature of picorna-like viruses is that their genomic RNA contains, instead of cap structure, a small viral .. protein covalently linked to the 5'-end of the genome.

Herein, we most preferably focus on viruses of the so-called Sindbis-like superfamily that comprises many plant viruses, in particular, more than a dozen of viruses belonging to the genus *Tobamovirus* (for review, see A.Gibbs, 1999, Philosoph. Transact. of Royal Soc., B, 354. 593-602). The technology ensures cap-independent and viral promoter independent expression of foreign genes.

The genome of tobamoviruses (TMV U1 is the type member) contains four large ORFs. The two components of the replicase (the 130-kDa and its readthrough 183-kDa proteins) are encoded by the 5'-proximal region of the genomic RNA and are translated directly from genomic RNA. The 3'-terminal 15 nucleotides of the 180-kDa protein gene of TMV U1 overlap with the ORF coding for the 30-kDa protein responsible for cell-to-cell movement of TMV infection (movement protein, MP). In TMV U1 this gene terminates two nucleotides before the initiation codon of the last gene which encodes the 17-kDa coat protein (CP) located upstream of the 3-proximal nontranslated region (3'-NTR) consisting of 204 nucleotides (in TMV U1).

Translation of RNA of tobamoviruses occurs by a ribosome scanning mechanism common for the majority of eukaryotic mRNAs (for reviews, see Kozak, 1989, J. Mol. Biol. 108, 229-241; Pain, 1996 ; Merrick and Hershey,1996, In "Translational control", eds. Hershey, Matthews and Sonenberg, Cold Spring Harbour Press, pp. 31-69; Sachs and Varani, 2000, Nature Structural Biology 7, 356-360). In accordance with this mechanism, structurally polycistronic tobamovirus RNA is functionally monocistronic, i.e., only the 5'-proximal ORF encoding the replicative proteins (130-kDa protein and its readthrough product) can be translated from full-length genomic RNA (reviewed by Palukaitis and Zaitlin,1986, In "The Plant Viruses", van Regermortel and Fraenkel-Conrat eds., vol.2, pp.105-131, Plenum Press, NY). It should be emphasized that the 68-nucleotide 5'-terminal nontranslated leader sequence of TMV U1 termed omega (Ω) has been shown to play the role of an efficient translational enhancer stimulating the translation of the 5'-proximal ORF.

The 5'-distal MP and CP genes are translationally silent in full-length TMV U1 RNA, however, they are translated from separate mRNAs referred to as subgenomic RNAs (sgRNA). Apparently, the tobamovirus sgRNAs are transcribed from negative-sense genomic RNA and share a common 3'-terminus. The expression of TMV genes that are translated from sgRNAs is regulated independently, both quantitatively and temporarily: the MP is produced transiently during early steps of infection and accumulates to relatively low levels (about 1 % of total plant protein), whereas the CP constitutes up to 70% of total plant protein synthesis and the CP can accumulate up to 10% of total cellular protein (Fraser, 1987, In "Biochemistry of virus-infected plants", pp.1-7, Research Studies Press Ltd., Letchworth, England).

It is clear that production of each sgRNA is controlled by different *cis*-acting sequences termed "subgenomic mRNA promoter" (sgPR). Generally, this term indicates the region of the viral genome (presumably in a minus-sense RNA copy) that can be recognized by the replicase complex to initiate transcription from the internally located sgPR sequence to produce sgRNA. However, for convenience, by the term "subgenomic promoter" we conventionally mean a nucleotide sequence in plus-sense viral RNA that is usually located upstream of the coding sequence and the start point of sgRNA and which is functionally involved in the initiation of the sgRNA synthesis. However, it should be taken into consideration that some viral sgPRs are located not only upstream of the controlled viral gene, but can even overlap with this gene (Balmori et al., 1993, Biochimie (Paris) 75, 517-521).

Each sgPR occupies a different position in the TMV genome. None of the sgPRs of TMV has been precisely mapped, but the 250 nucleotides upstream of the CP gene have been shown to promote synthesis of the CP sgRNA (Dawson et al., 1989, Virology 172, 285-292).

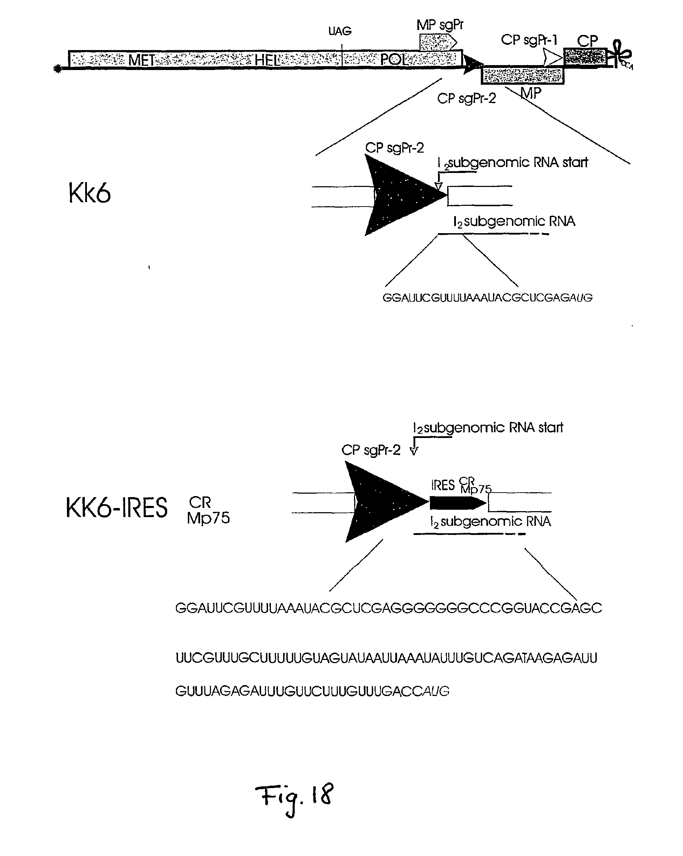

Lehto et al. (1990, Virology 174, 145-157) inserted in the TMV genome (in front of the MP gene) sequences (253 and 49 nucleotides) preceding the CP gene in order to estimate the size of the CP sgPR. The insertion did not remove the native MP sgPR, but separated it from the MP ORF. The mutant (called KK6) with an inserted 253nt promoter region replicated stably and moved systemically over the infected plant. It is not unexpected that in the KK6 mutant the insertion changed the length of the MP sgRNA leader (Lehto et al., 1990, Virology 174, 145-157) (see Fig. 18). The KK6 MP sgRNA leader was 24 nucleotides compared to 9 b.p. for the CP sgRNA.

By contrast, the mutant with an inserted 49-nt fragment of the promoter region replicated only transiently before being overtaken by a progeny of wild-type virus with the insert deleted. In addition, it has been shown (Meshi et al., 1987, EMBO J., 6, 2557-2563) that production of the CP sgRNA was reduced when the 96-nt region derived from CP sgPR was used. It is concluded that the 49-96nt sequences upstream of the CP gene did not contain the entire sgPR of the TMV U1 CP gene, whereas the 250-nt sequence included complete sgPR.There is little information about the structure and mapping of sgPR controlling the expression of the TMV MP gene. Because the putative MP sgPR sequence overlaps with the 183-kDa replicase protein, the mutational analysis of the MP sgPR was complicated. Preliminary results of W. Dawson and co-workers reported recently delineated the boundaries of the minimal and full MP sgPR of TMV U1 (Grdzelishvili et al., 2000, Virology 276, in press). Computer folding of the region upstream of the MP gene reveals two stem-loop structures, located 5'-proximally to the 75-nt region preceding AUG codon of the MP gene.

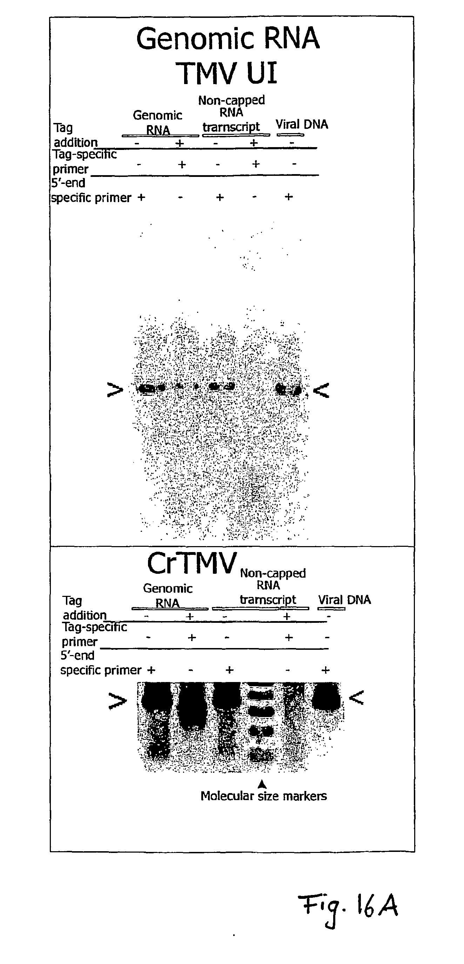

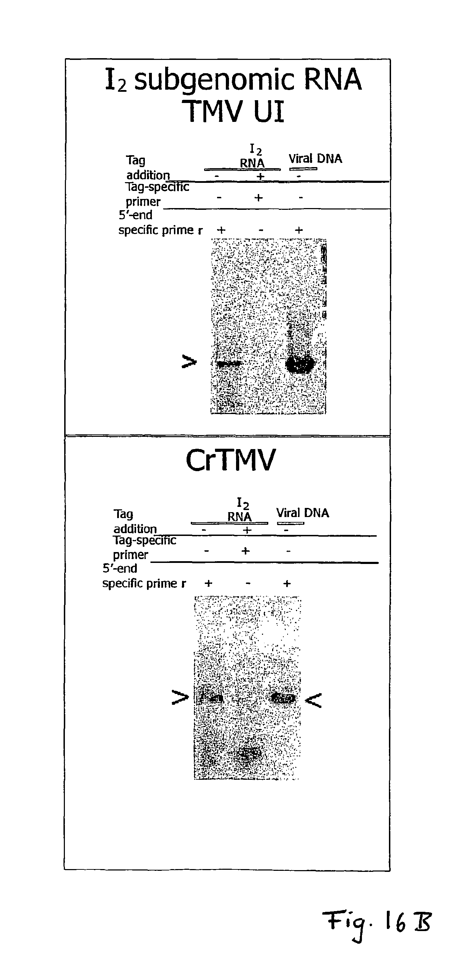

It is assumed that in contrast to genomic RNA and the CP sgRNA, the sgRNA of the MP gene (so called I₂ sgRNA) is uncapped (for review see: Okada, 1999, Philosoph. Transact. Of Royal Soc., B, 354, 569-582). The present invention provides the results confirming the absence of the cap-structure in I₂ sgRNAs of both TMV U1 and crTMV (Fig. 16).

It has been shown by W. Dawson with colleagues that an important factor affecting the expression of a foreign gene from the vector virus is the position of the foreign gene relative to the 3'-terminus of viral genome: the efficiency of expression increased dramatically when the gene was placed closer to the 3-terminus (Culver et al., 1993, Proc. Natl.. Acad. Sci. USA 90, 2055-2059). The highest expressed gene is that of the CP which is adjacent to the 3'-NTR that consists (in TMV U1 RNA) of three pseudoknots followed by a tRNA-like structure. It was suggested (Shivprasad et al., 1999, Virology 355, 312-323) that the proximity of the gene to the pseudoknots rather than to the 3-terminus was the main factor increasing expression of the foreign gene. Many important aspects of the TMV sg PRs structure were clarified due to the efforts of W. Dawson's group, however, the general conclusion of these authors was that "we are still in the empirical stage of vector building" (Shivprasad et al, 1999, Virology 355, 312-323).

The above shows that the synthesis of sgRNAs is essential for expression of the 5'-distal genes of TMV genome, since these genes are translationally silent in full-length RNA. The mechanism of gene autonomization by subgenomization can be regarded as a strategy used by TMV in order to overcome the inability of eukaryotic ribosomes to initiate translation of the 5'-distal genes from polycistronic mRNA. According to the traditional ribosome scanning model (Kozak, 1999, Gene 234, 187-208), the internal genes of a polycistronic eukaryotic mRNA are not accessible to ribosomes.

Recently, we have isolated a crucifer infecting tobamovirus (crTMV) from *Oleracia officinalis* L. plants. A peculiar feature of crTMV was its ability to infect systemically members of *Brassicaceae* family. In addition, this virus was able to systemically infect plants of the Solanaceae family and other plants susceptible to TMV U1. The genome of crTMV (6312 nucleotides) was sequenced (Dorokhov et al., 1994, FEBS Letters 350, 5-8) and was shown to contain four traditional ORFs encoding proteins of 122-kDa (ORF1), 178-kDa (ORF2), the readthrough product of 122-kDa protein, a 30-kDa MP (ORF3), and a 17-kDa CP (ORF4). A unique structural feature of crTMV RNA was that, unlike other tobamoviruses, the coding regions of the MP and CP genes of crTMV are overlapped by 75 nucleotides, i.e. the 5'-proximal part of the CP coding region also encodes the C-terminal part of the MP.

In order to provide a clear and consistent understanding of this annex, the following definitions are provided:
Adjacent: A position in a nucleotide sequence immediately 5' or 3' to a defined sequence.
Amplification vector: A type of gene vector that, upon introduction into a host cell, is capable of replicating therein.
Anti-Sense Mechanism: A type of gene regulation based on controlling the rate of translation of mRNA to protein due to the presence in a cell of an RNA molecule complementary to at least a portion of the mRNA being translated.
Chimeric Sequence or Gene: A nucleotide sequence derived from at least two heterologous parts. The sequence may comprise DNA or RNA.
Coding Sequence: A deoxyribonucleotide sequence which, when transcribed and translated, results in the formation of a cellular polypeptide or a ribonucleotide sequence which, when translated, results in the formation of a cellular polypeptide.
Compatible: The capability of operating with other components of a system. A vector or plant viral nucleic acid which is compatible with a host is one which is capable of replicating in that host. A coat protein which is compatible with a viral nucleotide sequence is one capable of encapsidating that viral sequence.
Gene: A discrete nucleic acid sequence responsible for a discrete cellular product.
Gene to be expressed: A gene of technological interest to be expressed.
Host: A cell, tissue or organism capable of replicating a vector or plant viral nucleic acid and which is capable of being infected by a virus containing the viral vector or plant viral nucleic acid. This term is intended to include procaryotic and eukaryotic cells, organs, tissues or organisms, where appropriate.
Host Plant Genome: This term mean preferably the nuclear genome of a host plant cell, but may also include mitochondrial or chloroplast DNA.
Infection: The ability of a virus or amplification-based vector to transfer its nucleic acid to a host or introduce nucleic acid into a host, wherein the viral nucleic acid or a vector is replicated, viral proteins are synthesized, and new viral particles assembled. In this context, the terms "transmissible" and "infective" are used interchangeably herein.
Internal Ribosome Entry Site (IRES) element, or IRES: a nucleotide sequence of viral, cellular or synthetic origin, which at the stage of translation is responsible for internal initiation.
IRES element necessary for translation of a gene downstream thereof: IRES element which is effective for translation of said gene in the sense that without such IRES element no technologically significant translation of this gene will occur.
Non-viral gene: A gene not functional for the life cycle of a virus.
Phenotypic Trait: An observable property resulting from the expression of a gene.
Plant Cell: The structural and physiological unit of plants, consisting of a protoplast and the cell wall.
Plant Organ: A distinct and visibly differentiated part of a plant, such as root, stem, leaf or embryo.
Plant Tissue: Any tissue of a plant in planta or in culture. This term is intended to include a whole plant, plant cell, plant organ, protoplast, cell culture, or any group of plant cells organized into a structural and functional unit.
Production Cell: A cell of a tissue or organism capable of replicating a vector or a viral vector, but which is not necessarily a host to the virus. This term is intended to include prokaryotic and eukaryotic cells, organs, tissues or organisms, such as bacteria, yeast, fungus and plant tissue.
Promoter: The 5'-non-coding sequence upstream to and operationally connected to a coding sequence which is involved in the initiation of transcription of the coding sequence.
Protoplast: An isolated plant cell without cell walls, having the potency of regeneration into cell culture or a whole plant.
Recombinant Plant Viral Nucleic Acid: Plant viral nucleic acid which has been modified to contain nonnative nucleic acid sequences.
Recombinant Plant Virus: A plant virus containing the recombinant plant viral nucleic acid.
Reporter Gene: A gene the gene product of which can be easily detected.
Subgenomic Promoter (sgPR): A promoter of a subgenomic mRNA of a vector or a viral nucleic acid.
Substantial Sequence Homology: Denotes nucleotide sequences that are homologous so as to be substantially functionally equivalent to one another. Nucleotide differences between such sequences having substantial sequence homology will be de minimus in affecting function of the gene products or an RNA coded for by such sequences.
Transcription: Production of an RNA molecule by RNA polymerase as a complementary copy of a DNA sequence.
Translation: Production of a polypeptide by a ribosome (frequently by means of *scanning* a messenger RNA).
Vector: A nucleic acid, which is capable of genetically modifying a host cell. The vector may be single-stranded (ss) (+), ss (-) or double-stranded (ds).
Virus: An infectious agent composed of a nucleic acid encapsidated in a protein. A virus may be a mono-, di-, tri- or multi-partite virus.

### Advantages

This annex describes a novel strategy for constructing the amplification-based vectors for foreign (heterologous, non-native) gene expression such that translation of these genes can occur through an IRES-mediated internal ribosome entry mechanism from a polycistronic RNA and/or through IRES-mediated cap-independent internal ribosome entry mechanism from bi- and multicistronic sgRNA produced from the vector in the infected cell. In either event, the IRES element is necessary for translation of a gene. One of the advantages of this strategy is that it does not require any specific manipulation in terms of sgPRs: the only sequences that should be inserted into the vector are the IRES-sequence(s) (native or/and non-native) upstream of gene(s) to be translated. As a result, translation of downstream gene(s) is promoted by the inserted IRES sequences, I.e. is cap-independent. The sequence segment harboring an IRES element preferably does not function as subgenomic promoter to a technically significant degree. This means that this sequence segment either does not cause any detectable production of corresponding subgenomic RNA or that for the translation of any such subgenomic RNA, if formed by any residual subgenomic promoter activity of said sequence segment, this IRES element is still necessary for the translation of a downstream gene. Consequently, in a special case, primary recombinant RNA produced by the vector comprises: one or more structural genes preferably of viral origin, said IRES sequence, the (foreign) gene of interest located downstream of the IRES and the 3-NTR. It is important that this strategy allows a simultaneous expression of more than one foreign gene by insertion of a tandem of two (or more) foreign genes, each being controlled by a separate IRES sequence. The present annex is preferably directed to nucleic acids and recombinant viruses which are characterised by cap- independent expression of the viral genome or of its subgenomic RNAs or of non-native (foreign) nucleic acid sequences and which are capable of expressing systemically in a host plant such foreign sequences via additional plant-specific IRES element(s).

In a first preferred embodiment, a plant viral nucleic acid is provided in which the native coat protein coding sequence and native CP subgenomic promoter have been deleted from a viral nucleic acid, and a non-native plant viral coat protein coding sequence with upstream located plant virus IRES element has been inserted that allows for cap-independent expression in a host plant, whereas packaging of the recombinant plant viral nucleic acid and subsequent systemic infection of the host by the recombinant plant viral nucleic acid are maintained. The recombinant plant viral nucleic acid may contain one or more additional native or non-native IRES elements that function as translation elements and which have no transcriptional activity, i.e. are effecticely unable to function as a subgenomic promoter. Each native or non-native IRES element is capable of providing cap-independent expression of adjacent genes or nucleic acid sequences in the host plant.

In a second preferred embodiment, an amplification and expression vector is provided in which native or non-native plant virus IRES element(s) located upstream of foreign nucleic acid sequences are inserted downstream of a native coat protein gene. The inserted plant virus IRES element may direct cap-independent expression of adjacent genes in a host plant. Non-native nucleic acid sequences may be inserted adjacent to the IRES element such that said sequences are expressed in the host plant under translational control of the IRES element to synthesize the desired product.

In a third preferred embodiment, a recombinant vector nucleic acid is provided as in the second embodiment except that the native or non-native plant viral IRES element(s) with downstream located foreign nucleic acid sequences are inserted upstream of native coat protein subgenomic promoter and coat protein gene.

In a fourth preferred embodiment, a recombinant vector nucleic acid is provided in which native or non-native plant viral IRES element(s) is (are) used at the 5' end of the viral genome or in the viral subgenomic RNAs so as to render translation of a downstream gene(s) cap-independent.

In a fifth preferred embodiment, inhibition of cap-dependent translation is being utilised to increase the level of cap-independent translation from said vectors.

The viral-based amplification vectors are encapsidated by the coat proteins encoded by the recombinant plant viral nucleic acid to produce a recombinant plant virus. The recombinant plant viral nucleic acid is capable of replication in the host, systemic spreading in the host, and cap-independent expression of foreign gene(s) or cap-independent expression of the whole viral genome or of subgenomic RNAs in the host to produce the desired product. Such products include therapeutic and other useful polypeptides or proteins such as, but not limited to, enzymes, complex biomolecules, or polypeptides-or-traits-or products resulting from antisense RNA production. Examples for desirable input traits are resistance to herbicides, resistance to insects, resistance to fungi, resistance to viruses, resistance to bacteria, resistance to abiotic stresses, and improved energy and material utilization. Examples for desirable output traits are modified carbohydrates, modified polysaccharides, modified lipids, modified amino acid content and amount, modified secondary metabolites, and pharmaceutical proteins, including enzymes, antibodies, antigens and the like. Examples for trait regulation components are gene switches, control of gene expression, control of hybrid seed production, and control of apomixis.

The present annex is also directed to methods for creation of artificial, non-natural IRES elements (as opposed to IRESs isolated from living organisms) providing cap-independent and promoter independent expression of a gene of interest in plant cells (and perhaps additionally in yeast or animal cells). Examples for living organisms from which IRESs may be isolated are animal viruses and plant viruses. Examples for animal viruses are hepatitis C virus, infectious bronchitis virus, picornaviruses such as poliovirus and encephalomiocarditis virus, and retroviruses such as moloney murine leukemia virus, and harvey murine sarcoma virus. Examples for plant viruses are potato virus X, potyviruses such as potato virus Y and turnip mosaic virus, tobamoviruses such as crucifer-infecting tobamovirus, and comoviruses such as cowpea mosaic virus. Alternatively, natural IRESs may be isolated from cellular messenger RNAs like those derived from antennapedia homeotic gene, human fibroblast growth factor 2, and translation initiation factor eIF-4G.

In a sixth preferred embodiment, artificial, non-natural IRES elements are created on the basis of complementarity to 18S rRNA of eukaryotic cells, including yeast, animal and plant cells.

In a seventh preferred embodiment, artificial, non-natural IRES elements are created on the basis of repeated short stretches of adenosin/guanosin bases.

In an eighth preferred embodiment, a method of engineering and using viral-based amplification vectors is presented, wherein viral genome expression in plant cells occurs under the control of a plant-specific artificial transcription promoter.

In a ninth preferred embodiment, a method of construction and using viral-based amplification vectors is presented, which vectors allow for expression from replicons being formed in plant cells as a result of primary nuclear transcript processing.

In a tenth preferred embodiment, a procedure is described for using circular single-stranded viral-based amplification vectors for cap-independent expression of foreign genes in plants.

In an eleventh preferred embodiment, methods are presented that allow for expression of a gene of interest in cells under conditions favoring cap-independent translation. In one example, cells infected with an amplification vector are treated with a compound inhibiting cap-dependent translation. In another example, the vector itself contains a gene, the product of which has an inhibiting effect on cap-dependent translation in the host or an anti-sense sequence having said function.

In a twelvth preferred embodiment, a method is described that allows, by using *in vivo* genetic selection, to identify an IRES sequence that provides cap-independent expression of gene of interest or a reporter gene in an expression vector.

### BRIEF DESCRIPTION OF THE FIGURES

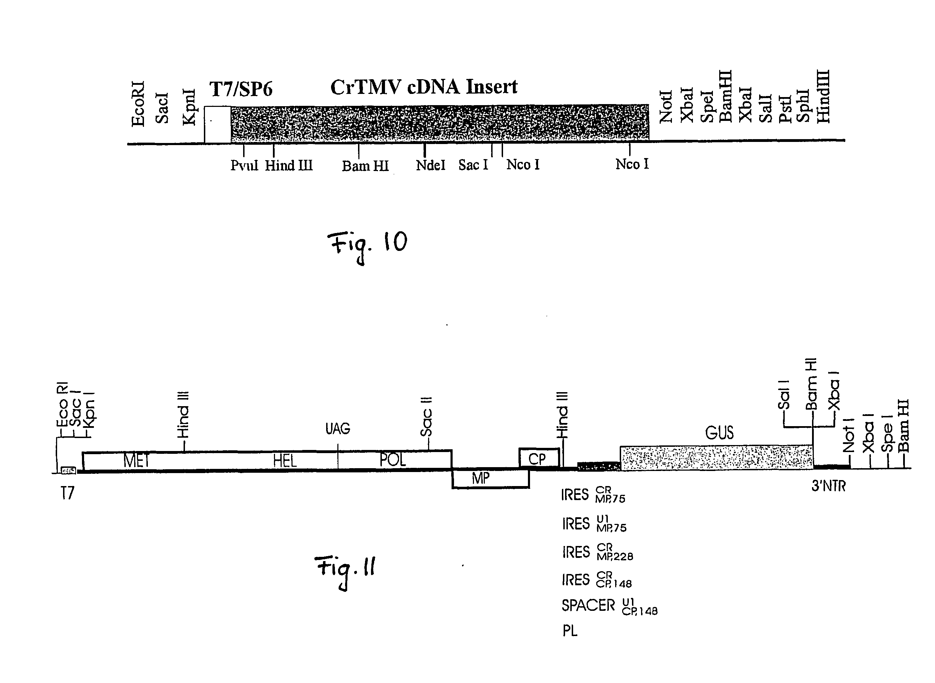

Fig. 10 depicts vectors T7/crTMV and SP6/crTMV.
Fig. 11 depicts vectors T7/crTMV/IRES_{MP,75}^{CR}-GUS, T7/crTMV/IRES_{MP,75}^{UI}-GUS, T7/crTMV/IRES_{MP,228}^{CR}-GUS, T7/crTMV/IRES_{CP,148}^{CR}-GUS, T7/crTMV/SPACER_{CP,148}^{UI}-GUS and T7/crTMV/PL-GUS.

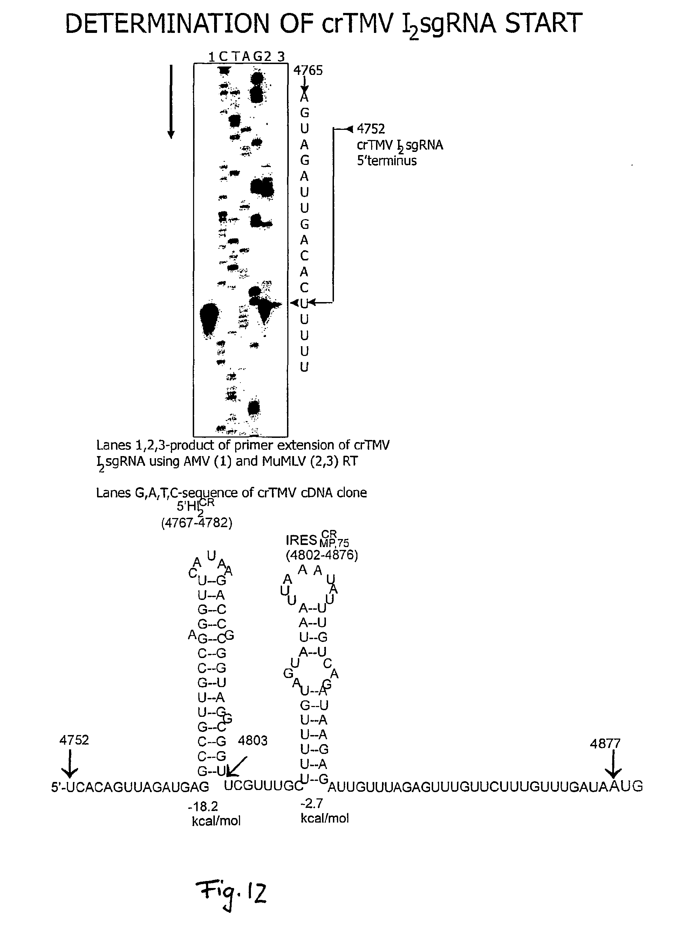

Fig. 12. Mapping of the 5'end of the crTMV I₂ sgRNA by primer extention (A) and putative secondary structure of I₂ sgRNA 5'NTR.

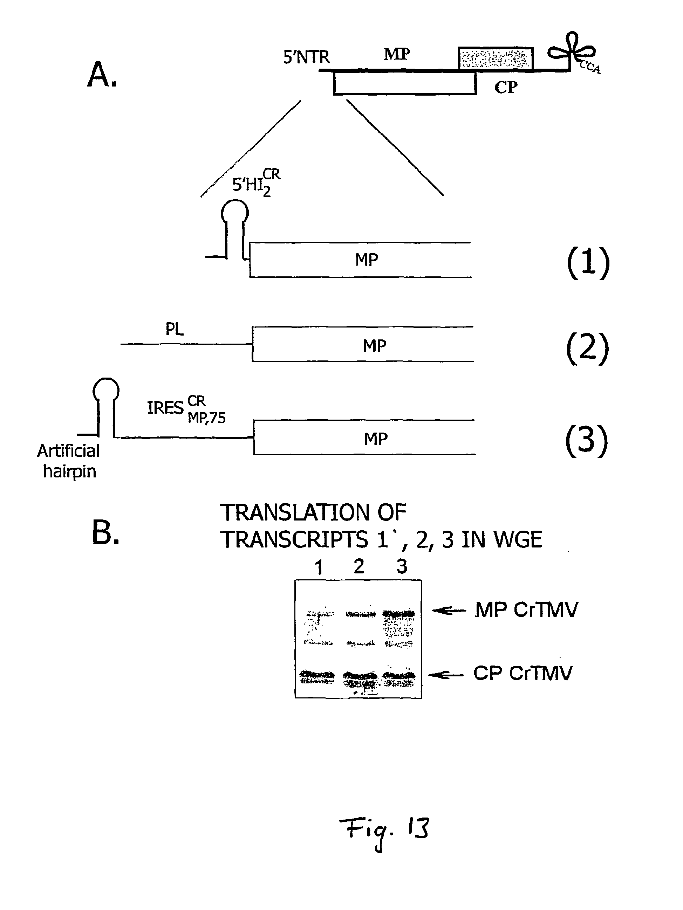

Fig. 13. crTMV I2 sgRNA 5'NTR contains translation inhibiting hairpin structure. (A)-depicts artificial transcripts used for *in vitro* translation in wheat germ extracts (WGE); (B)-shows translation products synthesized in WGE.

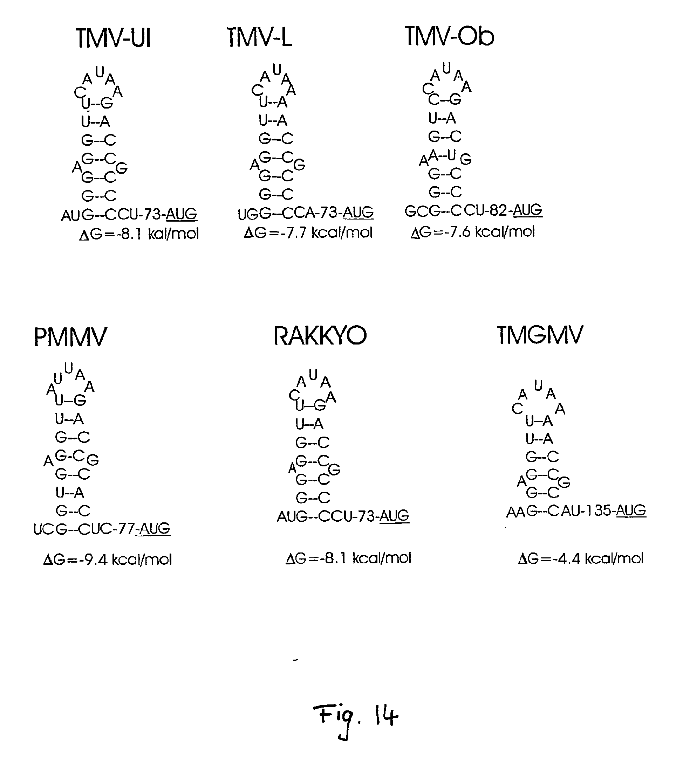

Fig. 14. Tobamoviruses contain a putative translation inhibiting hairpin structure upstream of the MP gene.

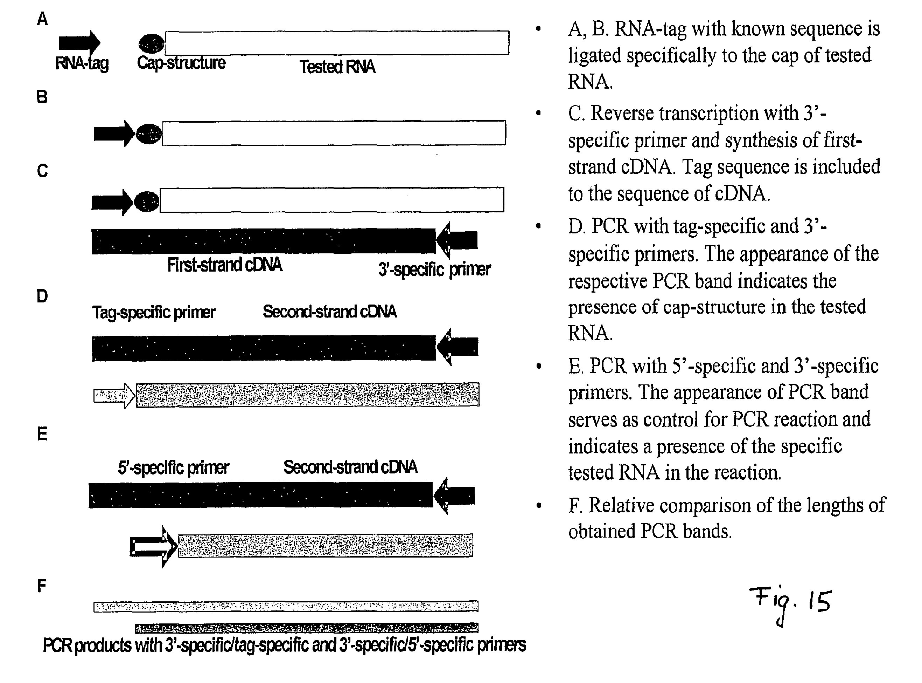

Fig. 15. Method of the specific detection of capped mRNAs. A, B. RNA-tag with known sequence is ligated specifically to the cap of tested RNA. C. Reverse transcription with 3'-specific primer and synthesis of first strand of cDNA. Tag sequence is included to the sequence of cDNA. D. PCR with tag-specific and 3'-specific primers. The appearance of the respective PCR band indicates the presence of cap-structure in the tested RNA. E. PCR with 5'-specific and 3'-specific primers. The appearance of PCR band serves as a control for PCR reaction and indicates a presence of the specific tested RNA in the reaction. F Relative comparison of the lengths of obtained PCR bands.
Fig. 16a and 16b. Detection of the presence of a cap-structure at the 5'-terminus of viral RNAs in a 2% agarose gel. Arrows indicate the respective PCR bands.

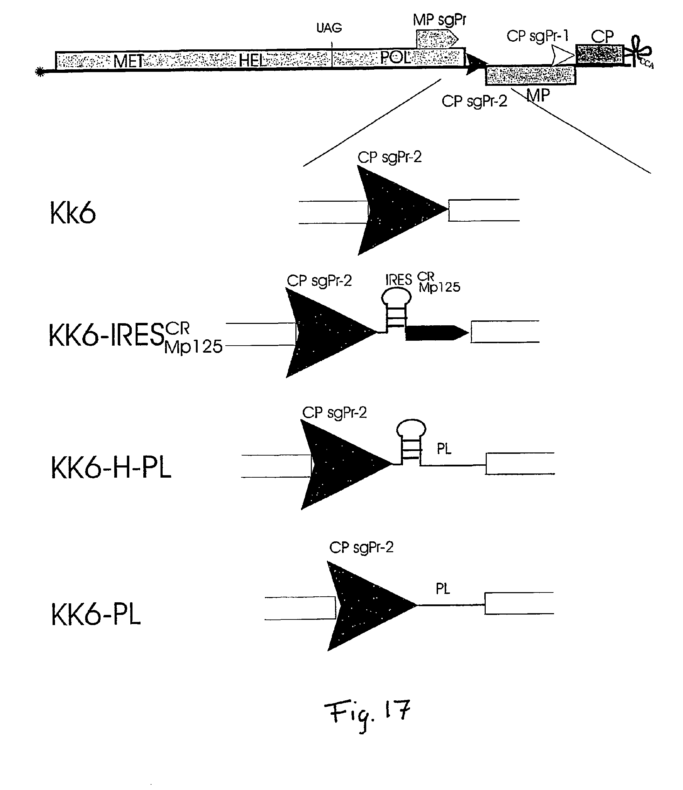

Fig. 17. depicts KK6-based TMV vectors.
Fig. 18. Nucleotide sequence of 5'NTR of KK6 and KK6-IRES_{MP,75}^{CR} I₂ sgRNA.

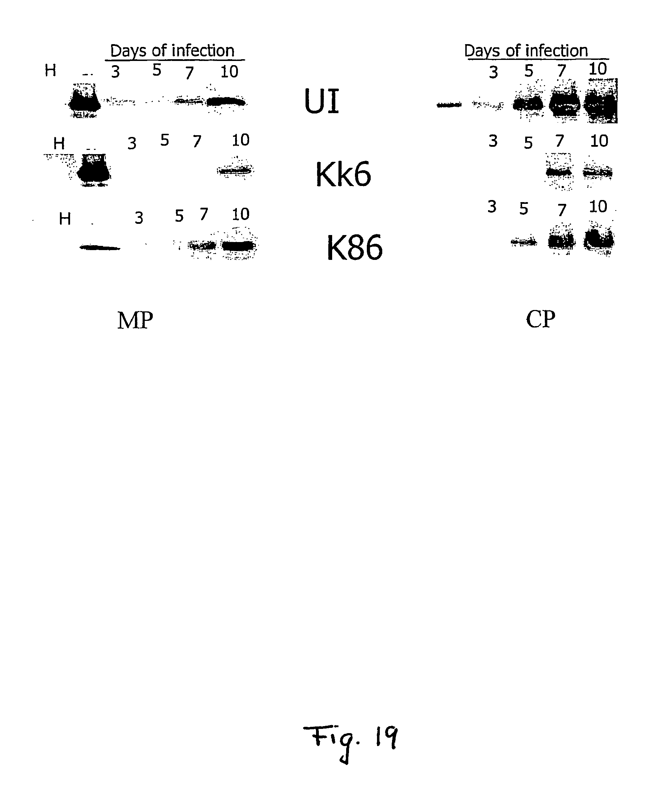

Fig. 19. Time-course of CP and MP accumulation in leaves inoculated with KK6-IRES_{MP,75}^{CR} (K86), KK6 and TMV UI.

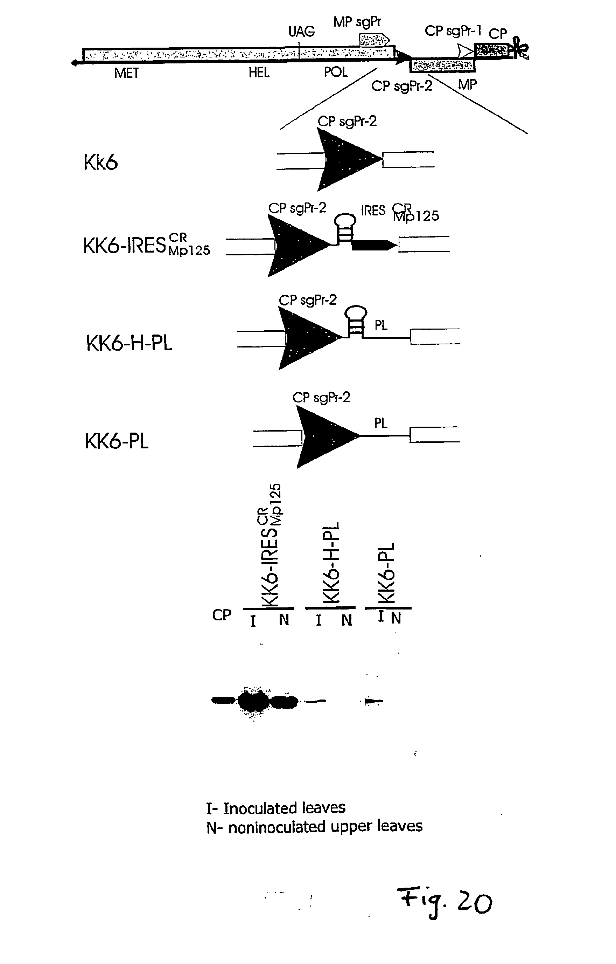

Fig. 20. CP accumulation in tobacco infected with KK6, KK6-IRES_{MP,75}^{CR}, KK6-IRES_{MP,125}^{CR}, and KK6-H-PL and KK6-PL.

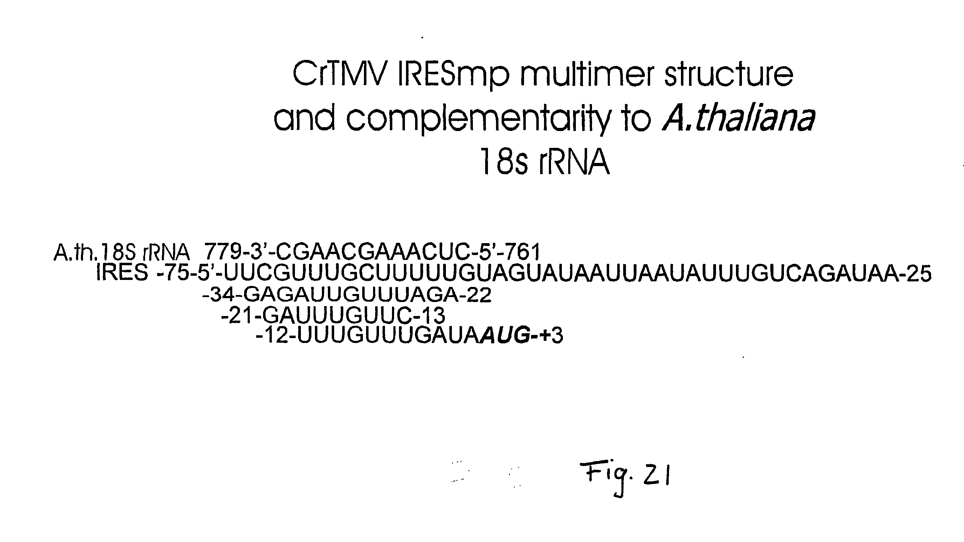

Fig. 21 depicts a crTMV IRESmp multimer structure and complementarity to 18S rRNA.

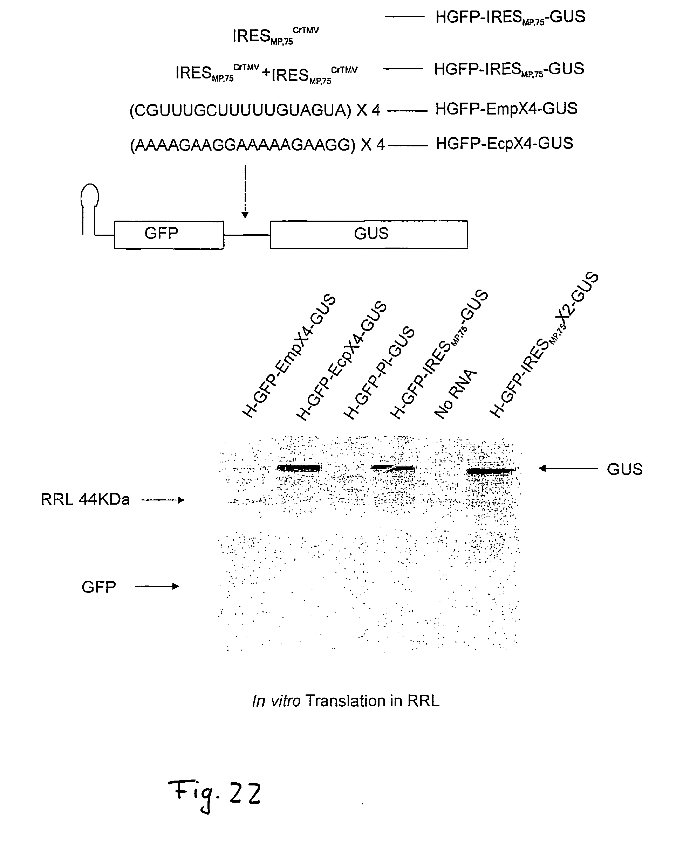

Fig. 22 depicts bicistronic transcripts containing IRES_{MP,75}^{CR}, the tetramers of 18-nt segment of IRES_{CP,148}^{CR}, I9-nt segment of IRES_{MP,75}^{CR}, polylinker (PL) as intercistronic spacer and products of their translation in RRL.

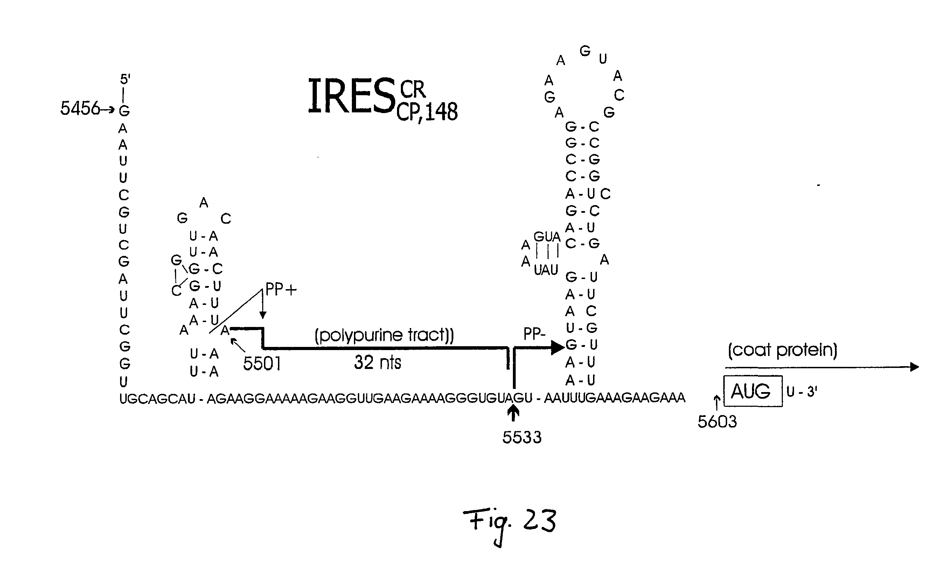

Fig. 23 depicts the IRES_{CP,148}^{CR} structure.

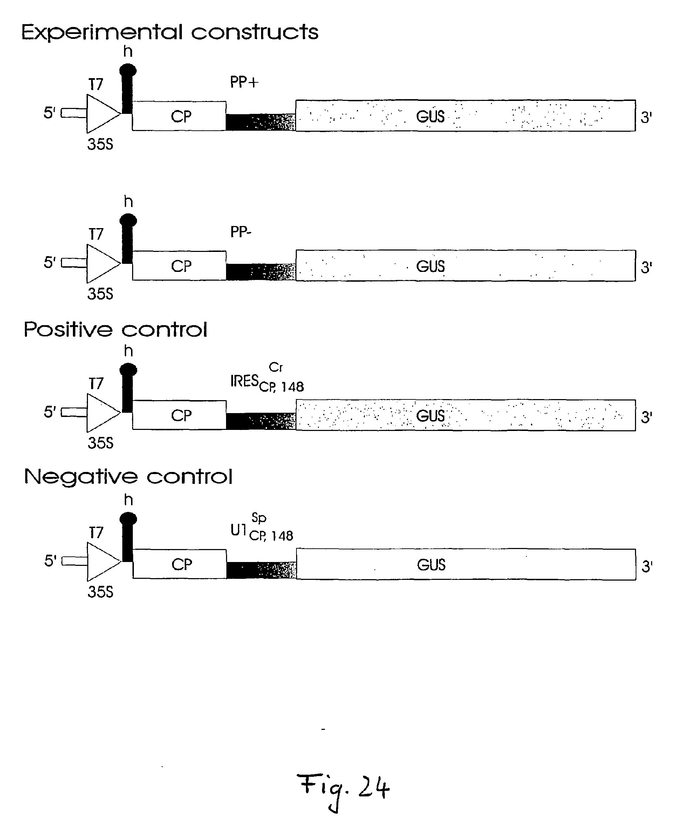

Fig. 24 depicts constructs used for IRES_{CP,148}^{CR} sequence elements testing *in vitro* and *in vivo*.

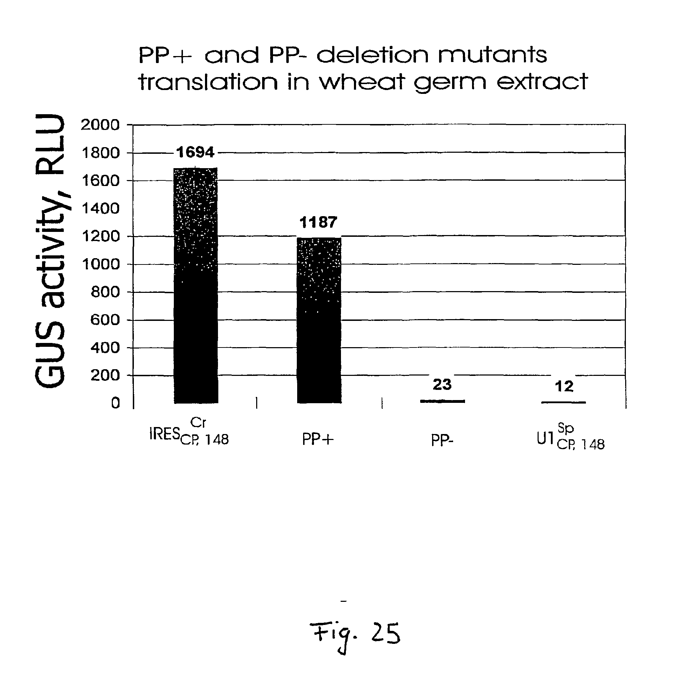

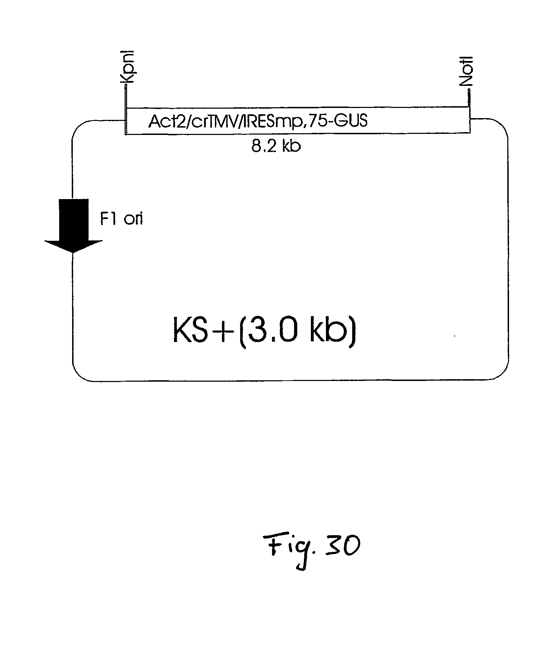

Fig. 25. GUS activity testing in WGE after translation of transcripts depicted in Fig. 30.

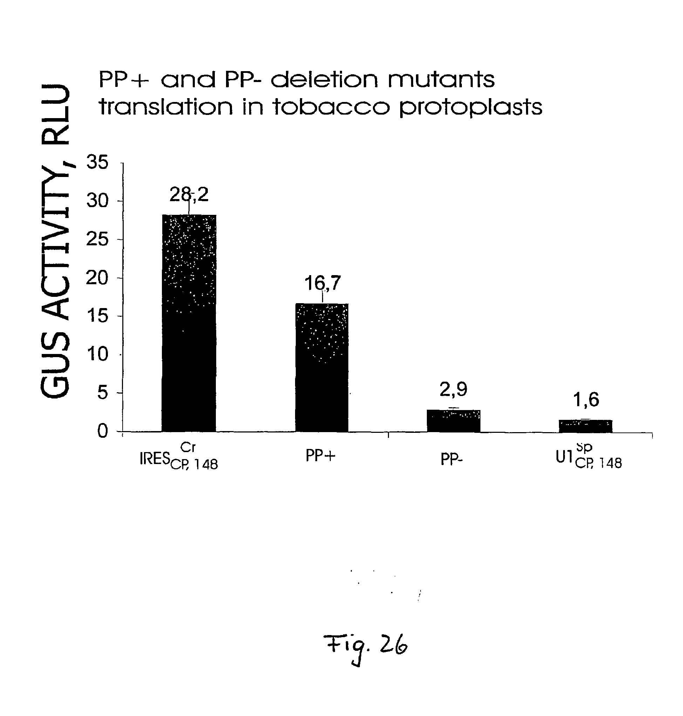

Fig. 26. GUS activity test in tobacco protoplasts transfected with 35S promoter-based constructs analogous to those depicted on Fig. 30.

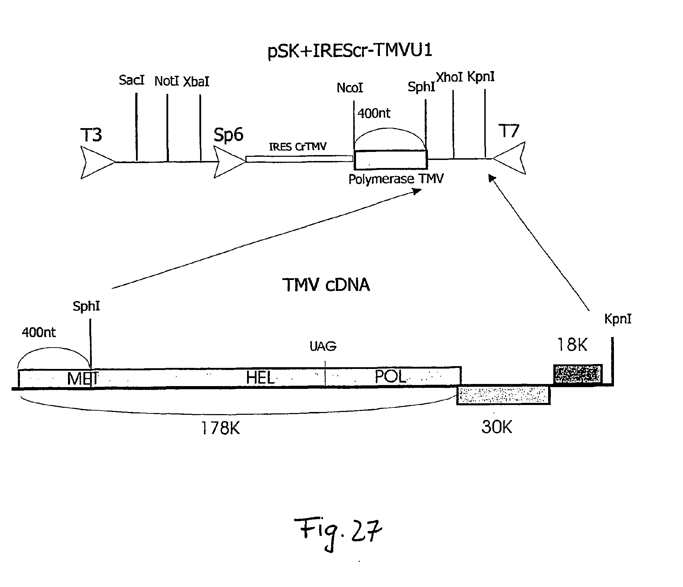

Fig. 27 depicts a scheme of cloning of two infectious TMV vectors containing IRES_{MP,75}^{CR} in 5'NTR.

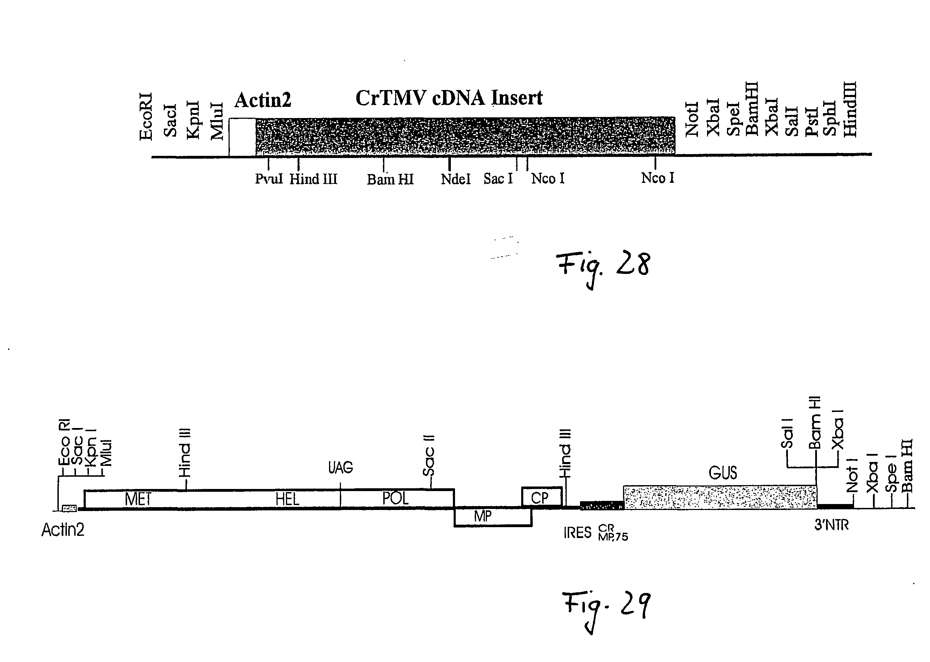

Fig. 28 depicts vector Act2/crTMV.
Fig. 29 depicts pUC-based vector Act2/crTMV/IRES_{MP,75}^{CR}-GUS
Fig. 30 depicts circular single-stranded vector KS/Act2/crTMV/IRES_{MP,75}^{CR}-GUS.

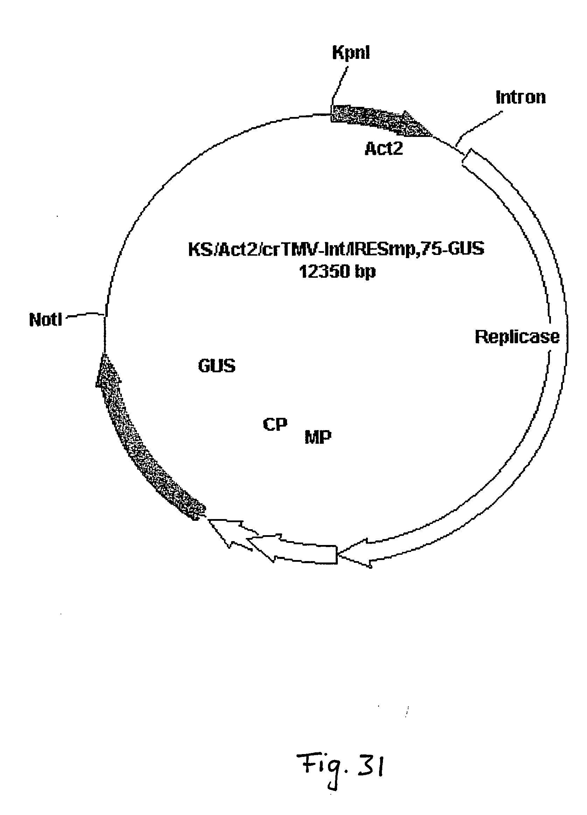

Fig. 31 depicts vector KS/Act2/crTMV/IRES_{MP,75}^{CR}-GUS

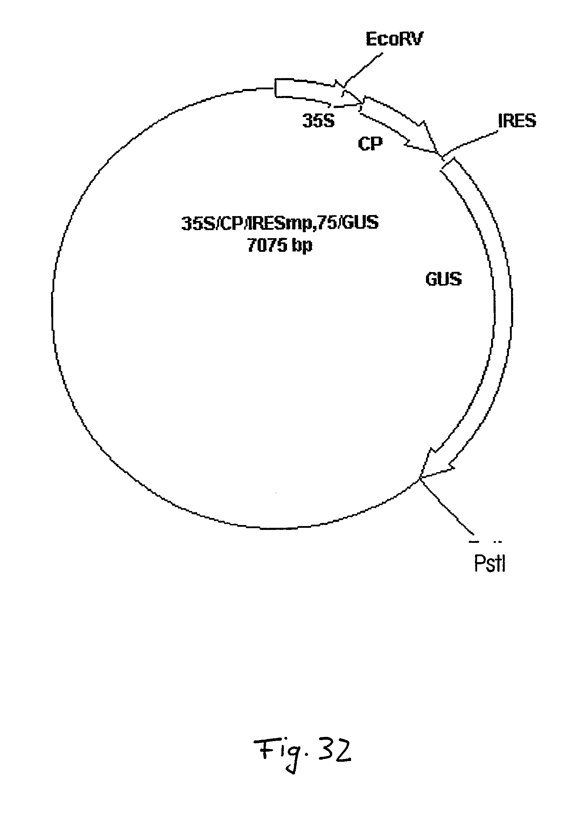

Fig. 32 depicts construct 35S/CP/IRES_{MP,75}^{CR}/GUS.

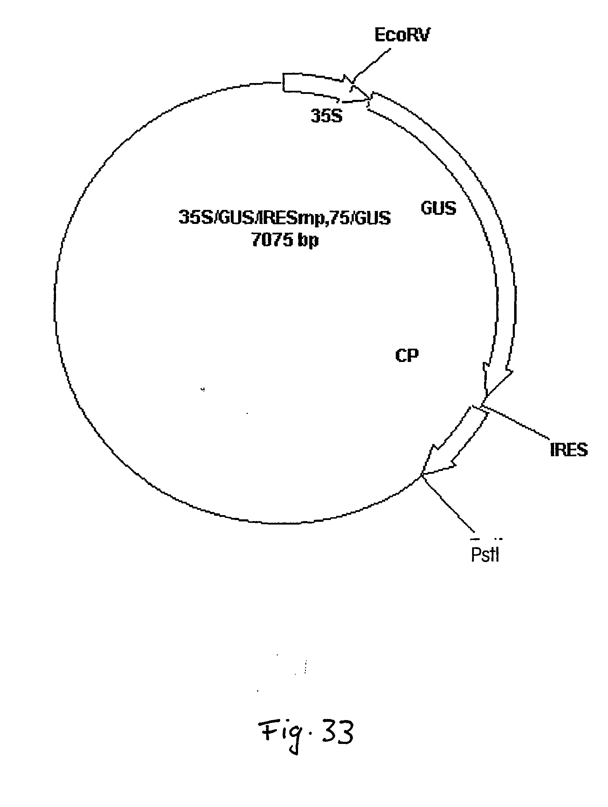

Fig. 33 depicts construct 35S/GUS/IRES_{MP,75}^{CR}/CP.

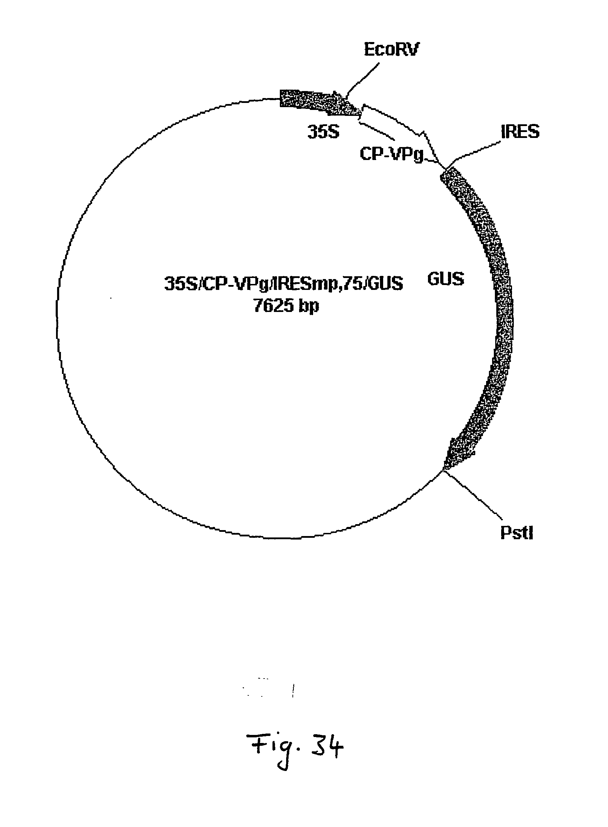

Fig. 34 depicts construct 35S/CP-VPg/IRES_{MP,75}^{CR}/GUS.

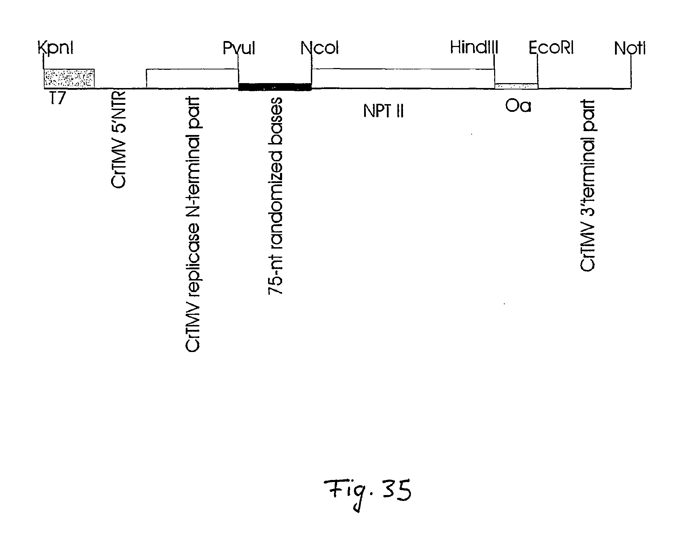

Fig. 35 shows a construct for *in vivo* genetic selection to identify a viral subgenomic promoter or an IRES sequence that provides cap-independent expression of a gene of interest in an expression vector.

### DETAILED DESCRIPTION

A primary objective of this annex is to provide a novel strategy for the construction of amplification-based vectors for foreign (heterologous; non-native) gene expression such that translation of these genes will occur by virtue of IRES-mediated cap-independent internal ribosome entry mechanism from polycistronic genomic viral RNAs and/or from bi- and multicistronic sgRNAs produced by an amplification vector, preferably a viral vector in a plant cell.

Construction of recombinant plant viral RNAs and creation of amplification-based vectors for the introduction and expression of foreign genes in plants has been demonstrated by numerous authors using the genomes of viruses belonging to different taxonomic groups (for review, see "Genetic Engineering With Plant Viruses", 1992, eds. Wilson and Davies, CRC Press, Inc.). Tobamoviruses are considered to be convenient subjects for the construction of viral vectors. Donson et al. (U.S. Patents Nos. 5,316,931; 5,589,367 and 5,866,785) created TMV-based vectors capable of expressing different foreign genes in a host plant. Thus, neomycin phosphotransferase, a-trichosantin and several other foreign genes were inserted adjacent to the subgenomic promoter (sgPR) of TMV CP. Donson *et al*., (1993, PCT WO 93/03161) developed on the basis of a tobamovirus "a recombinant plant viral nucleic acid comprising a native plant viral subgenomic promoter, at least one non-native plant viral subgenomic promoter and a plant viral coat protein coding sequence, wherein said non-native plant viral subgenomic promoter is capable of initiating transcription of an adjacent nucleic acid sequence in a host plant and is incapable of recombination with the recombinant plant viral nucleic acid subgenomic promoters and said recombinant plant viral nucleic acid is capable of systemic infection in a host plant".

Contrary to the technology of Donson *et al*., the present annex is not concerned with sgPRs in order to construct a viral replicon-based plant expression system. Instead of sgPRs, our technology manipulates with IRES-sequences of different origin (native or non-native for the virus), the sequences of which effectively lack sgPR activity, i.e. are effectively unable to promote sgRNA production. Therefore, these IRES sequences should not be regarded as sgPRs even in the case they represent a nonfunctional segment of a sgPR.

It is generally believed that uncapped transcripts of full-length viral RNA obtained after *in vitro* transcription of cDNA clones are generally non-infectious for intact plants and isolated protoplasts. Therefore, capping of a virus expression vector RNA transcript is generally considered as a prerequisite for *in vitro* transcript infectivity. Capped RNA transcripts are commonly used for introducing a viral vector RNA into a plant. It is important to note that in some cases viral RNA may be encapsidated by the coat protein using a simple procedure of *in vitro* assembly. Thus, TMV virions as well as pseudovirions containing vector RNA can be readily produced from CP and *in vitro* transcripts or purified authentic viral RNA. About fifteen years ago, it has been shown by Meshi et al. (1986, Proc. Natl. Acad. Sci. USA 85, 5043-5047) that (1) the uncapped transcripts of full-length TMV RNA produced *in vitro* are infectious in the absence of a cap analogue, although their specific infectivity is very low.

In the present annex, uncapped expression vector RNA reassembled with TMV CP can be used for plant inoculations in order to overcome its low infectivity. At least one of the additional approaches described herein opens the technical possibilities for plant infection with a cap-independent plant viral vector. This is the method of insertion of a full-length single-stranded (ss) DNA copy of a viral vector under control of an appropriate DNA promoter. After inoculation of a host plant with the recombinant viral DNA, the infectious full-length RNA of a plant viral vector, which will be able to replicate and spread over the plant, will be produced. In other words, these procedures, taken together with the fact of cap-independent expression of foreign gene(s) promoted by IRES sequences, make both processes, namely host plant inoculation and foreign gene expression, entirely cap-independent.

An important preferred object of this annex is the creation of a series of crTMV genome-based viral vectors with the "IRES-foreign gene" block inserted between the CP gene and 3'-NTR. Various IRES and control sequences were used (see Fig. 11) in combination with two different reporter genes (GUS and GFP). A unique feature is that the foreign genes that were located outside of the viral sgPR sequences were expressed in the infected plant cap-independently from the 3'-proximal position of genomic and sgRNAs produced by the vector. In particular, the IRES_{MP,75}^{CR} sequence representing the 3'-terminal part of the 5'-nontranslated leader sequence of crTMV sgRNA I₂ was efficient in mediating cap-independent expression of the 3'-proximal foreign gene in plants infected with a viral vector. It should be emphasized that said crTMV-based viral vectors produce three types of viral plus-sense ssRNAs in infected plants, including: i) full-length genomic RNA, ii) tricistronic I₂ sgRNA (our data show that the latter sgRNA is uncapped, contrary to full-length RNA), and iii) bicistronic sgRNA containing the first CP gene and the second foreign gene. Therefore, all these RNAs are 3'-coterminal and cap-independent translation of their 3'-proximal gene from either capped (full-length and bicistronic) or uncapped (tricistronic) RNAs is promoted by the preceding IRES sequence.

An important characteristic of virus-based vectors is their stability. However, the TMV-based vectors with foreign genes usually do not move efficiently through phloem in plants that can be systemically infected with wild-type virus. This may be due to increased length of the recombinant viral RNA and/or to the presence of the repeated sequences, which could lead to recombinations and deletions resulting in reversions to wild-type virus. The conversion of the progeny population to wild-type virus occurs in systemically infected leaves.

An important characteristic for a virus-based vector is the level of foreign protein gene expression and the level of protein accumulation. The vector is able to produce readily visible bands corresponding to GUS stained in SDS-PAGE.

The technologies suitable for construction of amplification-based vectors capable of expressing foreign sequences in host plants have been developed on the basis of different viral genomes (e.g., see G. Della-Cioppa *et al*., 1999, PCT WO 99/36516). The central feature of those inventions was that the recombinant plant viral nucleic acid "contains one or more non-native subgenomic promoters which are capable of transcribing or expressing adjacent nucleic acid sequences in the host plant. The recombinant plant viral nucleic acids may be further modified to delete all or part of the native coat protein coding sequence and to contain a non-native coat protein coding sequence under control of the native or one of the non-native plant viral subgenomic promoters, or put the native coat protein coding sequence under the control of a non-native plant viral subgenomic promoter". In other words, the most important element(s) of that invention is/are the native and non-native sgPR sequences used for artificial sgRNAs production by the viral vector. An important feature that distinguishes the teaching presented by our group from others is that according to WO 99/36516, the foreign gene must be inevitably located directly downstream of the sgPR sequence, i.e. should be located at the 5'-proximal position of the chimeric sgRNA produced by the viral vector in the host plant. By contrast, this annex proposes that the foreign gene is separated from a sgPR (if present) at least by one (or more) viral gene(s) such that said foreign gene is located 3'-proximally or internally within the functionally active chimeric sgRNA produced by the vector. Thus, foreign gene expression is promoted by the IRES sequence, native or non-native of the wild-type virus.

The next preferred object is the construction of a novel type of non-native IRES sequences, namely artificial, non-natural synthetic IRESs capable of promoting cap-independent translation of 5'-distal genes from eukaryotic polycistronic mRNAs. We show that intercistronic spacers complementary to 18S rRNA of varying length and composition are able to mediate cap-independent translation of the 3'-proximal GUS gene in bicistronic H-GFP-IRES-GUS mRNA (Fig. 22).

The last but not least advantage provided by the present annex is the possibility to combine repeats of two or more foreign genes each being preceded by the native or non-native IRES sequence in the amplification-based sector genome. Expression of such a cassette of an "IRES-foreign gene" will allow the simultaneous production of two or more foreign proteins by the vector.

Viruses belonging to different taxonomic groups can be used for the construction of virus-based vectors according to the principles of the present annex. This is right for both RNA- and DNA-containing viruses, examples for which are given in the following (throughout this document; each type species name is preceded by the name of the order, family and genus it belongs to. Names of orders, families and genera are in italic script, if they are approved by the ICTV. Taxa names in quotes (and not in italic script) indicate that this taxon does not have an ICTV international approved name. Species (vernacular) names are given in regular script. Viruses with no formal assignment to genus or family are indicated):

### DNA Viruses:

### Circular dsDNA Viruses:

Family: *Caulimoviridae*, Genus: *Badnavirus, Type species:* commelina yellow mottle virus, Genus: *Caulimovirus, Type species:* cauliflower mosaic virus, Genus "SbCMV-like viruses", *Type species:* Soybean chloroticmottle virus, Genus "CsVMV-like viruses". *Type species:* Cassava vein mosaicvirus, Genus "RTBV-like viruses", *Type species:* Rice tungro bacilliformvirus, Genus: "Petunia vein clearing-like viruses", *Type species:* *Petunia vein c*/*earing virus*;

**Circular ssDNA Viruses:** Family: *Geminiviridae,* Genus: Mastrevirus (Subgroup I Geminivirus), *Type species:* maize streak virus, Genus: *Curtovirus* (Subgroup II Geminivirus), *Type species:* beet curly top virus, Genus: *Begomovirus* (Subgroup III Geminivirus), *Type species:* bean golden mosaic virus;

### RNA Viruses:

**ssRNA Viruses:** Family: *Bromoviridae,* Genus: *Alfamovirus, Type species:* alfalfa mosaic virus, Genus: *Ilarvirus, Type species:* tobacco streak virus, Genus: *Bromovirus, Type species:* brome mosaic virus, Genus: *Cucumovirus, Type species:* cucumber mosaic virus;
Family: *Closteroviridae,* Genus: *Closterovirus, Type species:* beet yellows virus, Genus: *Crinivirus, Type species:* Lettuce infectious yellows virus, Family: *Comoviridae*, Genus: *Comovirus, Type species: cowpea mosaic virus*, Genus: *Fabavirus, Type species:* broad bean wilt virus 1, Genus: *Nepovirus, Type species:* tobacco ringspot virus;
Family: *Potyviridae*, Genus: *Potyvirus, Type species:* potato virus Y, Genus: *Rymovirus, Type species:* ryegrass mosaic virus, Genus: *Bymovirus, Type species:* barley yellow mosaic virus:
Family: *Sequiviridae*, Genus: *Sequivirus*, *Type species:* parsnip yellow fleck virus, Genus: *Waikavirus, Type species:* rice tungro spherical virus;
Family: *Tombusviridae,* Genus: *Carmovirus, Type species:* carnation mottle virus, Genus: *Dianthovirus, Type species:* carnation rinaspot virus, Genus: *Machlomovirus, Type species:* maize chlorotic mottle virus, Genus: *Necrovirus, Type species:* tobacco necrosis virus, Genus: *Tombusvirus, Type species:* tomato bushy stunt virus, Unassigned Genera of ssRNA viruses, Genus: *Capillovinis, Type species:* apple stem grooving virus;
Genus: *Carlavirus, Type species:* carnation latent virus;
Genus: *Enamovirus, Type species:* pea enation mosaic virus,
Genus: *Furovirus, Type species:* soil-borne wheat mosaic virus, Genus: *Hordeivirus, Type species:* barley stripe mosaic virus, Genus: *Idaeovirus, Type species:* raspberry bushy dwarf virus;
Genus: *Luteovirus, Type species:* barley yellow dwarf virus;
Genus: *Marafivirus, Type species:* maize rayado fino virus;
Genus: *Potexvirus, Type species:* potato virus X;
Genus: *Sobemovirus, Type species:* Southern bean mosaic virus, Genus: *Tenuivirus, Type species*: rice stripe virus,
Genus: *Tobamovirus, Type species:* tobacco mosaic virus,
Genus: *Tobravirus, Type species:* tobacco rattle virus,
Genus: *Trichovirus, Type species:* apple chlorotic leaf spot virus,
Genus: *Tymovirus, Type species:* turnip yellow mosaic virus,
Genus: *Umbravirus, Type species:* carrot mottle virus;
Negative ssRNA Viruses: Order: *Mononegavirales*, Family: *Rhabdoviridae,* Genus: *Cytorhabdovirus, Type Species:* lettuce necrotic yellows virus, Genus: *Nucleorhabdovirus, Type species:* potato yellow dwarf virus;
Negative ssRNA Viruses: Family: *Bunvaviridae,* Genus: *Tospovirus, Type species:* tomato spotted wilt virus;
**dsRNA Viruses**: Family: *Partitiviridae,* Genus: *Alphacryptovirus, Type species:* white clover cryptic virus 1, Genus: *Betacryptovirus, Type species:* white clover cryptic virus 2. Family: *Reoviridae,* Genus: *Fijivirus, Type species:* Fiji disease virus, Genus: *Phytoreovirus. Type species:* wound tumor virus. Genus: *Oryzavirus, Type species:* rice ragged stunt virus;
**Unassigned Viruses**: Genome *ssDNA*: Species banana bunchy top virus, Species coconut foliar decay virus, Species subterranean clover stunt virus,
Genome *dsDNA,* Species cucumber vein yellowing virus,
Genome *dsRNA,* Species tobacco stunt virus,
Genome *ssRNA,* Species Garlic viruses A,B,C,D, Species grapevine fleck virus, Species maize white line mosaic virus, Species olive latent virus 2, Species ourmia melon virus, Species Pelargonium zonate spot virus;
**Satellites and Viroids:** Satellites: ssRNA Satellite Viruses: *Subgroup 2* Satellite Viruses, *Type species:* tobacco necrosis satellite,
Satellite RNA, *Subgroup 2 B Type mRNA Satellites,* Subgroup 3 C Type linear RNA Satellites, *Subgroup* *4* D Type circular RNA Satellites,

### Viroids, Type species: potato spindle tuber viroid.

In particular, the methods of the present annex can preferably be applied to the construction of virus replicon-based vectors using the recombinant genomes of plus-sense ssRNA viruses preferably belonging to the genus *Tobamovirus* or to the families *Bromoviridae* or *Potyviridae* as well as DNA-containing viruses. In the latter case the foreign gene should preferably be located downstream of a viral gene and its expression can be mediated by the IRES sequence from bicistronic or polycistronic mRNA transcribed by a DNA-dependent RNA polymerase from a genomic transcription promoter.

A separate preferred aspect is concerned with the application of the methods of the invention to the construction of ssDNA-based vectors. The geminivirus-based vectors expressing the foreign gene(s) under control of an IRES sequence can exemplify this aspect.

The geminiviruses represent a group of plant viruses with monopartite or bipartite circular ssDNA that have twinned quasiicosahedral particles (reviewed by Hull and Davies, 1983, Adv. Virus Res; 28, 1-45; Mullineaux et al., 1992, "Genetic engineering with plant viruses", Wilson and Davies, eds.,1992, CRC Press, Inc.). The two ssDNA components of the bipartite geminiviruses referred to as A and B encode for 4 and 2 proteins, respectively. The DNA A contains the CP gene and three genes involved in DNA replication, whereas the DNA B encodes for two proteins essential for the viral movement. It has been demonstrated that the genomes of bipartite geminiviruses belonging to the genus *Begomovirus*, such as tomato golden mosaic virus (TGMV) and bean golden mosaic virus (BGMV) can replicate and spread over a certain host plant despite the deletion of the CP gene (Gardiner et al., 1988, EMBO J. 7, 899-904; Jeffrey et al., 1996, Virology 223, 208-218; Azzam et al., 1994, Virology 204, 289-296). It is noteworthy that some begomoviruses including BGMV exhibit phloem-limitation and are restricted to cells of the vascular system. Thus, BGMV remains phloem-limited, while TGMV is capable of invading the mesophyll tissue in systemically infected leaves (Petty and Morra, 2000, Abstracts of 19th Annual meeting of American Society for Virology, p.127). The present invention proposes to insert the foreign gene in a bipartite geminivirus genome by two ways: (i) downstream of one of its (e.g., BGMV) genes, in particular the CP gene such that the CP ORF will be intact or 3'-truncated and the IRES sequence will be inserted upstream of the foreign gene. Therefore, the mRNA transcription will proceed from the native DNA promoter resulting in production of bicistronic chimeric mRNA comprising the first viral gene (or a part thereof), the IRES sequence and the 3'-proximal foreign gene expression of which is mediated by the IRES. Alternatively (ii), the full-length DNA copy of the RNA genome of the viral vector can be inserted into a DNA of a CP-deficient bipartite geminivirus under control of the CP gene promoter. The RNA genome of the RNA-vector-virus will be produced as a result of DNA A transcription in the plant cell inoculated with a mixture of recombinant DNA A and unmodified DNA B. An advantage of this method is that the geminivirus-vector is needed as a vehicle used only for delivering the vector to primary-inoculated cells: all other steps will be performed by a tobamovirus vector itself including production of IRES-carrying vector RNA after geminivirus-vector DNA transcription by a cellular RNA polymerase, its replication, translation and systemic spread over the host plant and foreign gene(s) expression. As an additional possibility for the creation of a ssDNA vector, cloning of the viral cDNA and the foreign gene into a phagemid vector and production of the ssDNA according to standard methods can be mentioned.

Taking into account that tobamovirus-derived IRES sequences are shown to be functionally active in animal cells (our previous patent application), the methods of the present annex can be used for constructing the recombinant viral RNAs and producing the viral vectors on the basis of animal viruses, e.g. the viruses belonging to the families *Togaviridae, Caliciviridae, Astroviridae, Picornaviridae, Flaviviridae* in order to produce new vectors expressing the foreign genes under control of plant virus-derived IRES sequences. Such animal virus-based vectors for plants and animals can be useful in the fields of vaccine production or for gene therapy.

It should be noted, however, that the rod-like virions of Tobamoviruses and, in particular, the flexible and long virions of filamentous Potexviruses, Carlaviruses, Potyviruses and Closterovinrses apparently provide the best models for realization of the methods of the present annex.

The next preferred objective is to use the IRES sequence in such a way that the virus-based amplification vector will contain the IRES-sequence within its 5'-NTR. It is presumed that insertion of an IRES sequence does not prevent viral replication, but is able to ensure an efficient cap-independent translation of transcripts of genomic vector RNA.

Therefore, said construct may comprise: (i) An IRES element within or downstream of the 5'-untranslated leader sequence that is native or non-native for said viral vector and promotes cap-independent translation of the viral 5'-proximal gene (the RdRp), and (ii) at least one native or non-native IRES sequence located downstream of one or more viral structural genes and upstream of foreign gene(s) in order to promote their cap-independent translation.

According to this method, the specific infectivity of uncapped full-length vector transcripts will be increased due to efficient 5'-IRES-mediated translation of the parental RNA molecules in the primary inoculated cells.

Yet another preferred objective is the method of producing one or several protein(s) of interest in plant cells based on the introduction and cap-independent expression of a foreign gene from a mono- or polycistronic mRNA sequence mediated by the plant specific IRES sequence located upstream of said foreign gene sequence. A particular feature of this method is that the technology involves a procedure that allows to selectively switch off the cellular cap-dependent mRNA translation with the help of certain chemical compounds. However, this procedure does not affect the cap-independent IRES-mediated translation of mRNAs artificially introduced in the plant cells, thus allowing to control and enhance cap-independent expression.

Alternatively, the means for inhibiting the translation of cellular capped mRNA can be applied to plants infected with said viral vector itself that expresses the foreign gene(s) in a cap-independent manner. Under conditions when the translation of the cellular capped mRNAs is prevented, selective expression of the foreign gene(s) from said virus vector will occur.

The vector of this annex may be an RNA or DNA vector. It may be ss(+), ss(-) or ds. It may show any of the modes of amplification known from viruses. This includes the multiplication of the vector nucleic acid and optionally the production of coat protein and optionally the production of proteins for cell-to-cell movement or long-distance movement. The genes for the required replication and/or coat and/or movement may be wholly or partially encoded in an appropriately engineered host plant. In this manner, a system is generated consisting of mutually adapted vector and host plant.

The vector may be derived form a virus by modification or it may be synthesized *de novo.* It may have only IRES elements effectively devoid of any subgenomic promoter activity.

However, the vector may combine one or several subgenomic promoters with one or several IRES elements effectively devoid of subgenomic promoter function, so that the number of cistrons is greater than the number of promoters.

Considering the simplest case of one IRES element, said element may be located upstream of a (foreign) gene of interest to be expressed directly by said IRES element and optionally downstream of a (viral) gene for, say replication, to be expressed IRES-independent.

Alternatively, the gene of interest may be upstream of an IRES element and expressed IRES-independent and the IRES element serves for the expression of a downstream viral gene.

These simplest cases may of course be incorporated singly or multiply in a more complex vector.

The vector may contain a sequence in anti-sense orientation for suppressing a host gene.

This suppression function may exist alone or in combination with the expression of a (foreign) gene of interest. A particularly preferred case involves the suppression of a gene essential for cap-dependent translation, e.g. a gene for a translation initiation factor (e.g. eIF4) associated with cap-dependent translation, so that the translation machinery of the host plant is wholy in service of vector gene translation. In this case, the vector must be wholy cap-independent. Of course, the vector may be generated within a plant cell from a pro-vector by the plant nucleid acid processing machinery, e.g. by intron splicing.

The IRES element may be of plant viral origin. Alternatively, it may be of any other viral origin as long as it satisfies the requirement of operation in a plant cell. Further, an IRES element operative in a plant cell may be a synthetic or an artificial element. Synthesis may be guided by the sequence of the 18S rRNA of the host plant, namely the segment operative for IRES binding. It should be sufficiently complementary thereto. Sufficiency of complementarity can simply be monitored by testing for IRES functionality. Complementarity in this sense comprises GC, AU and to some extent GU base pairing. Further, such IRES element may be a multimer of such a complementary sequence to increase efficiency. The multimer may consist of identical essentially complementary sequence units or of different essentially complementary sequence units. Moreover, artificial IRES elements with high translation efficiency and effectively no subgenomic promoter activity may be generated by a process of directed evolution (as described e.g. in US 6,096,548 or US 6,117,679). This may be done *in vitro* in cell culture with a population of vectors with IRES element sequences that have been randomized as known per se. The clones which express a reporter gene operably linked to the potential IRES element are selected by a method known per se. Those clones which show subgenomic promoter activity are eliminated. Further rounds of randomization and selection may follow.

The IRES element of the vector of the annex may be effectively devoid of promoter activity.

This means that that the expression of a gene operably linked to an IRES element would not occur by a residual subgenomic promoter activity. This mode of action may be determined by standard molecular biology methods such as Northern blotting, primer extension analysis (Current Protocols in Molecular Biology, Ed. By F. Ausubel et al., 1999, John Wiley & Sons), 5' RACE technology (GibcoBRL, USA), and alike. It should be added that I RES elements that show detectable subgenomic promoter activity but operate essentially as translational rather than transcriptional elements, are also subject of our invention. Such discrimination could be derived, for example, by measuring quantitatively the relative amounts of two types of mRNAs on Northern blots, namely the short mRNA due to sgPR activity and the long mRNA not due to sgPR activity. If the IRES element does not essentially operate as a residual viral subgenomic promoter, the relative amount of corresponding short mRNA should be lower than 20%, preferably lower than 10% and most preferably, lower than 5% of the sum of the short and long mRNA. Thus we describe as a preferred embodiment a vector capable of amplification of a gene in a plant comprising a nucleic acid having a sequence for at least one non-viral gene to be expressed and having or coding for at least one IRES element necessary for translation of said gene in said plant with the proviso that the expression of said gene is essentially derived from translational rather than transcriptional properties of said IRES element sequence when measured by standard procedures of molecular biology.

The novel vectors of the annex open new avenues for genetic modification of plants. As a first possibility we suggest the use for determining the function of a structural gene of a plant.

This is notably of interest for genomics. Therefore, a plant for which the genome has been sequenced is of particular interest This is a small scale (plant-by plant) application. The vector of this annex is highly effective for this application, since it allows suppression of genes of interest and/or overexpression of genes to bring out the gene function to be discovered in an intensified manner.

In a large scale application the vector may be used to generate a trait or to produce a protein in a host plant. Infection of plants with the vector may be done on a farm field previously planted with unmodified plants. This allows for the first time a genetic modification of plants on a field, whereby the farmer has greatest freedom in terms of selection of seeds and vectors from a variety of sources for producing a desired protein or trait.

Examples for plant species of interest for the application of this teaching are monocotyledonous plants like wheat, maize, rice, barley, oats, millet and the like or dicotyledonous plants like rape seed, canola, sugar beet, soybean, peas, alfalfa, cotton, sunflower, potato, tomato, tobacco and the like.

In the following, the teaching of this annex will be further described using specific examples. Standard molecular biological techniques were carried out according to Sambrook et a/. (1989, Molecular Cloning: a Laboratory Manual. 2nd edn. Cold Spring Harbor Laboratory, Cold Spring Harbor, New York). All plasmids can be prepared according to the directions of the specification by a person of ordinary skill in the art without undue experimentation employing materials readily available in the art.

### EXAMPLE 1

### Construction of a tobamovirus vector infecting cruciferous plants

Virions of a known tobamovirus called crucifer tobamovirus (crTMV) which is able to infect systemically crucifer plants were isolated from *Olearacia officinalis L.* with mosaic symptoms.

Results of crTMV host-range examination are presented in Table1.

### Plasmid constructions

CrTMV cDNA was characterized by dideoxynucleotide sequencing (Dorokhov et a/., 1994 FEBS Letters 350, 5-8). Full length T7 RNA polymerase promoter-based infectious crTMV cDNA clones were obtained by RT-PCR from crTMV RNA using **oligonucleotides crTMV1-Kpn 5'-**gcat***ggtacc***ccttaatacgactcactataGTTTTAGTTTTATTGCAACAACAACAA (upstream), wherein the italic bold letters are a sequence of a Kpn I site, the underlined lowercase letters are nucleotide sequence of the T7 RNA polymerase promoter, the uppercase letters are from the 5'-termini of crTMV cDNA; and crTMV2 5'-gcat***gcggccgc***TGGGCCCCTACCCGGGGTTAGGG (downstream), wherein the italic bold letters are sequence of NotI site, the uppercase letters are from 3'-termini of crTMV cDNA and cloning into pUC19 between KpnI and Bam HI restriction sites (Fig. 10).

Full length SP6 RNA polymerase promoter-based infectious crTMV cDNA clones were obtained by RT-PCR from crTMV RNA by using oligonucleotides crTMV1-SP6 5'-gcat***ggtacc***atttaggtgacactatagaactcGTTTTAGTTTTATTGCAACAACAACAA (upstream), wherein the italic bold letters are a sequence of a Kpn I site, the underlined lowercase letters are a nucleotide sequence of the T7 RNA polymerase promoter, the uppercase letters are from the 5'-termini of crTMV cDNA; and crTMV2 5'-gcat***gcggccgc***TGGGCCCCTACCCGGGGTTAGGG (downstream), wherein the italic bold letters are a sequence of a Not I site, the uppercase letters are from 3'-termini of crTMV cDNA and cloning into pUC19 between Kpnl and Bam HI restriction sites (Fig. 10).

The full-length crTMV cDNA clones were characterized by dideoxynucleotide sequencing. The ability of crTMV infectious transcripts to infect systemically *Nicotiana* and crucifer species was confirmed by infection tests on respectively *Nicotiana tabacum* var. Samsun and *Arabidopsis thaliana.*

**TABLE 1. Virus detection and symptoms caused by crTMV in mechanically infected plants.**

| Species | Inoculated Leaves | | Non-inoculated Upper Leaves | |
|---|---|---|---|---|
| | Symptoms* | Virus** | Symptoms | Virus |
| *Nicotiana tabacum L.* | | | | |
| cv. Samsun | C | + | M | + |
| cv. Samsun NN. | L | + | s | |
| *Nicotiana clevelandii L.* | L+N | + | M | + |
| *Nicotiana glutinosa L.* | L+N | + | s | - |
| *Nicotiana sylvestris L.* | L+N | + | s | + |
| *Nicotiana benthamiana L.* | L+N | + | M | + |
| *Nicotiana rustica L.* | C | + | M | + |
| *Lycopersicum esculentum L.* | L+N | + | s | - |
| *Solanum tuberosum L.* | s | - | s | - |
| *Capsicum frutescens L.* | L+N | + | M | + |
| *Brassica chinensis L.* | C | + | M | + |
| *Brassica rapa L.* | C | + | M | + |
| *Brassica napus L* | C | + | M | + |
| *Brassica oleracea L.* | L | + | s | - |
| *Brassica compestris L.* | C | + | M | + |
| *Brassica cauliflora L.* | C | + | s | - |
| *Arabidopsis thaliana L.* | L+N | + | M | + |
| *Chenopodium amaranticolor L.* Coste and Reyn. | L+N | + | s | + |
| *Chenopodium quinoa L.* Willd. | L+N | + | s | - |
| *Chenopodium murale L.* | L+N | + | s | - |
| *Datura stramonium L.* | L+N | + | s | - |
| *Plantago major L.* | L+N | + | M | + |
| *Tetragonia expansa L.* | L+N | + | s | - |
| *Beta vulgaris L.* | L+N | + | s | - |
| *Petunia hybrida L.* | C | + | M | + |
| *Cucumis sativus L.* | L+N | + | s | - |
| *Phaseolus vulgaris L.* | s | - | s | - |
| *Raphanus sativus L.* | s | - | s | - |
| *Sinapis alba L.* | C | + | M | 0 |

| | | | | |
|---|---|---|---|---|
| *C, chlorosis; L, local lesion; M, mosaic; N, necrosis; s, symptomless. **Virus detected (+) or not (-) by ELISA. | | | | |

### EXAMPLE 2

### Construction of tobamoviral vectors for expression of GUS genes in Nicotiana and crucifer plants via viral IRESs

Series of IRES-mediated expression vectors T7/crTMV/GUS were constructed as follows. First, Hind III and Xba I sites were inserted in the end of the CP gene of Sac II/Not I fragment of T7/crTMV vector (Fig. 10) by a polymerase chain reaction (PCR) and two pairs of specific primers. Second, IRES_{MP,75}^{CR}-GUS, IRES_{MP,75}^{UI}-GUS, IRES_{MP,228}^{CR}-GUS, IRES_{CP,148}^{CR}-GUS, IRES_{CP,148}^{UI}-GUS, PL-GUS cDNA described in Skulachev *et al.* (1999, Virology 263, 139-154) were inserted into Hind III and Xba I containing Sac II/Not I fragment of T7/crTMV vector to obtain Sac I-IRES_{MP,75}^{CR}-GUS-Not I, Sac II-IRES_{MP,75}^{UI}-GUS-Not I, Sac II-IRES_{MP,228}^{CR}-GUS-Not I, Sac II-IRES_{CP,148}^{CR}-GUS-Not I, Sac II-IRES_{CP,148}^{UI}-GUS-Not I, Sac II-PL-GUS-Not I cDNA, respectively. Third, Sac II-Not I cDNA fragment of T7/crTMV vector was replaced by Sac I-IRES_{MP,75}^{CR}-GUS-Not I or Sac II-IRES_{MP,75}^{UI}-GUS-Not I or Sac II-IRES_{MP,228}^{CR}-GUS-Not I or Sac II-IRES_{CP,148}^{CR}-GUS-Not I or Sac II-IRES_{CP,148}^{UI}-GUS-Not I or Sac II-PL-GUS-Not I cDNA to obtain respectively, vectorT7/crTMV/IRES_{MP,75}^{CR}-GUS (Fig. 11), vector T7/crTMV/IRES_{MP,75}^{UI}-GUS (Fig. 11), vector T7/crTMVIIRES_{MP,228}^{CR}-GUS (Fig. 11), vector T7/crTMV/IRES_{CP,148}^{CR}-GUS (Fig. 11), vector T7/crTMV/IRES_{CP,148}^{UI}-GUS (Fig. 11 and vectorT7/crTMV/PL-GUS (Fig. 11).

### EXAMPLE 3

### Expression of GUS gene in transfected Nicotiana and crucifer plants via viral IRESs

This example demonstrates the tobamovirus IRES-mediated expression of the GUS gene in *Nicotiana benthamiana* and *Arabidopsis thaliana* plants infected crTMV-based vectors: T7/crTMV/IRES_{MP,75}^{CR}-GUS (Fig. 11), vector T7/crTMV/IRES_{MP,75}^{UI}-GUS (Fig. 11), vector T7/crTMV/IRES_{MP,228}^{CR}-GUS (Fig. 11), vector T7/crTMV/IRES_{CP,148}^{CR}-GUS (Fig. 11), vector T7/crTMV/IRES_{CP,148}^{UI}-GUS (Fig. 11) and vectorT7/crTMV/PL-GUS (Fig. 11).

### In vitro transcription

The plasmids T7/crTMV/IRES_{MP,75}^{CR}-GUS (Fig. 11), vector T7/crTMV/IRES_{MP,75}^{UI}-GUS (Fig. 11), vector T7/crTMV/IRES_{MP,228}^{CR}-GUS (Fig. 11), vector T7/crTMV/IRES_{CP,148}^{CR}-GUS (Fig. 11), vector T7/crTMV/IRES_{CP,148}^{UI}-GUS (Fig. 11) and vectorT7/crTMV/PL-GUS (Fig. 11) were linearized by Not I. The recombinant plasmids were transcribed *in vitro* as described by Dawson *et al.* (1986 Proc. Natl. Acad. Sci. USA 83, 1832-1836). Agarose gel electrophoresis of RNA transcripts confirmed that they were intact. The RNA concentration was quantified by agarose gel electrophoresis and spectrophotometry.

### GUS detection

Inoculated leaves were collected 10-14 days after transfection with capped full-length transcripts. IRES activity was monitored by histochemical detection of GUS expression as described earlier (Jefferson, 1987, Plant Molecular Biology Reporter 5, 387-405). Samples were infiltrated using the colorimetric GUS substrate, but the method (De Block and Debrouwer, 1992, Plant J. 2, 261-266) was modified to limit the diffusion of the intermediate products of the reaction: 0.115 M phosphate buffer, pH 7.0 containing 5-bromo-4-chloro-3-indolyl-β-D-glucuronide (X-Gluc) 600 µg/ml; 3 mM potassium ferricyanide; 10 mM EDTA. After incubation overnight at 37°C, the leaves were destained in 70% ethanol and examined by light microscopy.

### EXAMPLE 4

IRES_{MP,75'}^{CR} does not function as MP subgenomic promoter but provides MP gene expression via cap-independent internal initiation of translation in TMV-infected plants

This example uses different approaches to confirm the possibility of IRES_{MP,75}^{CR} used in viral vectors for cap-independent expression of a gene of interest.

### CrTMV MP subgenomic RNA has a 125-nt long 5'-nontranslated region (5'NTR) and contains a translation inhibiting stem-loop secondary structure

To determine the length and nucleotide sequence of TMV UI and crTMV MP subgenomic RNA (I₂ sgRNA) 5'NTR, the protocol of primer extension experiments described by Lehto et al. (1990, Virology 174, 145-157 ) was changed in the following way: (i) AMV reverse transcriptase (RT); (ii) RT reaction under 45°C; (iii) the GC-rich primer; (iv) increased dNTP concentration; (v) dITP to avoid secondary structure. It has been shown (Fig. 12) that the 5'UTR sequence of crTMV I₂ sgRNAs consists of 125 nucleotides. This result was confirmed by direct 5'UTR RT sequencing. Fig. 12B shows that crTMV 5'NTR contains a stable hairpin-loop structure. Being placed just upstream of the MP gene of artificial transcript, it is able to inhibit MP gene translation *in vitro* (Fig. 13). This means that IRES_{MP,75}^{CR} located between 5'HI₂^{CR} and the MP gene can provide efficient cap-independent internal initiation of translation. Fig. 14 shows that homologous to 5'HI₂^{CR} putative translation inhibiting hairpin-loop structure can be revealed in the 125-nt sequence upstream of the MP gene of other tobamoviruses.

### CrTMV and TMV UI MP subgenomic RNAs are not capped

To study the structure of the 5'-terminus of the subgenomic RNA coding for the 30K movement protein (MP) gene of crTMV, the "Jump-Start" method offered by Active Motif was used. Jump-Start^{™} is the method of chemical ligation of an RNA tag specifically to the 5'-end of capped mRNAs. During reverse transcription, the ribo-oligonucleotide tag of a known sequence becomes incorporated into the 3'-end of a first strand cDNA. This creates a known priming site suitable for PCR.

Initially, the 5'-terminal 2'-3'-cis-glycol groups of capped RNA were converted to reactive dialdehydes via sodium periodate oxidation. 1-2 µl of a tested RNA (1µg/µl) were mixed with 14 µl of pure water and 1 µl of sodium acetate buffer (pH 5.5), then 4 µl of 0.1 M sodium periodate were added and the reaction mixture was incubated for 1 hour.

Then a 3'-aminoalkyl derivatized synthetic ribo-oligonucleotide tag was chemically ligated to the di-aldehyde ends of oxidized RNA via reductive amination in the presence of sodium cyanoborohydride. 5 µl of sodium hypophosphite were added and the reaction mixture was incubated for 10 minutes. Then 23 µl of water, 1 µl of sodium acetate buffer (pH 4.5) and 2 µl of ribo-oligonucleotide tag 5'-CTAATACGACTCACTATAGGG (28,5 pmol/µl) were added to the reaction mixture and incubated for 15 minutes. Then 10 µl of sodium cyanoborohydride were added and incubated for 2 hours. Then 400 µl of 2 % lithium perchlorate in acetone were added, incubated for 15 minutes at -20°C and centrifugated for 5 minutes. The pellet was washed with acetone twice, then dissolved in 20 µl of water.

To remove an abundance of the RNA tag, CTAB precipitation in the presence of 0.3 M NaCl was used. CTAB is a strong cationic detergent that binds to nucleic acids to form an insoluble complex. Complex formation is influenced by the salt concentration: when the salt concentration is above 1 M, no complex formation occurs; when it is below 0.2 M, all nucleic acids are efficiently included in the complex; and when between 0.3 M and 0.4 M, the incorporation of small single- stranded nucleic acids into the complex is very inefficient (Belyavsky et al., 1989, Nucleic Acids Res. 25, 2919-2932; Bertioli et al., 1994, BioTechniques 16, 1054-1058). 10 µl of 1.2 M NaCl (to a final concentration of 0.4 M) and 3 µl of 10% CTAB (to a final concentration of 1 %) were added, the reaction mixture was incubated for 15 minutes at room temperature and then centrifugated for 5 minutes. The pellet was resuspended in 10 µl of NaCl, 20 µl of water and 3 µl 10% CTAB were added and the reaction mixture was incubated for 15 minutes at room temperature and then centrifugated for 5 minutes. The pellet was dissolved in 30 µl of 1.2 M NaCl, 80 µl of 96% ethanol was added, and the reaction mixture was incubated overnight at -20°C. Then it was centrifugated for 5 minutes and washed with 70% ethanol. Then the pellet of tagged RNA was dissolved in 24 µl of water.

Finally, reverse transcription with 3'-gene specific primers resulted in incorporation of the 5'-tag sequence at the 3'-terminus of first-strand cDNA. For reverse transcription, 12 µl of tagged RNA, 1 µl of specific 3'-end primers, 4 µl of 5x buffer for SuperScript^{™} II (Gibco BRL Life Technologies) containing 250 mM Tris-HCl (pH 8.3), 375 mM KCI, 15 mM MgCl₂ were mixed and heated at 95°C for 30 seconds, then cooled on ice. Then to the reaction mixture 0.5 µl of DTT (to 1 mM final concentration), 2 µl of 10 mM dNTP, 0.5 µl of RNAsine, 0.5 µl of SuperScript™ II were added and incubated for 1 hour at 42°C. Then 1 µl of 40 mM MnCl₂ was added and the reaction mixture was incubated for 15 minutes at 42°C. The presence of MnCl₂ in the reaction mixture allows SuperScript^{™} to overcome the cap structure during reverse transcription more efficiently: when using 3 mM MgCl₂ and 2 mM MnCl₂, the reverse transcriptase was shown to reveal an extraordinary high cap-dependent transferase activity, and typically the enzyme added preferentially three or four cytosine residues in the presence of 5'-capped mRNA templates (Chenchik et al., 1998, Gene cloning and analysis by RT-PCR, edited by Paul Siebert and James Larrick, BioTechniques Books, Natick, MA; Schmidt and Mueller, 1999, Nucleic Acids Res. 27, 331).

For the PCR reaction, two sets of primers were used for each tested RNA - 3'-specific/5'-specific primers and 3'-specific/tag-specific primers (Fig. 15).

To determine the possibility of using the method of chemical ligation of RNA with tag known sequence specifically to the cap-structure of viral RNAs, the genomic RNA of tobacco mosaic virus (TMV) U1 strain which is known to be capped (Dunigan and Zaitlin, 1990, J. Biol. Chem. 265, 7779-7786.) was used as control. The respective PCR bands were detected when specific primers, U1-Spn and corresponding to RNA-tag primer 779 were used in the PCR reaction (Table 2, Fig. 16).

**TABLE 2. Templates and primers used for PCR.**

| Template | Forward primer | Reverse primer | Corresponding PCR band and detection of cap-structrure |
|---|---|---|---|
| Genomic TMV (U1) RNA | | U1-Spn | + |
| Genomic TMV (U1) RNA | 779 | U1-Spn | + (cap) |
| Non-capped RNA transcript of TMV | | U1-Spn | + |
| Non-capped RNA transcript of TMV | 779 | U1-Spn | - (non-capped) |
| Complete cDNA clone of TMV (U1) | | U1-Spn | + |
| Genomic crTMV RNA | K5 | 2PM | + |
| Genomic crTMV RNA | 779 | 2PM | + (? - cap?) |
| Non-capped RNA transcript of crTMV | K5 | 2PM | + |
| Non-capped RNA transcript of crTMV | 779 | 2PM | - (non-capped) |
| Complete cDNA clone of crTMV | K5 | 2PM | + |
| Subgenomic TMV (U1) RNA for MP | 2211 | UM50-54 | + |
| Subgenomic TMV (U1) RNA for MP | 779 | UM50-54 - | (non-capped) |
| Complete cDNA clone of TMV (U1) | 2211 | UM50-54 | + |
| Subgenomic crTMV RNA for MP | 1038 | CPF25 | + |
| Subgenomic crTMV RNA for MP | 779 | CPF25 | - (non-capped) |
| Complete cDNA clone of crTMV | 1038 | CPF25 | 0 |

As a control, the non-capped RNA-transcript of the complete cDNA clone of TMV (U1) was used, and the cap structure was not found as expected (Table 2, Fig. 16).

Then the presence of a cap structure at the 5'-terminus of the genomic RNA of crTMV was tested. For these experiments, the specific PCR primers K5, 2PM and primer 779 which corresponds to the RNA-tag were taken (Table 1, Fig. 16). Interestingly, the mobility of the PCR band observed with the primers 779 and 2PM, was higher than expected (Fig. 16). This could reflect the presence of a strong secondary structure at the 5'-terminus of the genomic RNA of crTMV (Dorokhov et al., 1994, FEBS Letters 350, 5-8). This secondary structure is absent at the 5'-terminal part of related TMVs (Goelet et al., 1982, Proc. Natl. Acad. Sci. USA 79, 5818-5822). In control experiments with non-capped transcript of the complete cDNA clone of crTMV, no respective PCR band was observed, as expected.

For subgenomic RNA coding for the TMV (U1) MP gene, the absence of a cap-structure at the 5'-terminus was proposed. We tested the respective sgRNA with the specific primers 2211, UM50-54 and primer 779 corresponding to the RNA-tag. No cap structure was found (Table 2, Fig. 16).

The same results were obtained with the respective subgenomic RNA of crTMV (Table 2, Fig. 16) indicating that cap-structure is absent at the 5'-terminus of this subgenomic RNA of tobamoviruses.

### Insertion of IRES_{MP,75}^{CR} into a TMV UI based vector that is deficient of MP gene expression, KK6 provides efficient cap-independent MP gene expression

The KK6 vector (Lehto et al., 1990, Virology 174, 145-157) contains two CP subgenomic promoters (sgPr). The first CP sgPr-1 is in its proper place, upstream of the CP gene, whereas the second, CP sgPr-2 is placed upstream of the MP gene. It was shown that the MP gene was expressed via CP sgPr-2 instead of native MP sgPr. As a result of this insertion, KK6 lost the capability of efficient cell-to-cell movement. Analysis showed that I₂ sgRNA does not contain an IRES_{MP,75}^{CR} element in its 5'-nontranslated leader. It has been proposed that IRES_{MP,75}^{CR} - lacking KK6 I₂ sgRNA cannot express the MP gene efficiently. In order to examine this suggestion, IRES_{MP,75}^{CR} was inserted into KK6 between the CP sgPr-2 and the MP gene and we were able to obtain KK6-IRES_{MP75} that was stable in progeny (Fig. 17). It was shown that KK6-IRES_{MP75} provides synthesis of I₂ sgRNA containing crTMV IRES_{MP75} (Fig. 18).

It can be seen that the start of KK6-IRES_{MP75} I₂ sgRNA is not changed in comparison to KK6, which means that IRES_{MP75} does not serve as MP sgPr.

This insertion drastically improved cell-to-cell movement. KK6 infected Samsun plants systemically but the first symptoms developed slowly (15-17 days) compared to those induced by wild-type TMV (TMV 304) (about 7 days). Symptoms in the upper leaves of KK6-infected plants were distinct: yellow spots in contrast to mosaic symptoms were produced by wild-type TMV.

KK6 virus progeny produced numerous lesions in *N. glutinosa* that developed slower than lesions induced by wild-type TMV UI. The average size of local lesions induced by KK6 was approximately 0.1 mm in comparison to those induced by TMV UI (1.1 mm).

Plants inoculated by KK6-IRES_{MP75} looked like KK6-infected Samsun plants but: (i) the first systemic symptoms were developed more rapidly (about 10 days) and (ii) they were much brighter including yellow spots and mosaic. In contrast to KK6 the average size of local lesions induced by K86 in *N. glutinosa* was increased to 0.6-0.7 mm. Examination of the time-course of MP accumulation showed that KK6-IRES_{MP75} MP is detected earlier than KK6 MP in inoculated leaves (Fig. 19). These results allowed the conclusion that insertion of IRES_{MP75}^{CR} upstream of the KK6 MP gene partially restores the movement properties of KK6 defective in cell-to-cell and long-distance transport.

In order to obtain additional evidences of the essential role of IRES in cap-independent MP gene expression of TMV cDNA vectors and in the life cycle of tobamoviruses, series of additional KK6-based vectors was constructed (Fig. 17). KK6-IRES_{MP125} contains a natural hairpin-loop structure which is able to inhibit translation of the MP gene *in vitro* in the presence of WT crTMV 5'leader of I₂ sgRNA (Fig. 13) and IRES_{MP75}. KK6-H-PL contains a natural hairpin-loop structure and a 72-nt artificial polylinker sequence. KK6-PL contains the polylinker region only. Results of tests for infectivity on *Nicotiana tabacum* cv. Samsun plants (systemic host) are presented in Table.3.

Fig. 20 shows the results of a Western test of CP accumulation in tobacco leaves infected with KK6-based vectors. Replacement of IRES_{MP75}^{CR} by a nonfunctional PL-sequence drastically blocked vector multiplication.

**TABLE 3. Virus accumulation in tobacco systemically infected by KK6-based vectors.**

| cDNA copies | Virus accumulation |
|---|---|
| TMV 304 (WT) | +++ |
| KK6 | + |
| KK6-IRES_{MP75} | ++ |
| KK6-IRES_{MP125} | ++ |
| KK6-H-PL | +/- |
| KK6-PL | +/- |

### EXAMPLE 5

### Creation of artificial, non-natural IRES elements without subgenomic promoter activity provides cap-independent expression of genes of interest in eukaryotic cells

The goal of this example is to demonstrate the approaches for creation of artificial, non-natural IRES elements free of subgenomic promoter activity, which provide cap-independent expression of a gene of interest in eukaryotic cells.

Construction of an artificial, non-natural IRES element on the basis of 18-nt segment of IRES_{MP,75}^{CR}

Analysis of the IRES_{MP,75}^{CR} nucleotide sequence shows that it has a multimer structure and contains four nucleotide sequence segments being a variation of element (-72) GUUUGCUUUUUG(-61) and having high complementarity to A. *thaliana* 18S rRNA (Fig. 21). In order to design an artificial, non-natural IRES, the 18-nt sequence CGUUUGCUUUUUGUAGUA was selected.

Four oligos were synthesized:
MP1(+):
   5'-CGCGCAAGCTTTGCTTTTTGTAGTACGTTTGCTTTTTGTAGTACTGCAGGCGGG -3'
MP1(-):
   5'-CCCGCCTGCAGTACTACAAAAAGCAAACGTACTACAAAAAGCAAAGCTTGCGCG - 3'
MP2(+):
   5'-GGCGGCTGCAGTTTGCTTTTTGTAGTACGTTTGCTTTTTGTAGTAGAATTCGG-GC-3'
MP2(-):
   5'-GCCCGAATTCTACTACAAAAAGCAAACGTACTACAAAAAGCAAACTGCAGCCG-CC-3'
Primers MP1 (+) and MP1(-) were annealed to each other yelding dsDNA fragment A: 
CGCGCAAGCTTTGCTTTTTGTAGTACGTTTGCTTTTTGTAGTACTGCAGGCGGG
GCGCGTTCGAAACGAAAAACATCATGCAAACGAAAAACATCATGACGTCCGCCC
HindIII PstI
Primers MP2(+) and MP2(-) were annealed to each other yelding dsDNA fragment B:

GGCGGCTGCAGTTTGCTTTTTGTAGTACGTTTGCTTTTTGTAGTAGAATTCGGGC
CCGCCGACGTCAAACGAAAAACATCATGCAAACGAAAAACATCATCTTAAGCCCG
PstI EcoRI

Both fragments were digested with PstI and ligated to each other. Then the ligation product A+B was extracted using agarose electrophoresis and digested with HindIII and EcoRI followed by ligation into the hGFP-GUS vector described by Skulachev et al. (1999, Virology 263, 139-154) using HindIII and EcoRI cloning sites (Fig. 22).

### Results

The transcripts depicted in Fig. 22 were translated in rabbit reticulocyte lysate (RRL) as described by Skulachev et al. (1999, Virology 263, 139-154) and synthesized products were analyzed by gel electrophoresis. Results represented in Fig. 22 show that an artificial, non-natural sequence based on a 18-nt segment of IRES_{MP,75}^{CR} provides 3'-proximal-located GUS gene expression. This means that two features, namely complementarity to 18S rRNA and multimer structure are essential for IRES_{MP,75}^{CR} function and effectiveness.

A tetramer of 18-nt segment does not reach the level of IRES_{MP,75}^{CR} activity but there is a way to improve the activity of artificial, non-natural IRES elements using the 12-nt segment GCUUGCUUUGAG which is complementary to 18S rRNA.

### Construction of an artificial, non-natural IRES using 19-nt segment of IRES_{CP,148}^{CR}

Analysis of structural elements essential for IRES_{CP,148}^{CR} activity (Figs. 23-26) shows that a polypurine (PP) segment is crucial for IRES_{CP,148}^{CR} functioning. As a prominent element of the PP tract, a 9-nt direct repeat in 19-nt sequence: AAAAGAAGGAAAAAGAAGG (called direct repeat (DR)) was used for the construction of an artificial IRES. In order to obtain the tetramer of DR the following primers were used:
CP1(+): 
5'-CGCGCAAGCTTAAAAGAAGGAAAAAGAAGGAAAAGAAGGAAAAAGAAGGCT-
GCAGGCGGG-3'
CP1(-): 
5'-CCCGCCTGCAGCCTTCTTTTTCCTTCTTTTCCTTCTTTTTCCTTCTTTTAAGCT-TGCGCG-
3'
CP2(+): 
5'-GGCGGCTGCAGAAAAGAAGGAAAAAGAAGGAAAAGAAGGAAAAAGAAGGAA-
TTCGGGC-3'
CP2(-):
   5'- GCCCGAATTCCTTCTTTTTCCTTCTTTTCCTTCTTTTTCCTTCTTTTCTGCAGC-CGCC -3'

According to the experimental procedure described above, the following IRES element was used as intercistronic spacer:

5'-CGCGCAAGCUUAAAAGAAGGAAAAAGAAGGAAAAGAAGGAAAAAGAAGGCU-GCAG
AAAAGAAGGAAAAAGAAGGAAAAGAAGGAAAAAGAAGGAAUUCAUG-3'

### Results

The transcripts depicted in Fig. 22 were translated in rabbit reticulocyte lysate (RRL) as described by Skulachev et al. (1999, Virology 263, 139-154) and synthesized products were analyzed by gel electrophoresis. The results represented in Fig. 22 show that an artificial, non-natural sequence based on repeated 19-nt segment of IRES_{CP,148}^{CR} provides the efficient expression of a 3'-proximally located GUS gene.

### EXAMPLE 6

### Construction of a TMV cDNA transcription vector expressing a replicase gene in infected cells in a cap-independent manner

The main goal of this example was to obtain two new TMV U1-based viruses with modified 5'UTR providing expression of the replicase gene in a cap-independent manner:
1) Omega-leader of TMV was completely substituted by IRES_{MP,75}^{CR}. 
GUUCGUUUCGUUUUUGUAGUAUAAUUAAAUAUUUGUCAGAUAAGAGAUUGGUUAGAG
AUUUGUUCUUUGUUUGACCAUGG.
2) Since it is believed that the first 8 nucleotides of the TMV 5'UTR are essential for virus replication (Watanabe et al., 1996, J. Gen. Virol. 77, 2353-2357), IRES_{MP,75}^{CR} was inserted into TMV leaving the first 8 nucleotides intact:

GUAUUUUGUAGUAUAAUUAAAUAUUGUCAGAUAAGAGAUUGGUUAGAGAUUUGUU

CUUUGUUUGACCAUGG.

The following primers were used:
a) SP6-IRES-1 (in the case of the first variant) 
XbaI SP6 Promotor IRES_{MP,75}^{CR}
GGGTCTAGATTTAGGTGACACTATAGTTCGTTTCGTTTTTGTAGTA
b) SP6-IRES-2 (in the case of the second variant) 
XbaI SP6 Promotor IRES_{MP,75}^{CR}
GGGTCTAGATTTAGGTGACACTATAGTATTTTTGTAGTATAATTAAATATTTGTC.
c) IRES-Ncol (reverse primer to obtain IRES with a NcoI site at 3'end):
   GGGCCATGGTCAAACAAAGAACAAATCTCTAAAC.
d) TMV-Ncol (direct primer to obtain TMV polymerase, starting from NcoI site):
   NcoI
      GGGCCATGGCATACACACAGACAGCTAC.
e) TMV-Xho (reverse primer to obtain 5'-part of replicase from AUG to SphI site)
   XhoI
      ATGTCTCGAGCGTCCAGGTTGGGC.

### Cloning strategy:

PCR fragment A was obtained using oligos SP6-IRES1 and IRES-Ncol and crTMV clone as template. PCR fragment B was obtained using oligos TMV-Ncol and TMV-Xhol and TMV-304L clone. Fragments A and B were cloned simultaneously into the pBluscriptSK+ vector using Xbal and XhoI sites (fragments were ligated together through NcoI site). The same procedure was applied to obtain the second variant of the virus using SP6-IRES2 oligo.

At the next stage, the whole TMV cDNA was cloned into the obtained vector using SphI and KpnI sites to restore the viral genome (Fig. 27).

### EXAMPLE 7

### Construction of tobamoviral vectors Act2/crTMV and Act2/crTMV IRES_{MP,75}^{CR}-GUS based on Actin 2 transcription promoters

The main goal of this example is the demonstration of the construction strategy of a new crTMV-based vector with which viral genome expression in plant cells occurs under the control of an efficient Actin 2 transcription promoter. It allows the use of the vector Act2/crTMV/ IRES_{MP,75}^{CR}-GUS for gene expression in plants.

### Cloning Act2 into pUC19

The Act2 transcription promoter (about 1 000 bp) was cut out of plasmid pACRS029 by digestion with KpnI and Pst and cloned into pUC19 digested with Kpnl and Pstl.

### Creation of a PstI site in plasmid T7/crTMV (see Fig. 10) upstream of crTMV genome start

334-nt cDNA fragment of the 5'-terminal portion of the crTMV genome obtained by PCR using the direct primer ATGCTGCAGGTTTTAGTTTTATTGCAACAACAA (the PstI site is underlined) and the reverse primer ATGCGATCGAAGCCACCGGCCAAGGAGTGCA (Pvul site is also underlined) was digested with Pvul and PstI and inserted into pUC19Act2 together with the part of crTMV genome (Pvul-Spel fragment).

### Cloning of the rest of the genome together with the last construct

The Act2 containing construct was inserted into plasmid T7/crTMV after digestion with Kpnl/Spel.

### Fusion of 5'-terminus of crTMV to Act2 transcriptional start without additional sequences

This step was carried out by site-directed mutagenesis using oligonucleotide primer specific for both Act2 and crTMV to obtain the final construct Act2/crTMV (Fig. 28).

To get the vector Act2/crTMV/ IRES_{MP,75}^{CR}-GUS (Fig. 29) the Spel-Notl cDNA fragment of plasmid Act2/crTMV (Fig. 28) was replaced by the Spel-Notl DNA fragment of T7/crTMV/ IRES_{MP,75}^{CR}-GUS construct (Fig. 11) that contains the GUS gene under the control of IRES_{MP,75}^{CR}.

### EXAMPLE 8

### Construction of circular single-stranded tobamoviral vector KS/Act2/crTMV/IRES_{MP,75}^{CR}-GUS (Fig. 30)

The main goal of this example is to demonstrate the possibility of using circular single-stranded DNA vectors for foreign gene expression in plants.

In order to construct KS/crTMV/IRES_{MP,75}^{CR}-GUS (Fig. 30), 9.2 kb Kpnl-Notl cDNA fragment of vector Act2/crTMV/IRES_{MP,75}^{CR}-GUS was inserted into plasmid pBluescript II KS+ (Stratagene) digested with Kpnl-Sall and containing the phage f1 replication origin. Single-stranded DNA of vector KS/Act2/crTMV/IRES_{MP,75}^{CR}-GUS was prepared according to Sambrook et al., 1989 (Molecular Cloning: a Laboratory Manual, 2ed edn. Cold Spring Harbor Laboratory, Cold Spring Harbor, New York).

### EXAMPLE 9

### Construction of tobamoviral vector KS/Act2/crTMV-Int/IRES_{MP,75}^{CR}-GUS containing oleosin intron from Arabidopsis thaliana

The main goal of this example is to create vector KS/Act2/crTMV/IRES_{MP,75}^{CR}-GUS containing *Arabidopsis thaliana* oleosin gene intron that should be removed after transcript processing (Fig. 31).

The cloning strategy comprised the following steps:

### 1. Cloning of A. thaliana oleosin gene intron.

*A. thaliana* oleosin gene intron was obtained by PCR using A. *thaliana* genomic DNA and specific primers : A.th./Int (direct) ATGCTGCAGgttttagttCAGTAAGCACACATTTATCATC (Pstl site is underlined, lowercase letters depict crTMV 5'terminal sequence) and A.th/Int (reverse) ATGAGGCCTGGTGCTCTCCCGTTGCGTACCTA (Stul is underlined).

### 2. Insertion of A. thaliana oleosin gene intron into 334-nt 5'-terminal fragment of crTMV cDNA.

cDNA containing *A*. *thaliana* oleosin gene intron was digested with Pstl/Stul and ligated with DNA fragment obtained by PCR using primers corresponding to positions 10-334 of crTMV genome: atgAGGCCTTTATTGCAACAACAACAACAAATTA (Stul site is underlined) and ATGCGATCGAAGCCACCGGCCAAGGAGTGCA (Pvul site is underlined).

The next steps were as described in EXAMPLE 7.

### EXAMPLE 10

### Influence of rapamycin as an inhibitor of cap-dependent initiation of translation on GUS gene expression in tobacco protoplasts transfected with IRES_{MP,75}^{CR} containing bicistronic transcription vectors, 35S/CP/IRES_{MP,75}^{CR}/GUS (Fig. 32) and 35S/GUS/ IRES_{MP,75}^{CR}/CP (Fig. 33)

The aim of this example is to demonstrate the principal possibility to use inhibitors of cap-dependent translation to increase efficiency of IRES-mediated cap-independent translation of a gene of interest.

Rapamycin as an inhibitor of cap-dependent initiation of translation was selected. Recently, a novel repressor of cap-mediated translation, termed 4E-BP1 (eIF-4E binding protein-1) or PHAS-1 was characterized (Lin et al., 1994, Science 266, 653-656; Pause et al., Nature 371, 762-767). 4E-BP1 is a heat- and acid-stable protein and its activity is regulated by phosphorylation (Lin et al., 1994 Science 266, 653-656; Pause et al., Nature 371, 762-767). Interaction of 4EBP1 with eIF-4E results in specific inhibition of cap-dependent translation, both *in vitro* and *in vivo* (Pause et_al., Nature 371, 762-767). It has been shown that rapamycin induces dephosphorylation and consequent activation of 4E-BP1 (Beretta et al., 1996, EMBO J. 15, 658-664).

Construction of IRES- and GUS gene-containing vectors 35S/CP/ IRES_{MP,75}^{CR}/GUS (Fig. 32), 35S/GUS/ IRES_{MP,75}^{CR}/CP (Fig. 33) and a method of tobacco protoplast transfection with 35S-based cDNA were described by Skulachev et al. (1999, Virology 263, 139-154). Comparison of GUS gene expression in tobacco protoplats treated by rapamycin and transfected with bicistronic cDNA with GUS gene in 3'- and 5'-proximal location shows the possibility to increase IRES-mediated cap-independent translation of the GUS gene.

### EXAMPLE 11

### Influence of potyvirus VPg as a inhibitor of cap-dependent initiation of translation on GUS gene in tobacco protoplasts transfected with IRES_{MP,75}^{CR} containing bicistronic transcription vectors 35S/CPIIRES_{MP,75}^{CR}/GUS (Fig. 32) and 35S/CP-VPg/IRES_{MP,75}^{CR}/GUS

This example demonstrates the principal possibility of using a gene product to inhibit cap-dependent translation (Fig. 34). Recently, interaction between the viral protein linked to the genome (VPg) of turnip mosaic potyvirus (TuMV) and the eukaryotic translation initiation factor eIF(iso)4E of *Arabidopsis thaliana* has been reported (Wittman et al., 1997, Virology 234, 84-92). Interaction domain of VPg was mapped to a stretch of 35 amino acids and substitution of an aspartic acid residue within this region completely abolished the interaction. The cap structure analogue m⁷GTP, but not GTP, inhibited VPg-eIF(iso)4E complex formation, suggesting that VPg and cellular mRNAs compete for eIF(iso)4E binding (Leonard et al., 2000, J. Virology 74, 7730-7737).

The capability of VPg to bind eIF(iso)4E could be used for inhibition of cap-dependent translation. We propose to use the vector 35S/CP-VPg/IRES_{MP,75}^{CR}/GUS (Fig. 34) wherein CP is fused with VPg from potyvirus potato virus A. Comparison of GUS gene expression in protoplasts transfected with 35SICP-VPg/IRES_{MP,75}^{CR}/GUS or 35S/CP /IRES_{MP,75}^{CR}/GUS would allow to increase IRES-mediated and cap-independent GUS gene expression.

### EXAMPLE 12

### In vivo genetic selection of an IRES sequence or a subgenomic promoter using TMV vector

This example demonstrates the possibility of using *in vivo* genetic selection or Systematic Evolution of Ligands by Exponential enrichment (SELEX) of a subgenomic promoter or an IRES sequence providing cap-independent expression of a gene of interest in a viral vector. This approach proposes using side-by-side selection from a large number of random sequences as well as sequence evolution (Ellington and Szostak, 1990, Nature 346, 818-822; Tuerk and Gold, 1990, Science 249, 505-510; Carpenter and Simon, 1998, Nucleic Acids Res. 26, 2426-2432). The project encompasses:
*In vitro* synthesis of crTMV-based defective-interfering (DI) transcript containing the following elements (5'-3' direction): (i) a T7 transcription promoter, (ii) a 5'-terminal part of crTMV genome with a sequence responsible for viral genome complementary (minus chain) synthesis, (iii) a sequence coding for the N-terminal part of a viral replicase, (iv) a sequence containing 75-nt randomized bases, (v) a neomycin phosphotransferase II (NPT II) gene, (vi) a crTMV origin of assembly (Oa), and (vii) a 3'-terminal part of the crTMV genome with minus chain genome promoter sequence (Fig. 35).
Co-transfection of tobacco protoplasts by a transcript together with crTMV genomic RNA (Fig. 10). Protoplasts will grow and regenerate in media containing kanamycin.
Selection and isolation of an IRES or a subgenomic promoter element providing protoplast survival and regeneration in the presence of kanamycin.

### ANNEX B

### PROCESSES AND VECTORS FOR PRODUCING TRANSGENIC PLANTS

### FIELD

The present annex relates to processes and vectors for producing transgenic plants as well as plant cells obtained thereby.

### BACKGROUND

Achievement of a desirable and stably inheritable pattern of transgene expression remains one of the major problems in plant biotechnology. The standard approach is to introduce a transgene as part of a fully independent transcription unit in a vector, where the transgene is under transcriptional control of a plant-specific heterologous or a homologous promoter and transcription termination sequences (for example, see US 05,591,605; US 05,977,441; WO 0053762 A2; US 05,352,605, etc). However, after the integration into the genomic DNA, because of random insertion of exogenous DNA into plant genomic DNA, gene expression from such transcriptional vectors becomes affected by many different host factors.
These factors make transgene expression unstable, unpredictable and often lead to the transgene silencing in progeny (Matzke & Matzke, 2000, Plant Mol Biol., 43, 401-415; S.B. Gelvin, 1998, Curr. Opin. Biotechnol., 9, 227-232; Vaucheret et al., 1998, Plant J., 16, 651-659). There are well-documented instances of transgene silencing in plants, which include the processes of transcriptional (TGS) and posttranscriptional gene silencing (PIGS). Recent findings reveal a close relationship between methylation and chromatin structure in TGS and involvement of RNA-dependent RNA-polymerase and a nuclease in PTGS (Meyer, P., 2000, Plant Mol. Biol.. 43, 221-234; Ding, S.W., 2000, Curr. Opin. Biotechnol., 11, 152-156; Iyer et al., Plant Mol. Biol., 2000, 43, 323-346). For example, in TGS, the promoter of the transgene can often undergo methylation at many integration sites with chromatin structure not favorable for stable transgene expression. As a result, practicing existing methods requires many independent transgenic plants to be produced and analyzed for several generations in order to find those with the desired stable expression pattern. Moreover, even such plants displaying a stable transgene expression pattern through the generations can become subsequently silenced under naturally occurring conditions, such as a stress or pathogen attack. Existing approaches aiming at improved expression control, such as use of scaffold attachment regions (Allen, G.C., 1996, Plant Cell, 8, 899-913; Clapham, D., 1995, J. Exp. Bot., 46, 655-662; Allen, G.C., 1993, Plant Cell, 5, 603-613) flanking the transcription unit, could potentially increase the independency and stability of transgene expression by decreasing dependency from so-called "position effect variation" (Matzke & Matzke, 1998, Curr.Opin. Plant Biol., 1, 142-148; S.B. Gelvin, 1998, Curr. Opin. Biotechnol., 9, 227-232; WO 9844 139 A1; WO 006757 A1; EP 1 005 560 A1; AU 00,018,331 A1). However, they only provide a partial solution to the existing problem of designing plants with a required expression pattern of a transgene.

Gene silencing can be triggered as a plant defence mechanism by viruses infecting the plant (Ratcliff et al., 1997, Science, 276, 1558-1560; Al-Kaff et al., 1998, Science, 279, 2113-2115). In non-transgenic plants, such silencing is directed against the pathogen, but in transgenic plants it can also silence the transgene, especially when the transgene shares homology with a pathogen. This is a problem, especially when many different elements of viral origin are used in designing transcriptional vectors. An illustrative example is the recent publication by Al-Kaff and colleagues (Al-Kaff et al., 2000, Nature Biotech., 18, 995-999) who demonstrated that CaMV (cauliflower mosaic virus) infection of a transgenic plant can silence the BAR gene under the control of the CaMV-derived 35S promoter.

During the last years, the set of cis-regulatory elements has significantly increased and presently includes tools for sophisticated spatial and temporal control of transgene expression.
These include several transcriptional elements such as various promoters and transcription terminators as well as translational regulatory elements/enhancers of gene expression. In general, translation enhancers can be defined as cis-acting elements which, together with cellular trans-acting factors, promote the translation of the mRNA. Translation in eukaryotic cells is generally initiated by ribosome scanning from the 5' end of the capped mRNA. However, initiation of translation may also occur by a mechanism which is independent of the cap structure. In this case, the ribosomes are directed to the translation start codon by internal ribosome entry site (IRES) elements. These elements, initially discovered in picornaviruses (Jackson & Kaminski, 1995, RNA, 1, 985-1000), have also been identified in other viral and cellular eucaryotic mRNAs. IRESs are cis-acting elements that, together with other cellular *trans-*acting factors, promote assembly of the ribosomal complex at the internal start codon of the mRNA. This feature of IRES elements has been exploited in vectors that allow for expression of two or more proteins from polycistronic transcription units in animal or insect cells. At present, they are widely used in bicistronic expression vectors for animal systems, in which the first gene is translated in a cap-dependent manner and the second one is under the control of an IRES element (Mountford & Smith, 1995, Trends Genet., 4, 179-184; Martines-Salas, 1999, Curr Opin Biotech., 19, 458-464). Usually the expression of a gene under the control of an IRES varies significantly and is within a range of 6-100% compared to cap-dependent expression of the first one (Mizuguchi et al., 2000, Mol. Ther., 1, 376-382). These findings have important implications for the use of IRESs, for example for determining which gene shall be used as the first one in a bicistronic vector. The presence of an IRES in an expression vector confers selective translation not only under normal conditions, but also under conditions when cap-dependent translation is inhibited. This usually happens under stress conditions (viral infection, heat shock, growth arrest, etc.), normally because of the absence of necessary trans-acting factors (Johannes & Sarnow, 1998, RNA, 4, 1500-1513; Sonenberg & Gingras, 1998, Cur. Opin. Cell Biol., 10, 268-275).

Translation-based vectors recently attracted attention of researchers working with animal cell systems. There is one report connected with the use of an IRES-Cre recombinase cassette for obtaining tissue-specific expression of cre recombinase in mice (Michael et al., 1999, Mech. Dev., 85, 35-47). In this work, a novel IRES-Cre cassette was introduced into the exon sequence of the EphA2 gene, encoding an Eph receptor of protein tyrosine kinase expressed early in development. This work is of specific interest as it is the first demonstration of the use of translational vectors for tissue-specific expression of a transgene in animal cells that relies on transcriptional control of the host DNA. Another important application for IRES elements is their use in vectors for the insertional mutagenesis. In such vectors, the reporter or selectable marker gene is under the control of an IRES element and can only be expressed if it inserts within the transcribed region of a transcriptionally active gene (Zambrowich et al., 1998, Nature, 392, 608-611; Araki et al., 1999, Cell Mol Biol., 45, 737-750). However, despite the progress made in the application of IRESs in animal systems, IRES elements from these systems are not functional in plant cells. Moreover, since site-directed or homologous recombination in plant cells is extremely rare and of no practical use, similar approaches with plant cells were not contemplated.

There are significantly less data about plant-specific IRES elements. Recently, however, several IRESs that are also active in plants were discovered in tobamovirus crTMV (a TMV virus infecting *Cruciferae* plants) (Ivanov et al., 1997, Virology, 232, 32-43; Skulachev et al., 1999, Virology, 263, 139-154; WO 98/54342) and there are indications of IRES translation control in other plant viruses (Hefferon et al., 1997, J. Gen Virol., 78, 3051-3059; Niepel & Gallie, 1999, J. Virol., 73, 9080-9088). IRES technology has a great potential for the use in transgenic plants and plant viral vectors providing convenient alternative to existing vectors. Up to date, the only known application of plant IRES elements for stable nuclear transformation is connected with the use of IRESs to express a gene of interest in bicistronic constructs (WO 98/54342). The construct in question comprises, in 5' to 3' direction, a transcription promoter, the first gene linked to the said transcription promoter, an IRES element located 3' to the first gene and the -second gene located 3' to the IRES element, i.e., it still contains a full set of transcription control elements.

Surprisingly, we have found that translational vectors that are devoid of their own transcription control elements and rely entirely on insertion into a transcriptionally active genomic DNA of a plant host, allow recovery of numerous transformants which express the gene of interest. Even more surprisingly, such transformants could be easily detected even in host plants with a very low proportion of transcriptionally active DNA in their genome such as wheat. This finding is the basis of the proposed process that allows for design of transgene expression that is entirely controlled by the host's transcriptional machinery, thus minimizing the amount of xenogenetic DNA elements known to trigger transgene silencing. It also allows to control transgene expression in a novel way, by modulating the ratio of cap-dependent versus cap-independent translation.

### BRIEF DESCRIPTION OF THE DRAWINGS

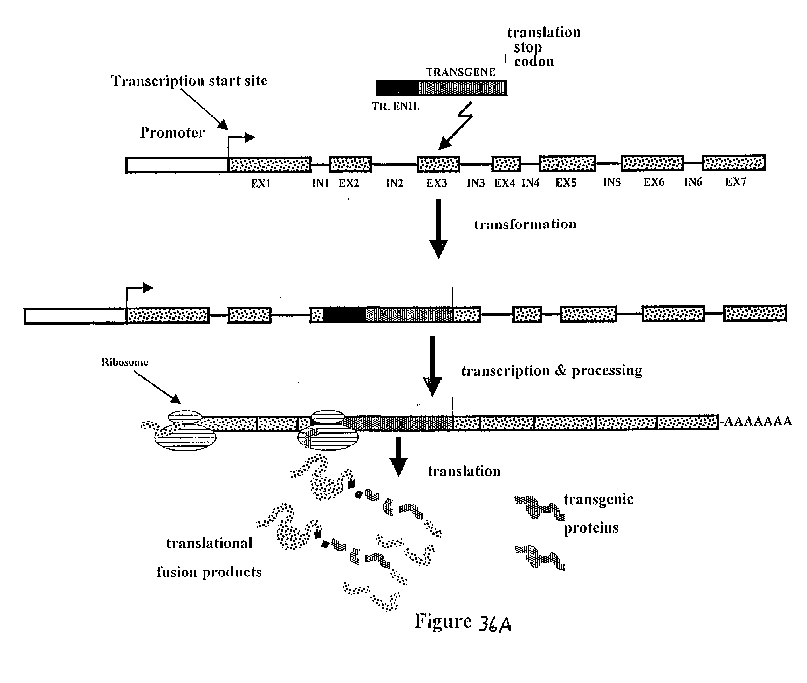

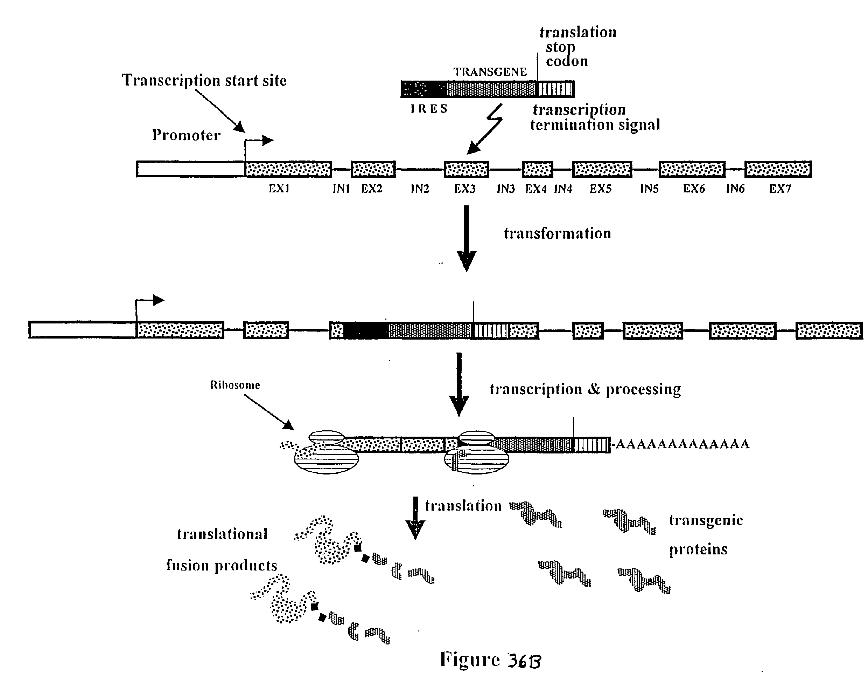

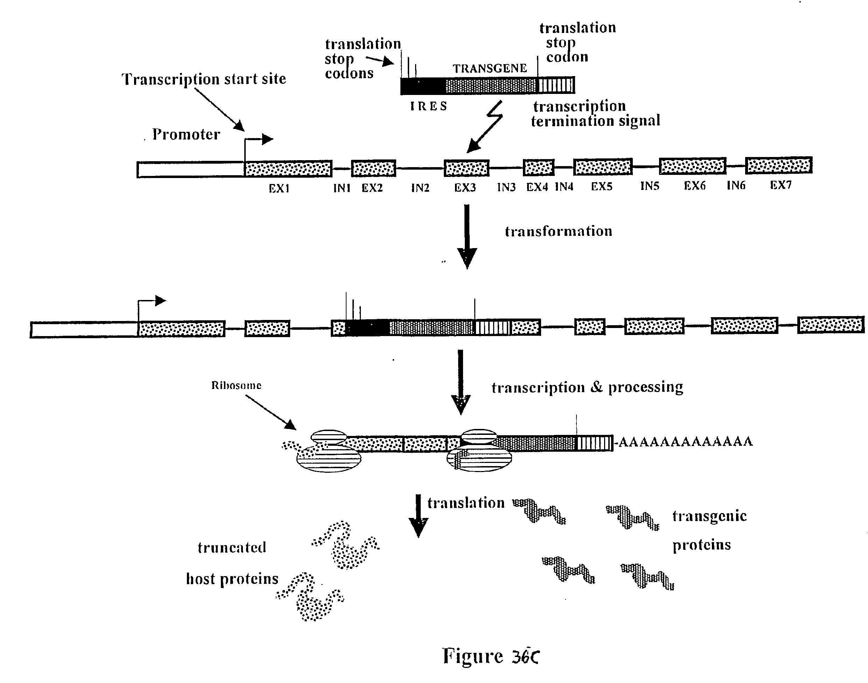

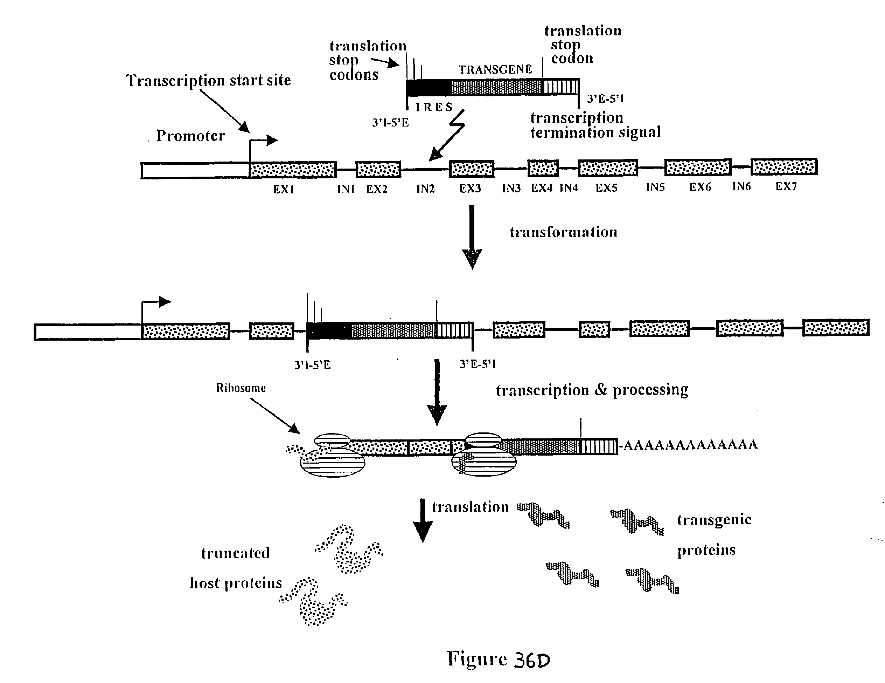

Fig. 36 shows transgene expression from four of many possible translational vector variants.
   A - the vector contains a translation enhancer and a translation stop codon;
   B - the vector contains an IRES as translation enhancer and a transcription termination region;
   C - as in B, except that the IRES is preceded by translation stop codons for all three reading frames;
   D - as in C, except that the vector is flanked by intron/exon boundary regions (3'I-5'E and 3'E-5'I) to provide the features of an exon and to facilitate its incorporation into the spliced mRNA.

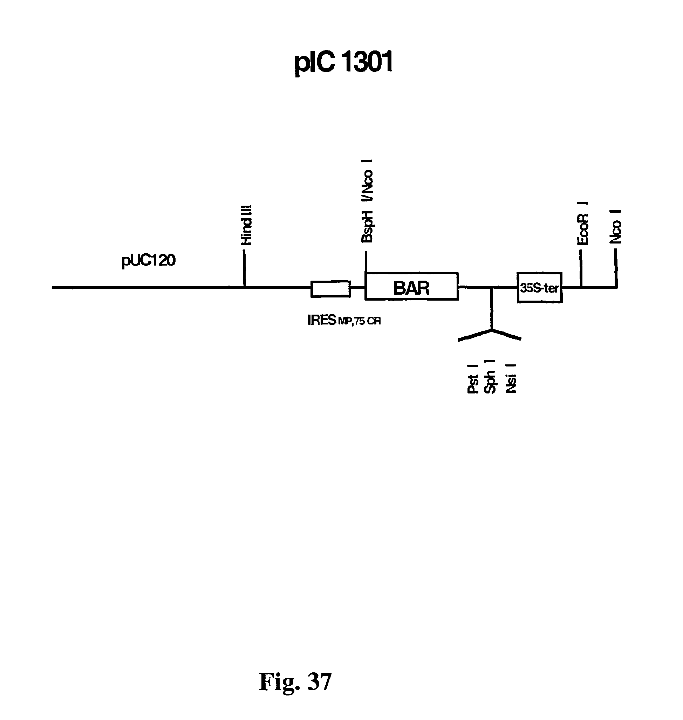

Fig. 37 depicts vector pIC1301 containing IRES_{MP,75}^{CR}, BAR and the 35S terminator.

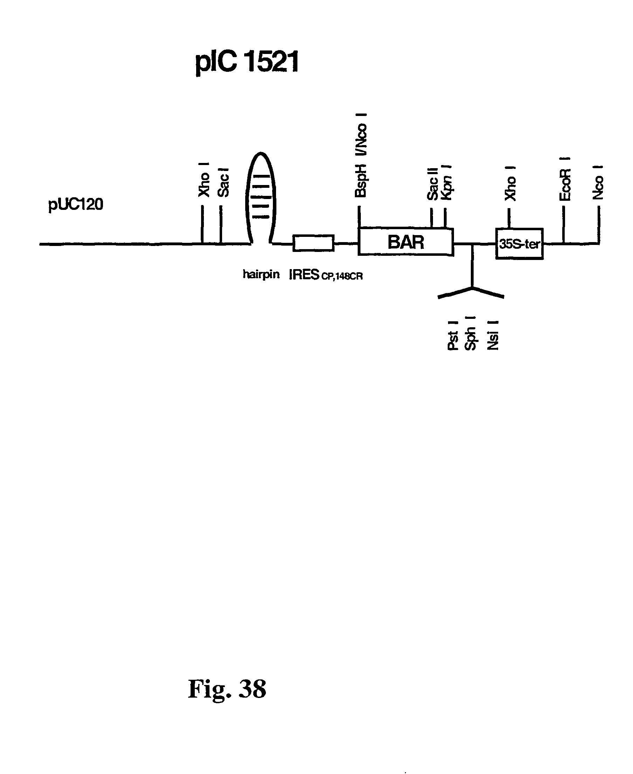

Fig. 38 depicts vector pIC1521 containing a "hairpin", IRES_{CP,148}^{CR}, BAR and the 35S terminator.
   The "hairpin" structure serves as an alternative to the translation stop codon, preventing the formation of the translational fusion products.

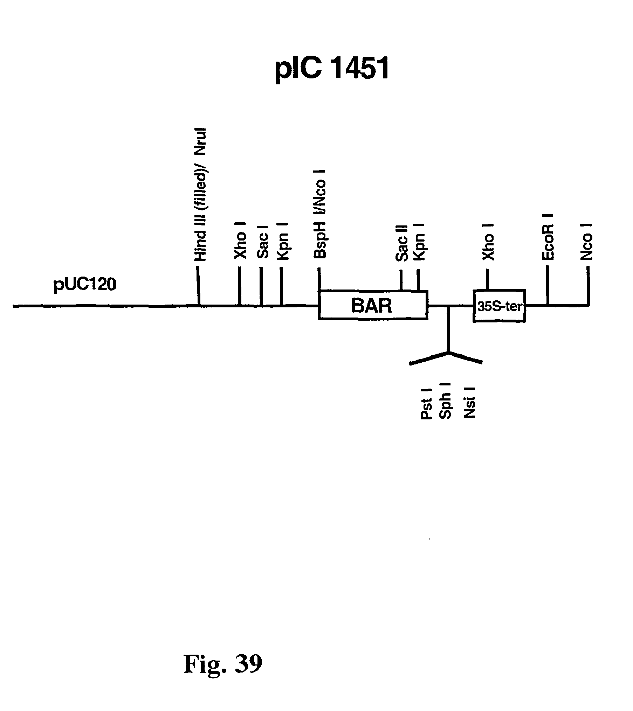

Fig. 39 depicts vector pIC1451 containing a promoterless BAR gene and the 35S terminator.

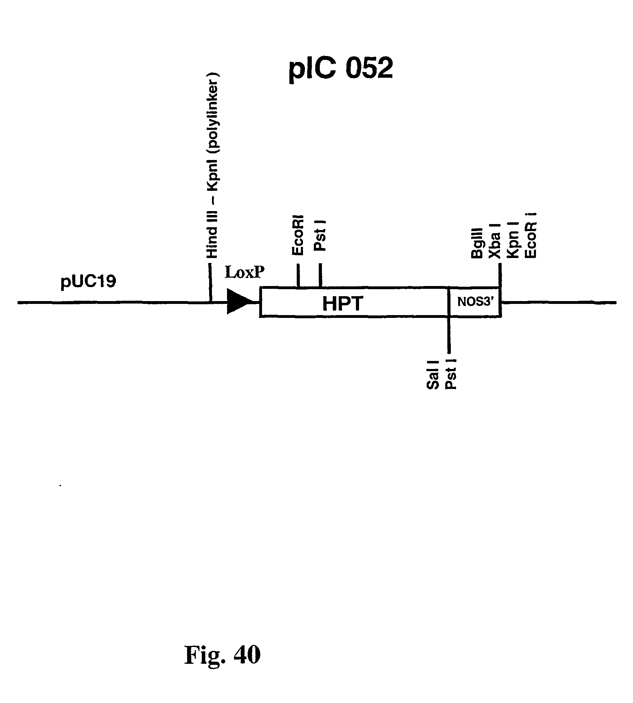

Fig. 40 depicts vector pIC052 containing IoxP, HPT and nos terminator.

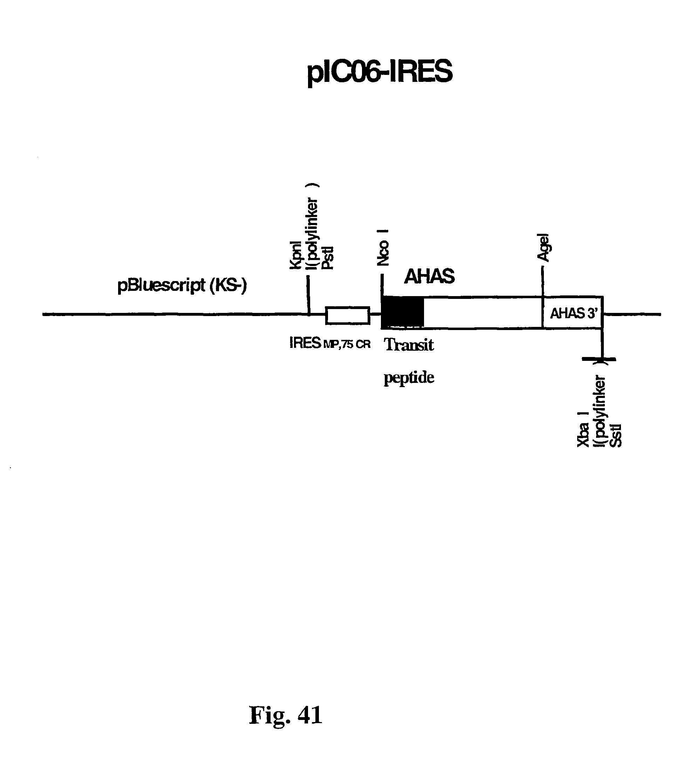

Fig. 41 depicts vector pIC06-IRES containing IRES_{MP7,5}^{CR}, the AHAS gene, whereby AHAS is the mutated version of the *Arabidopsis* acetohydroxyacid synthase gene conferring resistance to imidazoline herbicides.

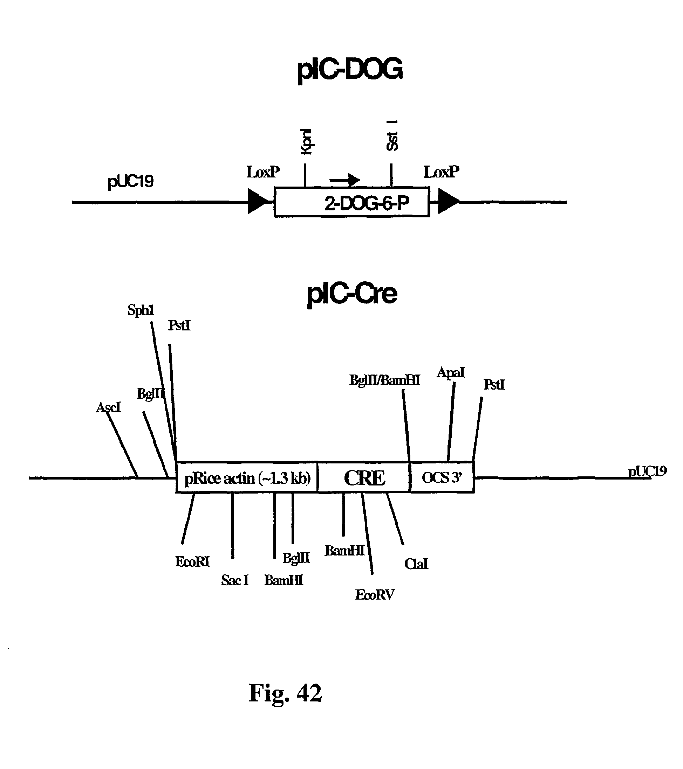

Fig. 42 depicts vectors pIC-DOG and pIC-CRE containing the coding sequence of the yeast 2-deoxyglucose-6-phosphate (2-DOG-6-P) phosphatase and cre recombinase under the control of the rice actin promoter, respectively.

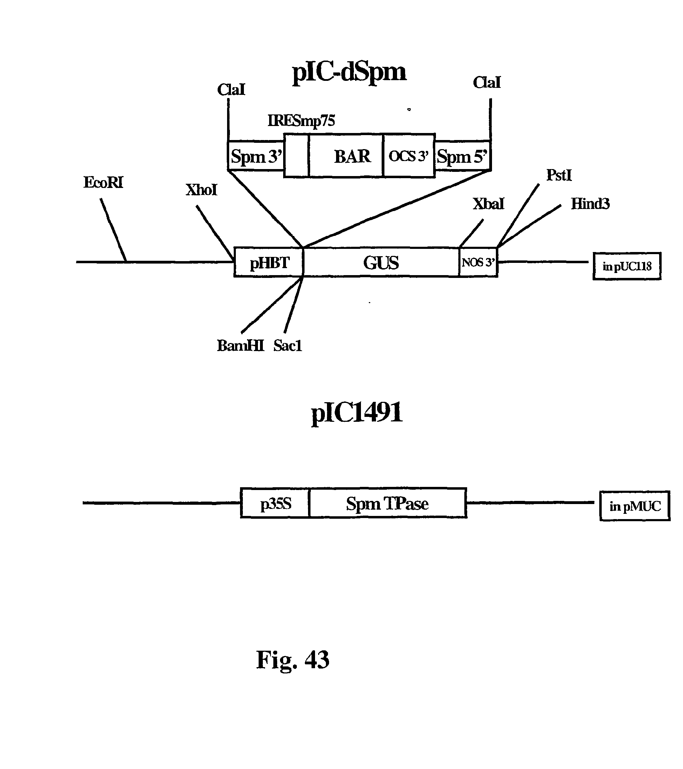

Fig. 43 depicts the transposon-incorporated translational vector pIC-dSpm and vector-pIC1491 containing a transposase. PHBT is a chimaeric promoter consisting of p35S enhancers fused to the basal part of the wheat C4PPDK gene.

### DETAILED DESCRIPTION

A primary objective is to provide a novel process or vector to produce transgenic plants for the stable expression of transgenic material integrated into a plant genome.

This object is achieved by a process for producing transgenic plants or plant cells capable of expressing a transgenic coding sequence of interest under transcriptional control of a host nuclear promoter by introducing into the nuclear genome a vector comprising in its transcript a sequence for binding a plant cytoplasmic ribosome in a form functional for initiation of translation and, downstream thereof, said transgenic coding sequence, and subsequently selecting plant cells or plants expressing said transgenic coding sequence. The gene of interest is under control of a translation signal, such as but not limited to, an IRES element and it has no promoter operably linked to it. Such vectors rely on transgene insertions into transcriptionally active DNA of the host genome.

Further a novel vector is described for transforming plant cells, comprising, optionally after processing in the host cell, in its transcript a sequence for binding a plant cytoplasmic ribosome in a form functional for the initiation of translation and, downstream thereof, a coding sequence, said vector being devoid of a promoter functional for the transcription of said coding sequence.

Construction of vectors for stable transformation of plants has been described by numerous authors (for review, see Hansen & Wright, 1999, Trends in Plant Science, 4, 226-231; Gelvin, S.B., 1998, Curr. Opin. Biotech., 9, 227-232). The basic principle of all these constructs is identical - a fully functional transcription unit consisting of, in 5'to 3'direction, a plant-specific promoter, a structural part of a gene of interest and a transcriptional terminator, has to be introduced into the plant cell and stably integrated into the genome in order to achieve expression of a gene of interest.

We have developed a different technology for obtaining stable nuclear transformants of plants, that relies on the surprising finding that the host plant transcription machinery is able to drive the formation of mRNA from a transgene of interest in a transformed plant cell.
The proposed process utilizes vectors having a gene of interest that is not operationally linked to a promoter in said vector. Rather, they comprise the coding region of a gene of interest under the control of translation elements only. Said translational element may be a sequence for binding, preferably after transcription, a plant cytoplasmic ribosome thus enabling translation of a coding sequence downstream thereof. Preferably, said translational element is a ribosome entry site functional in plants and more preferably a plant-specific IRES element, notably an IRES element of plant viral origin, of plant origin, of non-plant origin or an artificially designed IRES element.

Such a vector DNA, after integration into the transcribed region of a resident plant gene, yields chimaeric mRNA and is subsequently translated into the protein of interest via initiation of translation from said sequence for binding a plant cytoplasmic ribosome (Fig. 36). It was very surprising, that, given the low proportion of transcriptionally active DNA in most plant genomes, transformation experiments utilizing translation vectors described in the present invention, yielded numerous transformants expressing the gene of interest.

Our method addresses imminent problems of reliable transgene expression. The transgene integrated into host genome using our invented process, relies on the transcription machinery including all or most of the transcriptional regulatory elements of the host's resident gene, thus minimizing transgene silencing usually triggered by xenogenetic DNA elements.

The vectors for transgene delivery can be built in many different ways. The simplest versions consist only of the coding region of a gene of interest or a portion thereof with a translation signal (basic translational vector). In a preferred vector, a translational stop signal is provided upstream of said sequences for binding a plant cytoplasmic ribosome. The stop signal may for example be at least one stop codon and/or an RNA hairpin secondary structure or the like. This stop signal causes abortion of upstream translation. More advanced versions may include a plant-specific IRES element followed by the coding region (of a gene) of interest.
Advanced versions of the translational vector may include sequences for site-specific recombination (for review, see Corman & Bullock, 2000, Curr Opin Biotechnol., 11, 455-460) allowing either the replacement of an existing transgene or integration of any additional gene of interest into the transcribed region of the host DNA. Site-specific recombinases/integrases from bacteriophages and yeasts are widely used for manipulating DNA in vitro and in plants.
Examples for recombinases-recombination sites for the use in this invention include the following: cre recombinase-*LoxP* recombination site, FLP recombinase-*FRT* recombination sites, R recombinase-*RS* recombination sites, phiC31 integrase - *attP*/*attB* recombination sites etc.

The introduction of splicing sites into the translation vector may be used to increase the probability of transgene incorporation into the processed transcript.

The vector may further comprise a sequence coding for a targeting signal peptide between said sequence for binding a plant cytoplasmic ribosome and said coding sequence.
Preferable examples of such signal peptides include a plastid transit peptide, a mitochondrial transit peptide, a nuclear targeting signal peptide, a vacuole targeting peptide, and a secretion signal peptide.

Various methods can be used to deliver translational vectors into plant cells, including direct introduction of said vector into a plant cell by means of microprojectile bombardment, electroporation or PEG-mediated treatment of protoplasts. Agrobacterium-mediated plant transformation also presents an efficient way of the translational vector delivery. The T-DNA insertional mutagenesis in *Arabidopsis* and *Nicotiana* with the promoterless reporter APH(3')II gene closely linked to the right T-DNA border showed that at least 30% of all inserts induced transcriptional and translational gene fusions (Koncz et al., 1989, Proc. Natl. Acad. Sci., 86, 8467-8471).

A translational vector can also be cloned into transposable elements, facilitating the search for suitable transcribed regions and providing either a constitutive or tissue/organ-specific pattern of transgene expression. Transposable elements are extensively used in plants with the purpose of inactivation-based gene tagging (Pereira & Aarts, 1998, Methods Mol Biol., 82, 329-338; Long & Coupland, 82, 315-328; Martin GB., 1998, Curr Opin Biotechnol., 9, 220-226). Different versions of the transposon-tagging systems were developed. In the simplest version, transposons are used for insertional mutagenesis without any modifications except, possibly, for deletions or frame-shift mutations in order to generate non-autonomous transposable elements. In more sophisticated versions, additional genes are inserted into the transposable elements, e.g. reinsertion markers, reporter genes, plasmid-rescue vectors (Carroll et al., 1995, Genetics, 13, 407-420; Tissier et al., 1999, Plant Cell, 11, 1841-1852). There are so-called enhancer-trap and gene-trap systems (Sundaresan et al., 1995, Genes Dev., 9, 1797-810; Fedorov & Smith, 1993, Plant J., 3, 273-89). Transposable elements in such systems are equipped either with a promoterless reporter gene or a reporter gene under the control of a minimal promoter. In the first case, the reporter gene can be expressed following insertion into the transcribed region of host DNA just after the host promoter or insertion into the coding region of the host gene and creation of "in frame" fusion with the host gene transcript.

The chance for successful "in frame" fusion can be significantly increased by placing in front of the reporter gene a set of splicing donor and acceptor sites for all three reading frames ( Nussaume et al., 1995, Mol Gen Genet., 249, 91-101). In the second case, transcription of a reporter gene will be activated from the minimal promoter following insertion near the active host promoter (Klimyuk et al., 1995, Mol Gen Genet., 249, 357-65). The success of such approaches for transposon tagging favors the use of a similar approach for the translational vectors with IRES elements in front of the gene of interest.

All approaches described above aim at designing a system that places a transgene under expression control of the resident gene in which the insertion occurred. This might be advantageous for specific tasks and cases. In many other cases, a modified pattern of transgene expression might be preferable. For such purposes, the translational vector can be equipped with transcriptionally active elements, such as enhancers which can modulate the expression pattern of a transgene. It is known that enhancer sequences can affect the strength of promoters located as far as several thousand base pairs away (Müller, J., 2000, Current Biology, 10, R241-R244). The feasibility of such an approach was demonstrated in experiments with activation tagging in *Arabidopsis* (Weigel et al., 2000, Plant Physiol., 122, 1003-1013), where T-DNA-located 35S enhancer elements changed the expression pattern of resident genes, and in enhancer-trap transposon tagging described above. In the latter example, resident gene enhancers determined the expression pattern of the reporter transgene. This approach might be useful, for example, at the initial stages of plant transformation, or when modulation of the transgene expression pattern is required after the transformation. The enhancer sequences can be easily manipulated by means of sequence-specific recombination systems (inserted, replaced or removed) depending on the needs of the application.

Our approach was to preferably make a set of constructs based on different IRES elements functional in plant cells. The constructs contain IRES elements followed by a plant selectable marker gene and a transcription/translation termination signal. These constructs can be used directly for plant cells transformation after being linearized from the 5' end in front of the IRES sequences or can be cloned into the T-DNA for *Agrobacterium*-mediated DNA transfer. Another set of constructs, serving as controls, contained either a promoterless selectable gene (a negative control) or a selectable gene under the control of a constitutive promoter functional in monocot and/or dicot cells (a positive control). DNA was transformed into plant cells using different suitable technologies, such as Ti-plasmid vector carried by *Agrobacterium* (US 5,591,616; US 4,940,838; US 5,464,763), particle or microprojectile bombardment (US 05100792; EP 00444882 B1; EP 00434616 B1). In principle, other plant transformation methods could be used, such as but not limited to, microinjection (WO 09209696; WO 09400583 A1; EP 175966 B1), electroporation (EP 00564595 B1; EP 00290395 B1; WO 08706614 A1 ).

The transformation method depends on the plant species to be transformed. Our exemplification includes data on the transformaction efficiency for representatives of monocot (e.g. *Triticum monococcum*) and dicot (e.g. *Brassica napus, Orichophragmus violaceous*) plant species, thus demonstrating the feasibility of our approach for plant species of different phylogenetic origin and with different densities of transcribed regions within a species genome.
The transgenic coding sequence in the vector may represent only part of a gene of interest, which gene is reconstructed to a functional length as a result of site-directed or homologous recombination. The translation of the sequence of interest is preferably cap-independent. The host may be modified for inhibiting (or enhancing) cap-dependent translation or for enhancing (or inhibiting) cap-independent translation. This may be accomplished by treatment with exogenous agents or by including a sequence in the vector or said plant, which expression has the desired effect.

### EXAMPLES

### EXAMPLE 1

### Construction of IRES containing vectors

Series of IRES-mediated expression vectors were constructed using standard molecular biology techniques (Maniatis et al., 1982, Molecular cloning: a Laboratory Manual. Cold Spring Harbor Laboratory, New York). Vector pIC1301 (Fig. 37) was made by digesting plasmid pIC501 (p35S-GFP-IRES_{MP,75}^{CR} -BAR-35S terminator in pUC120) with HindIII and religating large gel-purified fragment. The IRES_{MP,75}^{CR} sequence represents the 3' terminal 75 bases of the 5'-nontranslated leader sequence of the subgenomic RNA of the movement protein (MP) of a crucifer (CR)-infecting tombamovirus.

Vector pIC1521 (Fig. 38) was made following three steps of cloning. In the first step pIC1311 was constructed by ligating the large HindIII-PstI fragment of pIC031 with the small HindIII-NcoI fragment of pIC032 and the small BspHI-Pstl fragment of pIC018. The resulting construct pIC1311 (not shown) containing the BAR gene under the control of the 35S promoter was used as the comparative control in the transformation experiments. Plasmid pIC1311 was digested with Hindlll- Nrul and blunt-ended by treatment with Klenow fragment of DNA polymerase I. The large restriction fragment was gel-purified and religated producing pIC1451 (promoterless BAR-35S terminator; see Fig. 39). Ligation of the large Sac1-Pst1 fragment of pIC1451 with the small Sacl-Ncol fragment of pIC033 and the small BspHI-Pstl fragment of pIC018 produced pIC1521 (Fig. 38). This construct contains a "hairpin" in front of the IRES_{cp,148}^{CR} (CP stands for coat protein) element. The "hairpin" structure is formed by the presence of an inverted tandem repeat formed by KpnI-EcoRI and Clal-Kpnl fragments from the Bluescript II SK+ polylinker sequence.

All vectors were linearized for use in the transformation experiments by digesting either with SacI (pIC1521; pIC1451) or HindIII (pIC1311; pIC1301) restriction enzyme and gel-purified to separate from undigested vectors.

### EXAMPLE 2

### PEG-mediated protoplast transformation of Brassica napus Isolation of protoplasts

The isolation of *Brassica* protoplasts was based on previously described protocols (Glimelius K., 1984, Physiol.Plant., 61, 38-44; Sundberg & Glimelius, 1986, Plant Science, 43, 155-162 and Sundberg et al., 1987, Theor. Appl. Genet., 75, 96-104).

Sterilized seeds (see Appendix) were germinated in 90 mm Petri dishes containing ½ MS medium with 0.3% Gelrite. The seeds were placed in rows slightly separated from each other. The Petri dishes were sealed, tilted at an angle of 45° and kept in the dark for 6 days at 28°C. The hypocotyls were cut into 1-3 mm long peaces with a sharp razor blade. The blades were often replaced to avoid the maceration of the material. The peaces of hypocotyls were placed into the TVL solution (see Appendix) to plasmolise the cells. The material was treated for 1-3 hours at room temperature. This pre-treatment significantly improves the yield of intact protoplasts. The preplasmolysis solution was replaced with 8-10 ml of enzyme solution (see Appendix). The enzyme solution should cover all the material but should not to be used in excess. The material was incubated at 20-25°C in dark for at least 15 hours. The Petri dishes were kept on a rotary shaker with very gentle agitation.

The mixture of protoplasts and cellular debris was filtered through 70 mm mesh size filter. The Petri dishes were rinsed with 5-10 ml of W5 solution (Menczel et al., 1981, Theor. Appl. Genet., 59, 191-195) (also see Appendix) that was also filtered and combined with the rest of the suspension. The protoplast suspension was transferred to 40 ml sterile Falcon tubes and the protoplasts were pelleted by centrifugation at 120 g for 7 min. The supernatant was removed and the pellet of protoplasts was re-suspended in 0.5 M sucrose. The suspension was placed into 10 ml sterile centrifuge tubes (8 ml per tube) and loaded with 2 ml of W5 solution. After 10 min of centrifugation at 190 g the intact protoplasts were collected from the interphase with a Pasteur pipette. They were transferred to new centrifuge tubes, resuspended in 0.5 M mannitol with 10 mM CaCl₂ and pelleted at 120 g for 5 min.

### PEG treatment

The protoplasts were resuspended in the transformation buffer (see Appendix). The protoplast concentration was determined using the counting chamber and than adjusted to 1-1.5x10⁶ protoplasts/ml. The 100 µl drop of this suspension was placed at the lower edge of the tilted 6-cm Petri dish and left for a few minutes allowing the protoplasts to settle. The protoplasts were than gently mixed with 50-100 µl of DNA solution (Qiagen purified, dissolved in TE at the concentration 1 mg/ml). Than 200 µl of PEG solution (see Appendix) was added dropwise to the protoplasts/DNA mixture. After 15-30 min the transformation buffer (or W5 solution) was added in small aliquots (dropwise) until the dish was almost filled (∼6 ml). The suspension was left to settle for 1-5 hours. Then the protoplast were transferred to the centrifuge tubes, re-suspended in W5 solution and pelleted at 120 g for 5-7 min.

### Protoplast culture and selection for transformants

The protoplasts were transferred to the culture media 8pM (Kao & Michayluk, 1975, *Planta,* 126, 105-110; also see the Appendix) and incubated at 25 °C, low light density, in 2.5 cm or 5 cm Petri dishes with 0.5 ml or 1.5 ml of media, respectively. Protoplast density was 2.5x10⁴ protoplasts/ml. The three volumes of fresh 8pM media without any hormones were added right after the first protoplasts division. The cells were incubated at high light intensity, 16 hours per day.

After 10-14 days the cells were transferred to K3 media (Nagy & Maliga, 1976, Z. Pflanzenphysiol., 78, 453-455) with 0.1 M sucrose, 0.13% agarose, 5-15 mg/L of PPT and the hormone concentration four times less than in 8pM medium. To facilitate the transfer to fresh media, the cells were placed on the top of sterile filter paper by carefully spreading them in a thin layer. The cells were kept at high light intensity, 16 hours per day. The cell colonies were transferred to Petri dishes with differentiation media K3 after their size had reached about 0.5 cm in diameter.

### EXAMPLE 3

### Transformation of Triticum monococcum by microprojectile bombardment

### Plant cell culture

Suspension cell line of T. *monococcum* L. was grown in MS2 ( MS salts (Murashige & Skoog, 1962 Physiol. Plant., 15, 473-497), 0.5 mg/L Thiamine HCl, 100 mg/L inosit, 30 g/L sucrose, 200 mg/L Bacto-Tryptone, 2 mg/L 2,4-D) medium in 250 ml flasks on a gyrotary shaker at 160 rpm at 25°C and was subcultured weekly. Four days after a subculture the cells were spread onto sterile 50 mm filter paper disks on a gelrite-solidified (4 g/L) MS2 with 0.5 M sucrose.

### Microproiectile bombardment

Microprojectile bombardment was performed utilizing the Biolistic PDS-1000/He Particle Delivery System (Bio-Rad). The cells were bombarded at 900-1100 psi, with 15 mm distance from a macrocarrier launch point to the stopping screen and 60 mm distance from the stopping screen to a target tissue. The distance between the rupture disk and a launch point of the macrocarrier was 12 mm. The cells were bombarded after 4 hours of osmotic pretreatment.

A DNA-gold coating according to the original Bio-Rad's protocol (Sanford et al., 1993, In: Methods in Enzymology, ed. R.Wu, 217, 483-509) was done as follows: 25 µl of gold powder (0.6, 1.0 mm) in 50% glycerol (60 mg/ml) was mixed with 5 µl of plasmid DNA at 0.2 µg/µl, 25 µl CaCl₂ (2.5 M) and 10 µl of 0.1 M spermidine. The mixture was vortexed for 2 min followed by incubation for 30 min at room temperature, centrifugation (2000 rpm, 1 min), washing by 70% and 99.5% ethanol. Finally, the pellet was resuspended in 30 µl of 99.5% ethanol (6 µl/shot). A new DNA-gold coating procedure (PEG/Mg) was performed as follows: 25 µl of gold suspension (60 mg/ml in 50% glycerol) was mixed with 5 µl of plasmid DNA in an Eppendorf tube and supplemented subsequently by 30 µl of 40% PEG in 1.0 M MgCl₂. The mixture was vortexed for 2 min and than incubated for 30 min at room temperature without mixing. After centrifugation (2000 rpm, 1 min) the pellet was washed twice with 1 ml of 70% ethanol, once by 1 ml of 99.5% ethanol and dispersed finally in 30 µl of 99.5% ethanol. Aliquots (6 µl) of DNA-gold suspension in ethanol were loaded onto macrocarrier disks and allowed to dry up for 5-10 min.

### Plasmid DNA preparation

Plasmids were transformed into *E.coli* strain DH10B, maxi preps were grown in LB medium and DNA was purified using the Qiagen kit.

### Selection

For stable transformation experiments, the filters with the treated cells were transferred onto the solid MS2 medium with the appropriate filter-sterilized selective agent (150 mg/L hygromycin B (Duchefa); 10 mg/L bialaphos (Duchefa). The plates were incubated in the dark at 26°C.

### EXAMPLE 4

### Transformation of Orychophragmus violaceus by microprojectile bombardment

### Preparation of the suspension culture

Plants of *O*. *violaceus* are grown in vitro on MS medium, 0.3% Gelrite (alternatively, 1/2 MS, 2% sucrose and 0.8% agar) at 24° C and 16/8 hours day/night photoperiod for 3-4 weeks. Four-six leaves (depending of size) were cut into small peaces and transferred to the Magenta box with 30 ml of Callus Inducing Medium (CIM) (see the Appendix). The material was kept for 4-5 weeks at dim light (or in dark) at 24° C and vigorous agitation. During this period the fresh CIM media was added to keep the plant tissue in the Magenta box covered with liquid. The cells sticking to the wall of the Magenta box were released into the media by vigorous inverting and shaking of the box.

### Preparation of plant material for microprojectile bombardment

The aliquote of cell suspension was carefully placed onto the sterile filter paper supported by solid CIM media in Petri dish. The Petri dish with plant material was kept in the dark for 5-7 days. Four hours before the procedure, the filter paper with cells was moved to fresh CIM with 10% sucrose. Microprojectile bombardment was performed as described in Example 3. Fourteen hours after the bombardment the material was transferred to CIM with 3% sucrose and kept in the dark.

### Selection for transformants

Two-four days after the bombardment, the filter paper with cells was transferred to the plate with CIM supplemented with the appropriate selection agent (10-15 µg/ml PPT). Every seven days the material was transferred to fresh selection media. The plates were kept in the dark and after approximately 6 weeks the plant material was transferred to the Petri plates with Morphogenesis Inducing Medium (MIM) (see the Appendix) supplemented with the appropriate selection agent (10-15 µg/ml PPT). The plates were incubated at high light intensity, 16 hours day length.

### EXAMPLE 5

### Transformation of Triticum monococcum with promoterless IoxP-HPT gene

The construct pIC052 (Fig. 41) was linearized by digestion with HindIII restriction enzyme, gel-purified to separate undigested material and used for the microprojectile bombardment as described above (see EXAMPLE 3). The linearized vector contains pUC19 polylinker (57 bp) followed by a IoxP site from the 5' end of the HPT gene. In general, approximately 100 bp is located at the 5' end of translation start codon of HPT gene. Thirty four plates were transformed and after 1.5 months of selection on hygromycin-conatining media (EXAMPLE 3), three hygromycin resistant colonies were recovered. The sequence of the integration sites recovered by IPCR, confirmed the independency of all three transformants.

### EXAMPLE 6

### Transformation of Orychophragmus leaves with promoterless IRES_{MP,75}^{CR}-AHAS

Plant acetohydroxyacid synthase (AHAS) is a nuclear encoded, chloroplast targeted protein which catalyses the first step in the biosynthesis of the branched chain amino acids. It is under allosteric control by these amino acids and can be inhibited by several classes of herbicides.

The construct pIC06-IRES was made by replacing the promoter of the *Arabidopsis* AHAS(Ser653-Asn) gene (1.3 Kb Pstl-Ncol fragment) in pIC06 with the IRES_{MP,75}^{CR} sequence. The final construct (Fig. 41) contained the mutated version of the *Arabidopsis* acetohydroxyacid synthase (AHAS) gene with a single amino acid substitution (Ser653Asn) conferring resistance to the imidazoline herbicide family (Sathasivan, Haughn & Murai, 1991, Plant Physiol., 97, 1044-1050). The plasmid was linearized by treatment with Sall restriction enzyme and used for microprojectile bombardment of freshly induced *O. violaceous* suspension culture. Leaves of sterile *O. violaceous* plants were cut onto the small peaces and placed in the liquid High Auxin Medium (HAM) (see the Appendix) in Magenta boxes on a rotary shaker to induce suspension culture. After 7-14 days the suspension culture was transferred to the Petri dishes with Greening Medium (GM) covered by sterile filter paper (see the Appendix). After 3 days the filter paper with the cells was transferred on GM supplemented with 0.4 M sucrose. After four hours the cells were used for microprojectile bombardment with linearized DNA of pIC06-IRES, as described in EXAMPLE 3. After 14 hours the filter paper with cells was transferred to GM, 3% sucrose. Two days later the cells were transferred to GM with 0.7 µM imazethapyr (AC263, 499 or Pursuit, American Cyanamid). The cells were subcultured every 7-10 days. Putative events were identified after approximately four - six weeks and the transformants were selected under high light intensity, 16 hours per day, on the regeneration medium (RM) with 1-2 µM imazethapyr.

### EXAMPLE 7.

### Expression of 2-DOG-6-P gene using translational vector

The aim of this example is to demonstrate the possibility of manipulation with transgenic plant cells already containing translational vector sequences with the sequence-specific recombination sites.

The hygromycin-resistant *T*. *monococcum* cells transformed with vector pIC052 (EXAMPLE 5) were used for microprojectile co-bombardment with two plasmids, pIC-DOG and pIC-CRE (Fig. 42). Plasmid pIC-DOG contains promoterless 2-deoxyglucose-6-phosphate (2-DOG-6-P) phosphatase cDNA (patent WO 98/45456) flanked by two IoxP sites. Cre-mediated integration of the 2-DOG-6-P gene into the IoxP site of pIC052-containing transformants leads to the expression of 2-DOG-6-P from a resident promoter. Such expression confers resistance to 2-deoxyglucose (2-DOG). The resistant colonies were selected as described in EXAMPLE 3, but using 0.075 - 0.1% of 2-DOG as the selective agent.

### EXAMPLE 8.

### Transposon-incorporated translational vector

The aim of this example is to show an alternative way to the direct transformation of directing translational vector to a desired transcriptional site in a host genome.

Co-transformation of *O. violaceous* cells with the constructs shown in Fig. 43 and selection for transformants was performed as described in EXAMPLE 4. The non-autonomous transposable d*Spm* element contains a promoterless BAR gene preceeded from its 5' end IRES_{MP,75}^{CR}. The transposition induced by Spm transposase facilitates the search for transcriptionally active regions with a desired expression pattern (in this case - constitutive) in said host genome, thus increasing the number of recovered primary transformants. Indeed, the number of transformants was 3-4 times higher than with the IRES_{MP,75}^{CR}-BAR gene alone (pIC1301, Fig. 37).

### Appendix

### Seed sterilization

Soak the seeds in 1% PPM solution for at least 2 hours (overnight is preferable). Wash the seeds in 70% EtOH for 1 minute than sterilize in 10% chlorine solution with 0.01% SDS or Tween 20) in 250 ml flask placed on the rotary shaker. Wash the seeds in 0.5 L of sterile water.

| TVL | Enzyme solution |
|---|---|
| 0.3 M sorbitol | 1% cellulase R10 |
| 0.05 M CaCl₂x2H₂O | 0.2% macerase R10 |
| pH 5.6-5.8 | 0.1 % dricelase |
| | dissolved in 8pM macrosalt with 0.5 M pH 5.6-5.8 |

| W5 | PEG solution |
|---|---|
| 18.4 g/L CaCl₂x2H₂O | 40% (w/v) of PEG-2000 in H₂O |
| 9.0 g/L NaCl | |
| 1.0 g/L glucose | |
| 0.8 g/L KCI | |
| pH 5.6-5.8 | |

| CIM | | MIM | |
|---|---|---|---|
| Macro MS | | Macro MS | |
| Micro MS | | Micro MS | |
| Vitamin B5 | | Vitamin B5 | |
| MES | 500 mg/L | MES | 500 mg/L |
| PVP | 500 mg/L | PVP | 500 mg/L |
| Sucrose | 30 g/L | Sucrose | 30 g/L |
| 2.4-D | 5 mg/L | ABA | 1 mg/L |
| Kin | 0.25 mg/L | BA | 0.5 mg/L |
| Gelrite | 3g/L | IAA | 0.1 mg/L |
| pH | 5.6-5.8 | Gelrite | 3 g/L |
| | | pH | 5.6-5.8 |

| Greening Medium (GM) | | High Auxine Medium (HAM) | |
|---|---|---|---|
| Macro MS | | Macro MS | |
| Micro MS | | Micro MS | |
| Vit B5 | | Vit B5 | |
| MES | 500mg/L | MES | 500 mg/L |
| PVP | 500 mg/L | PVP | 500 mg/L |
| Sucrose | 30 g/L | Sucrose | 30 g/L |
| BA | 2 mg/L | NAA | 5 mg/L |
| Kin | 0.5 mg/L | Kin | 0.25 mg/L |
| NAA | 0.1 mg/L | BA | 0.25 mg/L |
| pH | 5.6-5.8 | pH | 5.6-5.8 |

| Regeneration Medium | |
|---|---|
| Macro MS | |
| Micro MS | |
| Vit B5 | |
| MES | 500 mg/L |
| PVP | 500 mg/L |
| Sucrose | 30 g/L |
| ABA | 1 mg/L |
| BA | 0.5 mg/L |
| IAA | 0.1 mg/L |
| pH | 5.6-5.8 |

Hormone solutions were filter sterilized and added to the autoclaved media.

## Claims

1. A process of controlling a biochemical process (II) or biochemical cascade (III) of interest in a plant, said process being **characterized by** comprising the following steps:
(a) introducing into the nuclear genome of the plant one or more first heterologous DNA sequences,
(b) infecting the plant with at least one viral transfection vector containing in its genome one or more second heterologous DNA or RNA sequences, thus triggering a process of interaction (I) in the plant between
(i) expression products of the first heterologous DNA sequence(s), and
(ii) one or more second heterologous DNA or RNA sequences of the transfection vector and/or expression products of the second heterologous DNA or RNA sequences, and
(iii) optionally one or more externally added low molecular weight components, thus switching on the biochemical process (II) or biochemical cascade (III) of interest that was not operable prior to said interaction.

2. The process according to claim 1, wherein the infection of the plant in step (b) is achieved by an assembled virus particle or infectious viral nucleic acids, or by activating a transfection process by release of viral nucleic acids that were previously incorporated into the plant genome.

3. The process of claim 2, wherein said assembled virus particle or said infectious viral nucleic acid is or comprises RNA.

4. The process according to claim 1, wherein the infection of the plant in step (b) comprises *Agrobacterium*-mediated transfer of nucleic acid sequences into cells of said plant.

5. The process according to any one of claims 1 to 4, wherein a further vector is introduced in step (b) and wherein a sequence and/or an expression product of said further vector is involved in said process of interaction.

6. The process according to any one of claims 1 to 5, wherein the infection of the plant in step (b) is achieved by introducing one or more vectors into cells of said plant, whereby said vector(s) are adapted to undergo processing to generate said viral transfection vector in cells of said plant.

7. The process according to one of claims 1 to 6, wherein said process of interaction is a viral transfection vector-generating process.

8. The process according to any one of claims 1 to 7, wherein the process of interaction involves DNA transposition.

9. The process according to any one of claims 1 to 8, wherein the process of interaction involves DNA recombination.

10. The process according to claim 9, wherein the biochemical process or cascade of interest comprises expression of a first or second DNA or RNA sequence comprising a promoterless gene in anti-sense orientation which is placed into sense orientation towards a constitutive promoter in said process of interaction.

11. The process according to any one of claims 1 to 7, wherein the process of interaction involves recognition of a heterologous promoter by a heterologous RNA polymerase.

12. The process according to claim 11, wherein said first or said second DNA or RNA sequence comprises a heterologous sequence to be expressed under the control of a heterologous promoter not recognized by a plant RNA polymerase, and transcription of said sequence to be expressed is switched on by interaction of said promoter with an RNA polymerase functional therewith and being encoded by said second or said first DNA sequence, respectively.

13. The process according to claim 12, wherein said RNA polymerase is a bacteriophage RNA polymerase and said heterologous promoter is a bacteriophage promoter.

14. The process according to any one of claims 1 to 13, wherein the process of interaction involves a DNA reaction such as DNA replication, ligation, hybridisation, transcription, or DNA restriction.

15. The process according to one of claims 1 to 14, wherein the process of interaction involves an RNA reaction such as replication, processing, splicing, reverse transcription, hybridization or translation, or activation, inhibition or modification thereof.

16. The process according to any one of claims 1 to 15, wherein the process of interaction involves a protein reaction such as protein folding, assembly, activation, posttranslational modification, targeting, binding, enzymatic activity or signal transduction, or activation, inhibition or modification thereof.

17. The process according to one of claims 1 to 9, wherein
(i) the biochemical process or cascade of interest comprises expression of the second DNA sequence separated from its promoter by a DNA insert capable of preventing transcription of the second DNA sequence, and
(ii) the process of interaction triggered in step (b) results in the excision of the DNA insert whereby the second DNA sequence is expressed.

18. The process according to claim 17, wherein the DNA insert is a non-autonomous transposable element which is excised by a transposase encoded by a first DNA sequence in the nuclear genome for an insert in the viral vector.

19. The process according to claim 17, wherein the DNA insert is flanked by unidirectional sites recognizable by a site-specific DNA recombinase encoded by a first DNA sequence in the nuclear genome for an insert in the viral vector.

20. The process according to one of claims 1 to 7, wherein transcription of the second DNA sequence is switched on by a heterologous or engineered transcription factor capable of recognizing a heterologous or engineered or chimaeric promoter operably linked to a heterologous gene of interest of said second DNA sequence, whereby said promoter is not recognizable by any natural plant transcription factor and said heterologous or engineered transrciption factor is encoded by the first DNA sequence.

21. The process according to claim 20, wherein the transcription factor is inducible by an externally applied low molecular weight component.

22. The process according to one of claims 1 to 21, wherein said first heterologous DNA sequence of step (a) is not of plant viral origin.

23. A process of controlling a biochemical process (II) or biochemical cascade (III) of interest in a plant, said process being **characterized by** comprising the following steps:
(a) introducing into the nuclear genome of the plant one or more first heterologous DNA sequences,
(b) infecting the plant with at least one viral transfection vector containing in its genome one or more second heterologous DNA or RNA sequences, thus triggering a process of interaction (I) in the plant between
(i) one or more first heterologous DNA sequences of the nuclear genome and/or expression products of the first heterologous DNA sequences, and
(ii) expression products of the second heterologous DNA or RNA sequence(s), and
(iii) optionally one or more externally added low molecular weight components, wherein the process of interaction involves a process selected from the following group: DNA replication, DNA ligation, DNA hybridisation, transcription, DNA restriction, RNA replication, RNA processing, RNA splicing, reverse transcription, RNA hybridization, RNA translation, RNA activation, RNA inhibition, RNA modification thereof, protein folding, protein assembly, protein activation, protein posttranslational modification, protein targeting, protein binding, enzymatic activity, protein signal transduction, or protein activation, inhibition or modification thereof, thus switching on the biochemical process (II) or biochemical cascade (III) of interest that was not operable prior to said interaction.

24. The process according to claim 23, wherein the infection of the plant in step (b) is achieved by an assembled virus particle or infectious viral nucleic acids, or by activating a transfection process by release of viral nucleic acids that were previously incorporated into the plant genome.

25. The process of claim 24, wherein said assembled virus particle or said infectious viral nucleic acid is or comprises RNA.

26. The process according to any one of claims 23 to 25, wherein the infection of the plant in step (b) comprises *Agrobacterium*-mediated transfer of nucleic acid sequences into cells of said plant.

27. The process according to any one of claims 23 to 26, wherein a further vector is introduced in step (b) and wherein a sequence and/or an expression product of said further vector is involved in said process of interaction.

28. The process according to any one of claims 23 to 27, wherein the infection of the plant in step (b) is achieved by introducing one or more vectors into cells of said plant, whereby said vector(s) are adapted to undergo processing to generate said viral transfection vector in cells of said plant.

29. The process according to one of claims 23 to 28, wherein said process of interaction is a viral transfection vector-generating process.

30. The process according to any one of claims 23 to 29, wherein the process of interaction involves DNA transposition.

31. The process according to claim 23 to 29, wherein the biochemical process or cascade of interest comprises expression of a first or second DNA or RNA sequence comprising a promoterless gene in anti-sense orientation which is placed into sense orientation towards a constitutive promoter in said process of interaction.

32. The process according to any one of claims 23 to 29, wherein the process of interaction involves recognition of a heterologous promoter by a heterologous RNA polymerase.

33. The process according to claim 32, wherein said first or said second DNA or RNA sequence comprises a heterologous sequence to be expressed under the control of a heterologous promoter not recognized by a plant RNA polymerase, and transcription of said sequence to be expressed is switched on by interaction of said promoter with an RNA polymerase functional therewith and being encoded by said second or said first DNA sequence, respectively.

34. The process according to claim 33, wherein said RNA polymerase is a bacteriophage RNA polymerase and said heterologous promoter is a bacteriophage promoter.

35. The process according to one of claims 23 to 30, wherein
(i) the biochemical process or cascade of interest comprises expression of a first or second DNA sequence separated from its promoter by a DNA insert capable of preventing transcription of the first or second DNA sequence, and
(ii) the process of interaction triggered in step (b) results in the excision of the DNA insert whereby the first or second DNA sequence is expressed.

36. The process according to claim 35, wherein the DNA insert is a non-autonomous transposable element which is excised by a transposase encoded by a second DNA sequence on the viral vector for an insert in the nuclear genome.

37. The process according to one of claims 23 to 29, wherein transcription of the first DNA sequence is switched on by a heterologous or engineered transcription factor capable of recognizing a heterologous or engineered or chimaeric promoter operably linked to a heterologous gene of interest of said second DNA sequence, whereby said promoter is not recognizable by any natural plant transcription factor and said heterologous or engineered transrciption factor is encoded by the second DNA sequence.

38. The process according to claim 37, wherein the transcription factor is inducible by an externally applied low molecular weight component.

39. The process according to one of claims 23 to 38, wherein said first heterologous DNA sequence of step (a) is not of plant viral origin.

40. A process of controlling a biochemical process (II) or biochemical cascade (III) of interest in a plant, said process being **characterized by** comprising the following steps:
(a) introducing into the nuclear genome of the plant one or more first heterologous nucleic acid sequences,
(b) infecting the plant with two or more vectors containing in their genome one or more second heterologous nucleic acid sequences, thus triggering a process of interaction (I) in the plant between
(i) a site-specific DNA recombinase as an expression product of the first heterologous nucleic acid sequences, and
(ii) two or more second heterologous nucleic acid sequences of said vectors as elements of a viral transfection vector;
whereby a viral transfection vector is generated in cells of said plant by site-specific recombination, thus switching on the biochemical process (II) or biochemical cascade (III) of interest that was not operable prior to said interaction.

41. The process of claim 40, further **characterized by** features as defined in one of claims 1 to 39.

42. Process of producing a product in a transgenic plant comprising the steps of the process of one of claims 1 to 40.

43. The process of claim 42 further comprising the following steps:
(a) growing the transgenic plant to a desired stage, followed by
(b) infecting the plant with one or more viral transfection vectors, and optionally contacting the plant with one or more low molecular weight components, thus switching on the biochemical process or cascade necessary for the production of the product, said process or cascade not being operable prior to said interaction, and
(c) producing the product in the plant.

## Patentansprüche

1. Verfahren zur Kontrolle eines gewünschten biochemischen Prozesses (II) oder einer gewünschten biochemischen Kaskade (III) in einer Pflanze, das **dadurch gekennzeichnet ist, dass** es die folgenden Schritte umfasst:
(a) Einführen von einer oder mehreren ersten heterologen DNA-Sequenzen in das Kerngenom der Pflanze,
(b) Infizieren der Pflanze mit mindestens einem viralen Transfektionsvektor, der in seinem Genom eine oder mehrere zweite heterologe DNA- oder RNA-Sequenzen enthält,
wodurch in der Pflanze ein Interaktionsprozess (I) ausgelöst wird zwischen
(i) Expressionsprodukten der(den) ersten heterologen DNA Sequenz(en) und
(ii) einer oder mehreren zweiten heterologen DNA oder RNA-Sequenz(en) des Transfektionsvektors und/oder Expressionsprodukten der zweiten heterologen DNA- oder RNA-Sequenzen und,
(iii) wahlweise, einer oder mehreren von außen zugegebenen Komponenten niederen Molekulargewichts,
wodurch der gewünschte biochemische Prozess (II) oder die gewünschte biochemische Kaskade (III), der/die vor der Interaktion nicht funktionsfähig war, angeschaltet wird.

2. Verfahren gemäß Anspruch 1, wobei die Infektion der Pflanze in Schritt (b) mittels eines rekonstituierten Viruspartikels oder infektiöser viraler Nukleinsäuren erreicht wird, oder durch Aktivieren einer Transfektion durch Freisetzen viraler Nukleinsäuren, die zuvor in das Pflanzengenom eingebaut wurden.

3. Verfahren gemäß Anspruch 2, wobei das rekonstituierte Viruspartikel oder die infektiöse virale Nukleinsäure RNA ist oder enthält.

4. Verfahren gemäß Anspruch 1, wobei die Infektion der Pflanze in Schritt (b) *Agrobacterium*-vermittelten Transfer von Nukleinsäuresequenzen in Zellen der Pflanze umfasst.

5. Verfahren gemäß einem der Ansprüche 1 bis 4, wobei ein weiterer Vektor in Schritt (b) eingeführt wird und wobei eine Sequenz und/oder ein Expressionsprodukt des weiteren Vektors in dem Interaktionsprozess involviert ist.

6. Verfahren gemäß einem der Ansprüche 1 bis 5, wobei die Infektion der Pflanze in Schritt (b) erreicht wird durch Einführen eines oder mehrerer Vektoren in Zellen der Pflanze, wobei der/die Vektor(en) so angepasst sind, dass sie den viralen Transfektionsvektor in Zellen der Pflanze durch Prozessierung generieren.

7. Verfahren gemäß einem der Ansprüche 1 bis 6, wobei der Interaktionsprozess ein einen viralen Transfektionsvektor erzeugender Prozess ist.

8. Verfahren gemäß einem der Ansprüche 1 bis 7, wobei der Interaktionsprozess eine DNA-Transposition umfasst.

9. Verfahren gemäß einem der Ansprüche 1 bis 8, wobei der Interaktionsprozess eine DNA-Rekombination umfasst.

10. Verfahren gemäß Anspruch 9, wobei der gewünschte biochemische Prozess oder die biochemische Kaskade die Expression einer ersten oder einer zweiten DNA- oder RNA-Sequenz umfasst, die ein promotorloses Gen in antisense Orientierung enthält, das bei dem Interaktionsprozess in positive (sense) Orientierung zu einem konstitutiven Promotor hin platziert wird.

11. Verfahren gemäß einem der Ansprüche 1 bis 7, wobei der Interaktionsprozess die Erkennung eines heterologen Promotors durch eine heterologe RNA-Polymerase beinhaltet.

12. Verfahren gemäß Anspruch 11, wobei die erste oder die zweite DNA- oder RNA-Sequenz eine zu exprimierende heterologe Sequenz unter der Kontrolle eines heterologen Promotors, der von einer pflanzlichen RNA-Polymerase nicht erkannt wird, aufweist, und die Transkription der zu exprimierenden Sequenz wird durch Interaktion des Promotors mit einer mit diesem funktionierenden RNA-Polymerase angeschaltet, wobei die RNA-Polymerase von der zweiten beziehungsweise von der ersten DNA-Sequenz kodiert wird.

13. Verfahren gemäß Anspruch 12, wobei die RNA-Polymerase und der heterologe Promotor aus Bakteriophagen stammen.

14. Verfahren gemäß einem der Ansprüche 1 bis 13, wobei der Interaktionsprozess eine DNA-Reaktion wie eine DNA-Replikation, Ligation, Hybridisierung, Transkription oder eine DNA-Restriktion umfasst.

15. Verfahren gemäß einem der Ansprüche 1 bis 14, wobei der Interaktionsprozess eine RNA Reaktion wie eine Replikation, Prozessierung, Spleißen, reverse Transkription, Hybridisierung oder Translation oder eine Aktivierung, Inhibierung oder Modifizierung einer solchen Reaktion umfasst.

16. Verfahren gemäß einem der Ansprüche 1 bis 15, wobei der Interaktionsprozess eine Proteinreaktion wie Proteinfaltung, Zusammenbau, Aktivierung, posttranslationale Modifizierung, Targeting, Binden, enzymatische Aktivität oder Signaltransduktion oder eine Aktivierung, Inhibierung oder Modifizierung einer solchen Reaktion umfasst.

17. Verfahren gemäß einem der Ansprüche 1 bis 9, wobei
(i) der gewünschte biochemische Prozess oder die Kaskade die Expression der zweiten DNA-Sequenz beinhaltet, die von ihrem Promotor durch eine DNA-Insertion getrennt ist, die die Transkription der zweiten DNA-Sequenz verhindern kann, und
(ii) der in Schritt (b) ausgelöste Interaktionsprozess zur Entfernung der DNA-Insertion führt, wodurch die erste oder die zweite DNA-Sequenz exprimiert wird.

18. Verfahren gemäß Anspruch 17, wobei die DNA-Insertion ein nichtautonomes transponierbares Element ist, das durch eine Transposase entfernt wird, die von einer ersten DNA-Sequenz im Kerngenom kodiert wird für eine Insertion im viralen Vektor.

19. Verfahren gemäß Anspruch 17, wobei die DNA-Insertion von gerichteten Stellen flankiert ist, die von einer ortsspezifischen Rekombinase erkannt werden können, die für eine Insertion im viralen Vektor von einer ersten DNA-Sequenz im Kerngenom kodiert wird.

20. Verfahren gemäß einem der Ansprüche 1 bis 7, wobei die Transkription der zweiten DNA Sequenz von einem heterologen oder künstlich veränderten Transkriptionsfaktor angeschalten wird, der einen heterologen oder künstlich veränderten oder chimären Promotor erkennen kann, der funktionell an ein heterologes Zielgen der zweiten DNA-Sequenz gekoppelt ist, wobei der Promotor von keinem natürlichen pflanzlichen Transkriptionsfaktor erkannt werden kann und wobei der heterologe oder künstlich veränderte Transkriptionsfaktor von der ersten DNA-Sequenz codiert wird.

21. Verfahren gemäß Anspruch 20, wobei der Transkriptionsfaktor durch eine von außen zugegebene Komponente von niederem Molekulargewicht induzierbar ist.

22. Verfahren gemäß einem der Ansprüche 1 bis 21, wobei die erste heterologe DNA-Sequenz von Schritt (a) nicht pflanzenviralen Ursprungs ist.

23. Verfahren zur Kontrolle eines gewünschten biochemischen Prozesses (II) oder einer gewünschten biochemischen Kaskade (III) in einer Pflanze, das **dadurch gekennzeichnet ist, dass** es die folgenden Schritte umfasst:
(a) Einführen von einer oder mehreren ersten heterologen DNA-Sequenzen in das Kerngenom der Pflanze,
(b) Infizieren der Pflanze mit mindestens einem viralen Transfektionsvektor, der in seinem Genom eine oder mehrere zweite heterologe DNA- oder RNA-Sequenzen enthält,
wodurch in der Pflanze ein Interaktionsprozess (I) ausgelöst wird zwischen
(i) einer oder mehreren ersten heterologen DNA-Sequenzen des Kerngenoms und/oder Expressionsprodukten der ersten heterologen DNA Sequenzen und
(ii) Expressionsprodukten der zweiten heterologen DNA- oder RNA-Sequenzen und,
(iii) wahlweise, einer oder mehreren von außen zugegebenen Komponenten niederen Molekulargewichts,
wobei der Interaktionsprozess einen Prozess ausgewählt aus der folgenden Gruppe beinhaltet: DNA-Replikation, DNA-Ligation, DNA-Hybridisierung, Transkription, DNA-Restrkition, RNA-Replikation, RNA-Prozessierung, RNA-Spleißen, reverse Transkription, RNA-Hybridisierung, RNA-Translation, RNA-Aktivierung, RNA-Inhibierung, RNA-Modifizierung derselben, Proteinfaltung, Proteinassemblierung, Proteinaktivierung, posttranslationale Proteinmodifizierung, Protein-Targeting, Proteinbindung, enzymatische Aktivität, Protein-Signaltransduktion oder Proteinaktivierung, Inhibierung oder Modifizierung derselben,
wodurch der gewünschte biochemische Prozess (II) oder die gewünschte biochemische Kaskade (III), der/die vor der Interaktion nicht funktionsfähig war, angeschaltet wird.

24. Das Verfahren gemäß Anspruch 23, wobei die Infektion der Pflanze in Schritt (b) mittels eines rekonstituierten Viruspartikels oder infektiöser viraler Nukleinsäuren erreicht wird, oder durch Aktivieren einer Transfektion durch Freisetzen viraler Nukleinsäuren, die zuvor in das Pflanzengenom eingebaut wurden.

25. Verfahren gemäß Anspruch 24, wobei das rekonstituierte Viruspartikel oder die infektiöse virale Nukleinsäure RNA ist oder enthält.

26. Verfahren gemäß einem der Ansprüche 23 bis 25, wobei die Infektion der Pflanze in Schritt (b) *Agrobacterium*-vermittelten Transfer von Nukleinsäuresequenzen in Zellen der Pflanze umfasst.

27. Verfahren gemäß einem der Ansprüche 23 bis 26, wobei ein weiterer Vektor in Schritt (b) eingeführt wird und wobei eine Sequenz und/oder ein Expressionsprodukt des weiteren Vektors in dem Interaktionsprozess involviert ist.

28. Verfahren gemäß einem der Ansprüche 23 bis 27, wobei die Infektion der Pflanze in Schritt (b) erreicht wird, durch Einführen eines oder mehrerer Vektoren in Zellen der Pflanze, wobei der/die Vektor(en) so angepasst sind, dass sie den viralen Transfektionsvektor in Zellen der Pflanze durch Prozessierung generieren.

29. Verfahren gemäß einem der Ansprüche 23 bis 28, wobei der Interaktionsprozess ein einen viralen Transfektionsvektor erzeugender Prozess ist.

30. Verfahren gemäß einem der Ansprüche 23 bis 29, wobei der Interaktionsprozess eine DNA Transposition umfasst.

31. Verfahren gemäß einem der Ansprüche 23 bis 29, wobei der gewünschte biochemische Prozess oder die biochemische Kaskade die Expression einer ersten oder einer zweiten DNA- oder RNA-Sequenz umfasst, die ein promotorloses Gen in antisense Orientierung enthält, das bei dem Interaktionsprozess in sense Orientierung zu einem konstitutiven Promotor hin platziert wird.

32. Verfahren gemäß einem der Ansprüche 23 bis 29, wobei der Interaktionsprozess die Erkennung eines heterologen Promotors durch eine heterologe RNA-Polymerase beinhaltet.

33. Verfahren gemäß Anspruch 32, wobei die erste oder die zweite DNA- oder RNA-Sequenz eine zu exprimierende heterologe Sequenz unter der Kontrolle eines heterologen Promotors, der von einer pflanzlichen RNA-Polymerase nicht erkannt wird, aufweist, und die Transkription der zu exprimierenden Sequenz wird durch Interaktion des Promotors mit einer mit diesem funktionierenden RNA-Polymerase angeschaltet, wobei die RNA-Polymerase von der zweiten beziehungsweise von der ersten DNA-Sequenz kodiert wird.

34. Verfahren gemäß Anspruch 33, wobei die RNA-Polymerase und der heterologe Promotor aus Bakteriophagen stammen.

35. Verfahren gemäß einem der Ansprüche 23 bis 30, wobei
(i) der gewünschte biochemische Prozess oder die Kaskade die Expression einer ersten oder einer zweiten DNA-Sequenz beinhaltet, die von ihrem Promotor durch eine DNA-Insertion getrennt ist, die die Transkription der ersten oder der zweiten DNA-Sequenz verhindern kann, und
(ii) der in Schritt (b) ausgelöste Interaktionsprozess zur Entfernung der DNA-Insertion führt, wodurch die erste oder die zweite DNA-Sequenz exprimiert wird.

36. Verfahren gemäß Anspruch 35, wobei die DNA-Insertion ein nichtautonomes transponierbares Element ist, das durch eine Transposase entfernt wird, die von einer zweiten DNA-Sequenz im viralen Vektor kodiert wird für eine Insertion im Kerngenom.

37. Verfahren gemäß einem der Ansprüche 23 bis 29, wobei die Transkription der ersten DNA-Sequenz von einem heterologen oder künstlich veränderten Transkriptionsfaktor angeschalten wird, der einen heterologen oder künstlich veränderten oder chimären Promotor erkennen kann, der funktionell an ein heterologes Zielgen der zweiten DNA-Sequenz gekoppelt ist, wobei der Promotor von keinem natürlichen pflanzlichen Transkriptionsfaktor erkannt werden kann und wobei der heterologe oder künstlich veränderte Transkriptionsfaktor von der zweiten DNA-Sequenz codiert wird.

38. Verfahren gemäß Anspruch 37, wobei der Transkriptionsfaktor durch eine von außen zugegebene Komponente von niederem Molekulargewicht induzierbar ist.

39. Verfahren gemäß einem der Ansprüche 23 bis 38, wobei die erste heterologe DNA-Sequenz von Schritt (a) nicht pflanzenviralen Ursprungs ist.

40. Verfahren zur Kontrolle eines gewünschten biochemischen Prozesses (II) oder einer gewünschten biochemischen Kaskade (III) in einer Pflanze, das **dadurch gekennzeichnet ist, dass** es die folgenden Schritte umfasst:
(a) Einführen von einer oder mehreren ersten heterologen DNA-Sequenzen in das Kerngenom der Pflanze,
(b) Infizieren der Pflanze mit zwei oder mehr Vektoren, die in ihrem Genom eine oder mehrere zweite heterologe Nukleinsäuresequenzen enthalten,
wodurch in der Pflanze ein Interaktionsprozess (I) ausgelöst wird zwischen
(i) einer ortsspezifischen Rekombinase als Expressionsprodukt der ersten heterologen Nukleinsäuresequenzen und
(ii) zwei oder mehr zweiten heterologen Nukleinsäuresuenzen der Vektoren als Elemente eines viralen Transfektionsvektors;
wodurch ein viraler Transfektionsvektor in Zellen der Pflanze mittels ortsspezifischer Rekombination generiert wird, wodurch der gewünschte biochemische Prozess (II) oder die gewünschte biochemische Kaskade (III), der/die vor der Interaktion nicht funktionsfähig war, angeschaltet wird.

41. Das Verfahren gemäß Anspruch 40, ferner durch Merkmale wie in einem der Ansprüche 1 bis 39 **gekennzeichnet**.

42. Verfahren zur Erzeugung eines Produkts in einer transgenen Pflanze, umfassend die Schritte des Verfahrens von einem der Ansprüche 1 bis 40.

43. Das Verfahren gemäß Anspruch 42, ferner die folgenden Schritte umfassend:
(a) Wachsenlassen der transgenen Pflanze bis zu einer gewünschten Stufe gefolgt von
(b) Infizieren der Pflanze mit einem oder mehreren viralen Transfektionsvektoren und optionales Kontaktieren der Pflanze mit einem oder mehreren niedermolekularen Komponenten, wodurch der biochemische Prozess oder die biochemische Kaskade, der/die für die Expression des Produkts nötig ist, angeschaltet wird, wobei der Prozess oder die Kaskade vor der Interaktion nicht funktionsfähig war, und
(c) Produzieren des Produkts in der Pflanze.

## Revendications

1. Procédé pour contrôler un processus biochimique (II) ou une cascade biochimique (III) présentant un intérêt dans une plante, ledit procédé étant **caractérisé en ce qu'**il comprend les étapes suivantes :
(a) introduction, dans le génome nucléaire de la plante, d'une ou plusieurs premières séquences d'ADN hétérologues,
(b) infection de la plante avec au moins un vecteur de transfection viral contenant dans son génome une ou plusieurs deuxièmes séquences d'ADN ou d'ARN hétérologues,
ce qui déclenche un processus d'interaction (I) dans la plante entre
(i) les produits d'expression de la ou des premières séquences d'ADN hétérologues, et
(ii) une ou plusieurs deuxièmes séquences d'ADN ou d'ARN hétérologues du vecteur de transfection et/ou les produits d'expression des deuxièmes séquences d'ADN ou d'ARN hétérologues, et
(iii)éventuellement un ou plusieurs composants de faible poids moléculaire ajoutés depuis l'extérieur,
ce qui réalise ainsi un déclenchement du processus biochimique (II) ou de la cascade biochimique (III) présentant un intérêt qui n'était pas fonctionnel avant ladite interaction.

2. Procédé selon la revendication 1, dans lequel l'infection de la plante dans l'étape (b) est effectuée par une particule virale assemblée ou des acides nucléiques viraux infectieux, ou par activation d'un processus de transfection par libération d'acides nucléiques viraux qui avaient été incorporés au préalable dans le génome de la plante.

3. Procédé selon la revendication 2, dans lequel ladite particule virale assemblée ou ledit acide nucléique viral infectieux est ou comprend un ARN.

4. Procédé selon la revendication 1, dans lequel l'infection de la plante dans l'étape (b) comprend un transfert à médiation par *Agrobacterium* de séquences d'acide nucléique dans des cellules de ladite plante.

5. Procédé selon l'une quelconque des revendications 1 à 4, dans lequel un autre vecteur est introduit dans l'étape (b) et dans lequel une séquence et/ou un produit d'expression dudit autre vecteur sont impliqués dans ledit processus d'interaction.

6. Procédé selon l'une quelconque des revendications 1 à 5, dans lequel l'infection de la plante dans l'étape (b) est effectuée par introduction d'un ou plusieurs vecteurs dans des cellules de ladite plante, ledit ou lesdits vecteurs étant adaptés pour subir une transformation afin de générer ledit vecteur de transfection viral dans des cellules de ladite plante.

7. Procédé selon l'une des revendications 1 à 6,
dans lequel ledit processus d'interaction est un processus de génération de vecteur de transfection viral.

8. Procédé selon l'une quelconque des revendications 1 à 7, dans lequel le processus d'interaction met en jeu une transposition d'ADN.

9. Procédé selon l'une quelconque des revendications 1 à 8, dans lequel le processus d'interaction met en jeu une recombinaison d'ADN.

10. Procédé selon la revendication 9, dans lequel le processus ou la cascade biochimique présentant un intérêt comprend l'expression d'une première ou deuxième séquence d'ADN ou d'ARN comprenant un gène sans promoteur en orientation antisens qui est placé en orientation sens vis-à-vis d'un promoteur constitutif dans ledit processus d'interaction.

11. Procédé selon l'une quelconque des revendications 1 à 7, dans lequel le processus d'interaction met en jeu la reconnaissance d'un promoteur hétérologue par une ARN polymérase hétérologue.

12. Procédé selon la revendication 11, dans lequel ladite première ou ladite deuxième séquence d'ADN ou d'ARN comprend une séquence hétérologue devant être exprimée sous le contrôle d'un promoteur hétérologue non reconnu par une ARN polymérase végétale, et la transcription de ladite séquence devant être exprimée est déclenchée par interaction dudit promoteur avec une ARN polymérase fonctionnelle avec celui-ci et codée par ladite deuxième ou ladite première séquence d'ADN, respectivement.

13. Procédé selon la revendication 12, dans lequel ladite ARN polymérase est une ARN polymérase de bactériophage et ledit promoteur hétérologue est un promoteur de bactériophage.

14. Procédé selon l'une quelconque des revendications 1 à 13, dans lequel le processus d'interaction met en jeu une réaction d'ADN telle qu'une réplication, une ligature, une hybridation, une transcription, ou une restriction d'ADN.

15. Procédé selon l'une des revendications 1 à 14,
dans lequel le processus d'interaction met en jeu une réaction d'un ARN telle qu'une réplication, une transformation, un épissage, une transcription inverse, une hybridation ou une traduction, ou une activation, une inhibition ou une modification de celle-ci.

16. Procédé selon l'une quelconque des revendications 1 à 15, dans lequel le processus d'interaction met en jeu une réaction d'une protéine telle qu'un repliement, un assemblage, une activation, une modification post-traductionnelle, un ciblage, une liaison, une activité enzymatique ou une transduction du signal, ou une activation, une inhibition ou une modification de celle-ci.

17. Procédé selon l'une des revendications 1 à 9,
dans lequel.
(i) le processus ou la cascade biochimique présentant un intérêt comprend l'expression de la deuxième séquence d'ADN séparée de son promoteur par un insérat d'ADN capable d'empêcher la transcription de la deuxième séquence d'ADN, et
(ii) le processus d'interaction déclenché dans l'étape (b) a pour résultat l'excision de l'insérat d'ADN, ce par quoi la deuxième séquence d'ADN est exprimée.

18. Procédé selon la revendication 17, dans lequel l'insérat d'ADN est un élément transposable non autonome qui est excisé par une transposase codée par une première séquence d'ADN dans le génome nucléaire pour une insertion dans le vecteur viral.

19. Procédé selon la revendication 17, dans lequel l'insérat d'ADN est flanqué par des sites unidirectionnels reconnaissables par une ADN recombinase à spécificité de site codée par une première séquence d'ADN dans le génome nucléaire pour une insertion dans le vecteur viral.

20. Procédé selon l'une des revendications 1 à 7,
dans lequel la transcription de la deuxième séquence d'ADN est déclenchée par un facteur de transcription hétérologue ou spécifiquement conçu, capable de reconnaître un promoteur hétérologue ou spécifiquement conçu ou chimère lié de manière fonctionnelle à un gène hétérologue présentant un intérêt de la deuxième séquence d'ADN, ledit promoteur n'étant pas reconnaissable par un quelconque facteur de transcription végétal naturel et ledit facteur de transcription hétérologue ou spécifiquement conçu étant codé par la première séquence d'ADN.

21. Procédé selon la revendication 20, dans lequel le facteur de transcription est inductible par un composant de faible poids moléculaire appliqué depuis l'extérieur.

22. Procédé selon l'une des revendications 1 à 21,
dans lequel ladite première séquence d'ADN hétérologue de l'étape (a) n'est pas d'origine virale végétale.

23. Procédé pour contrôler un processus biochimique (II) ou une cascade biochimique (III) présentant un intérêt dans une plante, ledit procédé étant **caractérisé en ce qu'**il comprend les étapes suivantes :
(a) introduction, dans le génome nucléaire de la plante, d'une ou plusieurs premières séquences d'ADN hétérologues,
(b) infection de la plante avec au moins un vecteur de transfection viral contenant dans son génome une ou plusieurs deuxièmes séquences d'ADN ou d'ARN hétérologues,
ce qui déclenche un processus d'interaction (I) dans la plante entre
(i) une ou plusieurs premières séquences d'ADN hétérologues du génome nucléaire et/ou les produits d'expression des premières séquences d'ADN hétérologues, et
(ii) les produits d'expression de la ou des deuxièmes séquences d'ADN ou d'ARN hétérologues, et
(iii)éventuellement un ou plusieurs composants de faible poids moléculaire ajoutés depuis l'extérieur,
dans lequel le processus d'interaction met en jeu un processus choisi dans l'ensemble suivant : réplication d'ADN, ligature d'ADN, hybridation d'ADN, transcription, restriction d'ADN, réplication d'ARN, transformation d'ARN, épissage d'ARN, transcription inverse, hybridation d'ARN, traduction d'ARN, activation d'ARN, inhibition d'ARN, modification d'ARN des précédents, repliement de protéine, assemblage de protéine, activation de protéine, modification post-traductionnelle de protéine, ciblage de protéine, liaison de protéine, activité enzymatique, transduction de signal de protéine, ou activation de protéine, inhibition ou modification des précédents, ce qui réalise ainsi un déclenchement du processus biochimique (II) ou de la cascade biochimique (III) présentant un intérêt qui n'était pas fonctionnel avant ladite interaction.

24. Procédé selon la revendication 23, dans lequel l'infection de la plante dans l'étape (b) est effectuée par une particule virale assemblée ou des acides nucléiques viraux infectieux, ou par activation d'un processus de transfection par libération d'acides nucléiques viraux qui avaient été incorporés au préalable dans le génome de la plante.

25. Procédé selon la revendication 24, dans lequel ladite particule virale assemblée ou ledit acide nucléique viral infectieux est ou comprend un ARN.

26. Procédé selon l'une quelconque des revendications 23 à 25, dans lequel l'infection de la plante dans l'étape (b) comprend un transfert à médiation par *Agrobacterium* de séquences d'acide nucléique dans des cellules de ladite plante.

27. Procédé selon l'une quelconque des revendications 23 à 26, dans lequel un autre vecteur est introduit dans l'étape (b) et dans lequel une séquence et/ou un produit d'expression dudit autre vecteur sont impliqués dans ledit processus d'interaction.

28. Procédé selon l'une quelconque des revendications 23 à 27, dans lequel l'infection de la plante dans l'étape (b) est effectuée par introduction d'un ou plusieurs vecteurs dans des cellules de ladite plante, ledit ou lesdits vecteurs étant adaptés pour subir une transformation afin de générer ledit vecteur de transfection viral dans des cellules de ladite plante.

29. Procédé selon l'une des revendications 23 à 28,
dans lequel ledit processus d'interaction est un processus de génération de vecteur de transfection viral.

30. Procédé selon l'une quelconque des revendications 23 à 29, dans lequel le processus d'interaction met en jeu une transposition d'ADN.

31. Procédé selon les revendications 23 à 29, dans lequel le processus ou la cascade biochimique présentant un intérêt comprend l'expression d'une première ou deuxième séquence d'ADN ou d'ARN comprenant un gène sans promoteur en orientation antisens qui est placé en orientation sens vis-à-vis d'un promoteur constitutif dans ledit processus d'interaction.

32. Procédé selon l'une quelconque des revendications 23 à 29, dans lequel le processus d'interaction met en jeu la reconnaissance d'un promoteur hétérologue par une ARN polymérase hétérologue.

33. Procédé selon la revendication 32, dans lequel ladite première ou ladite deuxième séquence d'ADN ou d'ARN comprend une séquence hétérologue devant être exprimée sous le contrôle d'un promoteur hétérologue non reconnu par une ARN polymérase végétale, et la transcription de ladite séquence devant être exprimée est déclenchée par interaction dudit promoteur avec une ARN polymérase fonctionnelle avec celui-ci et codée par ladite deuxième ou ladite première séquence d'ADN, respectivement.

34. Procédé selon la revendication 33, dans lequel ladite ARN polymérase est une ARN polymérase de bactériophage et ledit promoteur hétérologue est un promoteur de bactériophage.

35. Procédé selon l'une des revendications 23 à 30,
dans lequel
(i) le processus ou la cascade biochimique présentant un intérêt comprend l'expression d'une première ou deuxième séquence d'ADN séparée de son promoteur par un insérat d'ADN capable d'empêcher la transcription de la première ou deuxième séquence d'ADN, et
(ii) le processus d'interaction déclenché dans l'étape (b) a pour résultat l'excision de l'insérat d'ADN, ce par quoi la première ou deuxième séquence d'ADN est exprimée.

36. Procédé selon la revendication 35, dans lequel l'insérat d'ADN est un élément transposable non autonome qui est excisé par une transposase codée par une deuxième séquence d'ADN sur le vecteur viral pour une insertion dans le génome nucléaire.

37. Procédé selon l'une des revendications 23 à 29,
dans lequel la transcription de la première séquence d'ADN est déclenchée par un facteur de transcription hétérologue ou spécifiquement conçu, capable de reconnaître un promoteur hétérologue ou spécifiquement conçu ou chimère lié de manière fonctionnelle à un gène hétérologue présentant un intérêt de ladite deuxième séquence d'ADN, ledit promoteur n'étant pas reconnaissable par un quelconque facteur de transcription végétal naturel et ledit facteur de transcription hétérologue ou spécifiquement conçu étant codé par la deuxième séquence d'ADN.

38. Procédé selon la revendication 37, dans lequel le facteur de transcription est inductible par un composant de faible poids moléculaire appliqué depuis l'extérieur.

39. Procédé selon l'une des revendications 23 à 38,
dans lequel ladite première séquence d'ADN hétérologue de l'étape (a) n'est pas d'origine virale végétale.

40. Procédé pour contrôler un processus biochimique (II) ou une cascade biochimique (III) présentant un intérêt dans une plante, ledit procédé étant **caractérisé en ce qu'**il comprend les étapes suivantes :
(a) introduction, dans le génome nucléaire de la plante, d'une ou plusieurs premières séquences d'acide nucléique hétérologues,
(b) infection de la plante avec deux ou plus de deux vecteurs contenant dans leur génome une ou plusieurs deuxièmes séquences d'acide nucléique hétérologues,
ce qui déclenche un processus d'interaction (I) dans la plante entre
(i) une ADN recombinase à spécificité de site en tant que produit d'expression des premières séquences d'acide nucléique hétérologues, et
(ii) deux ou plus de deux deuxièmes séquences d'acide nucléique hétérologues desdits vecteurs en tant qu'éléments d'un vecteur de transfection viral ;
ce par quoi un vecteur de transfection viral est généré dans des cellules de ladite plante par recombinaison à spécificité de site, ce qui réalise un déclenchement du processus biochimique (II) ou de la cascade biochimique
(III) présentant un intérêt qui n'était pas fonctionnel avant ladite interaction.

41. Procédé selon la revendication 40, **caractérisé en outre par** les caractéristiques telles que définies dans l'une des revendications 1 à 39.

42. Procédé pour produire un produit dans une plante transgénique, comprenant les étapes du procédé de l'une des revendications 1 à 40.

43. Procédé selon la revendication 42, comprenant en outre les étapes suivantes :
(a) croissance de la plante transgénique jusqu'à un stade souhaitée, suivie par
(b) infection de la plante avec un ou plusieurs vecteurs de transfection viraux, et éventuellement mise en contact de la plante avec un ou plusieurs composants de faible poids moléculaire, ce qui déclenche le processus ou la cascade biochimique nécessaire pour la production du produit, ledit processus ou cascade n'étant pas fonctionnel avant ladite interaction, et
(c) production du produit dans la plante.
